# EUROPEAN PATENT SPECIFICATION

(11) **EP 4 171 643 B1**
(45) Date of publication and mention of the grant of the patent: **04.06.2025**
(21) Application number: 21734544.6
(22) Date of filing: 23.06.2021
(51) Int. Cl.: A61K 47/54, A61K 45/06, A61P 35/00

(54) **THERAPEUTIC COMBINATION OF GALNAC-OLIGONUCLEOTIDE CONJUGATE AND SAPONIN, AND USES THEREOF**
THERAPEUTISCHE KOMBINATION AUS GALNAC-OLIGONUKLEOTID-KONJUGAT UND SAPONIN UND VERWENDUNGEN DAVON
COMBINAISON THÉRAPEUTIQUE DE CONJUGUÉ GALNAC-OLIGONUCLÉOTIDE ET DE SAPONINE, ET SES UTILISATIONS

(30) Priority: 24.06.2020 NL 2025902; 18.06.2021 WO PCT/NL2021/050384
(43) Date of publication of application: 03.05.2023
(60) Divisional application: 25173229.3
(73) Proprietor: Sapreme Technologies B.V., 3584 CM Utrecht (NL)
(72) Inventor: POSTEL, Ruben, 3584 CM Utrecht (NL); HERMANS, Guy, 9820 Merelbeke (BE)
(74) Representative: Nederlandsch Octrooibureau
(86) International application number: PCT/NL2021/050394
(87) International publication number: WO 2021/261998

(56) References cited:
- WO-A1-2011/054811
- WO-A1-2015/071388
- WO-A1-2020/126604
- HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), pages 14, XP055429235, DOI: 10.3390/biomedicines5020014
- SABINE SEWING ET AL: "GalNAc Conjugation Attenuates the Cytotoxicity of Antisense Oligonucleotide Drugs in Renal Tubular Cells", MOLECULAR THERAPY: NUCLEIC ACIDS., vol. 14, 1 March 2019 (2019-03-01), US, pages 67 - 79, XP055762603, ISSN: 2162-2531, DOI: 10.1016/j.omtn.2018.11.005
- ROGER GILABERT-ORIOL ET AL: "Modified Trastuzumab and Cetuximab Mediate Efficient Toxin Delivery While Retaining Antibody-Dependent Cell-Mediated Cytotoxicity in Target Cells", MOLECULAR PHARMACEUTICS, vol. 10, no. 11, 7 October 2013 (2013-10-07), US, pages 4347 - 4357, XP055677139, ISSN: 1543-8384, DOI: 10.1021/mp400444q
- GILABERT-ORIOL ROGER ET AL: "Dianthin-30 or gelonin versus monomethyl auristatin E, each configured with an anti-calcitonin receptor antibody, are differentially potent in vitro in high-grade glioma cell lines derived from glioblastoma", CANCER IMMUNOLOGY, IMMUNOTHERAPY, SPRINGER, BERLIN/HEIDELBERG, vol. 66, no. 9, 13 May 2017 (2017-05-13), pages 1217 - 1228, XP036309505, ISSN: 0340-7004, [retrieved on 20170513], DOI: 10.1007/S00262-017-2013-Z
- ROGER GILABERT-ORIOL ET AL: "Combinatorial approach to increase efficacy of Cetuximab, Panitumumab and Trastuzumab by dianthin conjugation and co-application of SO1861", BIOCHEMICAL PHARMACOLOGY, vol. 97, no. 3, 1 October 2015 (2015-10-01), US, pages 247 - 255, XP055677222, ISSN: 0006-2952, DOI: 10.1016/j.bcp.2015.07.040
- CHEENU BHARGAVA ET AL: "Targeted dianthin is a powerful toxin to treat pancreatic carcinoma when applied in combination with the glycosylated triterpene SO1861", MOLECULAR ONCOLOGY, vol. 11, no. 11, 15 September 2017 (2017-09-15), pages 1527 - 1543, XP055677214, ISSN: 1574-7891, DOI: 10.1002/1878-0261.12115
- ALEXANDER WENG ET AL: "The toxin component of targeted anti-tumor toxins determines their efficacy increase by saponins", MOLECULAR ONCOLOGY, ELSEVIER, vol. 6, no. 3, 12 January 2012 (2012-01-12), pages 323 - 332, XP028508748, ISSN: 1574-7891, [retrieved on 20120124], DOI: 10.1016/J.MOLONC.2012.01.004
- YUANYU HUANG: "Preclinical and Clinical Advances of GalNAc-Decorated Nucleic Acid Therapeutics", MOLECULAR THERAPY: NUCLEIC ACIDS., vol. 6, 1 March 2017 (2017-03-01), US, pages 116 - 132, XP055485332, ISSN: 2162-2531, DOI: 10.1016/j.omtn.2016.12.003
- AARON D. SPRINGER ET AL: "GalNAc-siRNA Conjugates: Leading the Way for Delivery of RNAi Therapeutics", NUCLEIC ACID THERAPEUTICS, vol. 28, no. 3, 1 June 2018 (2018-06-01), US, pages 109 - 118, XP055555952, ISSN: 2159-3337, DOI: 10.1089/nat.2018.0736

## Description

### TECHNOLOGICAL FIELD

The invention relates to a pharmaceutical combination comprising: a conjugate of an effector molecule and a ligand for ASGPR, wherein the ligand for ASGPR comprises at least one GalNAc moiety; and a saponin of the monodesmosidic or bidesmosidic triterpene glycoside type. The invention also relates to a pharmaceutical composition comprising the conjugate and the saponin. In addition, the invention relates to a pharmaceutical combination or composition of the invention, for use as a medicament, for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH, and/or for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease. Furthermore, the invention relates to an *in vitro* or *ex vivo* method for transferring an effector molecule of the invention from outside a cell to inside said cell, preferably into the cytosol of said cell. The invention also relates to an *in vitro* or *ex vivo* method for transferring the conjugate of the invention from outside a cell to inside said cell.

### BACKGROUND

The ability to inhibit a protein's function, whether in humans or in pathogens, is an integral part of the discovery of new drugs. Conventional small molecule drugs as well as antibodies are strongly limited to a subset of the available targets. Small molecules bind principally to cofactor sites or transmitter sites, effectively sites that are designed to accommodate small molecules. Antibodies can bind a greater variety of proteins but are commonly limited to extracellular targets.

Oligonucleotide therapy is a relatively young and fast-developing field which aims to overcome many of the issues encountered with small-molecule drugs or antibody drugs by directly manipulating the genetic transcription and translation pathways. The potency and versatility of oligonucleotides, in particular the prospect of suppressing genes encoding proteins that are 'undruggable' by classical small molecule drugs, makes them attractive drug candidates. The first oligonucleotide drugs were based on antisense technology, whereby single-stranded nucleic acid molecules would bind with sequence specificity to their complementary mRNA target, thus triggering degradation of the duplex by the RNase H system.

In 1998, Andrew Fire and Craig Mello published a seminal paper identifying double-stranded RNAs (dsRNAs) as the causative agents for post-transcriptional gene silencing (PTGS) in *Caenorhabditis elegans,* a phenomenon they termed RNA interference (RNAi). The discovery of RNAi explained puzzling observations of gene silencing in plants and fungi and kicked off a revolution in biology that eventually showed non-coding RNAs to be central regulators of gene expression in multicellular organisms. Shortly thereafter it was discovered that small dsRNAs (typically 15-30 bp) can catalytically induce RNAi silencing in mammalian cells without eliciting nonspecific interferon responses. Targeting the RNAi pathway through small dsRNAs such as small interfering RNAs (siRNAs) and short hairpin RNA (shRNA) has several theoretical advantages over antisense being notably more efficient (catalytic) and giving longer inhibition of gene expression. This could translate into lower doses and lower cost together with less frequent dosing. Lower exposures could also mean fewer toxicity problems for siRNA.

However, before siRNA reaches its target *in vivo,* it faces a number of significant barriers that block its pathway to the RNA-Induced Silencing Complex (RISC) machinery. Upon entering the bloodstream, siRNA is vulnerable to degradation by endogenous nucleases, and renal excretion due to its small size and highly anionic character. In addition, before reaching its target cell, the siRNA must navigate the tight endothelial junctions of the blood vessels and diffuse through the extracellular matrix. Due to its numerous negative charges, siRNA does not readily bind to or cross the cell membrane, and once inside cells, it must escape from endosomes to interact with its intracellular protein targets.

Hence, the success of oligonucleotide (such as siRNA) therapy strongly depends on an effective delivery of the drug from outside a cell into the cytosol. Consequently, much attention is directed at developing delivery systems which improve oligonucleotide (such as siRNA) delivery. Aside from viral delivery, the main methods employed to enhance siRNA (or other oligonucleotides) delivery to the cell employ liposomes, cell-penetrating peptides (CPPs) and their mimics, or nanoparticles (Gooding, Matt, et al. Chemical biology & drug design 80.6 (2012): 787-809). Safety concerns, such as the possibility of insertion mutagenesis and immunogenesis, are considered to limit the future of viral approaches.

Liposomes are the most commonly used delivery vector for oligonucleotides such as siRNA, where the oligonucleotide is encapsulated within a lipid bilayer. Several problems are encountered with liposomal oligonucleotide delivery, such as oxygen radical-mediated toxicity (typical for of cationic liposomes), cell toxicity, effects on gene regulation and inflammatory responses. Furthermore, *in vivo* delivery using lipids seems to predominantly target the liver and spleen.

Cell-penetrating peptides (CCP), also called protein transduction domains, are short peptides (usually <30 amino-acid residues long) which have the unusual property of being able to cross the cell membrane. Through covalent linking to the oligonucleotide or through the formation of non-covalent complexes with the oligonucleotide, a CPP may enhance cellular uptake of the oligonucleotide. The field of CPP mediated oligonucleotide delivery is extremely complex since it combines the challenges posed by both oligonucleotide technology and peptide technology, two fields which are far from mature, in a single drug. Aside from the oligonucleotide-related issues, typical challenges encountered for CPPs are related to the (lack of) *in vivo* stability of the peptide chain, often requiring the use of non-natural peptide derivatives which may be complex to synthesize and/or exhibit reduced cell-penetrating activity.

Despite the enhanced cellular delivery, overcoming entrapment by endosomes is one of the major challenges to the design of efficient CPPs and other transport systems (Gooding *et al*).

Nanoparticles and nanocarriers are nanoscale oligonucleotide delivery systems typically comprised of a polymer, biological stabilizers and cell-targeting ligands complexed with the oligonucleotide. An exemplary siRNA nanocarrier system is known under the name siRNA Dynamic Poly-Conjugates. This system is based around an amphipathic polymer linked to polyethylene glycol (PEG) as a biological stabilizer and a hepatocyte-targeting ligand wherein the siRNA is covalently bound to the polymer by a disulfide bond. The targeting moieties and PEG moieties are attached to the polymer via maleamate linkages, forming negatively charged nanoparticles, which do not bind to serum proteins. Following internalization by endocytosis, the maleamate bonds are readily hydrolysed on acidification of the endosome, exposing the cationic amine groups of the polymer and inducing endosomal escape via a proton sponge effect. Nanocarriers are also known in the form of cationic polymers such as polyethyleneimines (PEIs) (sometimes combined with cyclodextrins), which can form electrostatic complexes with oligonucleotides such as siRNA, affording protection from degradation and aiding internalisation. However, toxicity at higher concentrations resulting from membrane disruption and apoptosis induction may limit the applications of cationic polymers as therapeutic delivery agent. Aside from complex preparation of the nanoparticle and nanocarriers, their long-term stability is a major barrier to commercially viable use.

Despite the improved oligonucleotide (such as siRNA) delivery to the cell which may be achieved by some of these delivery systems, endosomal escape remains a major barrier to the application of oligonucleotide-based therapeutics such as RNAi-based therapeutics. Only a minuscule portion of endocytosed oligonucleotide escapes into the cytoplasmic space where it can exert its intended function, while the vast majority remains trapped in endocytic compartments and is inactive. Recent literature suggests a passive siRNA escape rate of <0.01% (Setten, Ryan L., et al. Nature Reviews Drug Discovery 18.6 (2019): 421-446). Furthermore, it has been shown that the capacity of cells to functionally internalize oligonucleotides to produce target effect (*e.g.* knockdown) appears to be independent of the extent to which cells internalize bulk oligonucleotides. These observations have led to the hypothesis of separate 'productive' and 'nonproductive' free-uptake pathways, although the molecular mechanisms distinguishing these pathways remain obscure (Setten, Ryan L., John J. Rossi, and Si-ping Han. Nature Reviews Drug Discovery 18.6 (2019): 421-446).

All in all, there remains a significant need for improved delivery systems of oligonucleotides which result in an increased potency (*e.g.* target knockdown), less toxicity and/or less off-target effects, regardless of the underlying mechanism (improved endosomal escape, increased 'productive' pathway uptake, or another mechanism).

An emerging strategy entails the conjugation of antisense oligonucleotides (ASOs) to receptor ligands in order to increase oligonucleotide potency and distribution to selected tissues. For instance, conjugation of triantennary N-acetyl galactosamine (GalNAc) to oligonucleotide therapeutics yields 10-30-fold increased potency in isolated hepatocytes, as well as in liver *in vivo.* GalNAc is found on damaged glycoproteins that have lost terminal sialic acid residues from their pendant oligosaccharides. The liver functions to clear these proteins from the systemic circulation by expressing trimeric asialoglycoprotein receptors (ASGPRs), preferably ASGPR1, at very high levels (order of 10⁵-10⁶ per cell) on the surface of hepatocytes. ASGPRs bind specifically to GalNAc at neutral pH for endocytosis of circulating macromolecules from the blood and release GalNAc at acidic pH (~5-6) for cargo drop-off in the early endosome. Freed ASGPRs are then recycled back to the cell surface for reuse. Approximately one-third of RNAi drugs currently in clinical trials are single- molecule, chemically modified RNAi triggers conjugated to multivalent GalNAc ligands targeting the ASGPRs. The suitable physiology of the liver, the unique properties of ASGPRs, the non- toxic nature of the GalNAc ligand and the simplicity of GalNAc-siRNA conjugates make this an attractive approach for systemic RNAi delivery to hepatocytes. WO 2015/071388 discloses a conjugate of an antisense ODN and GAL-NAc for the treatment of cancer and other diseases. H. Fuchs et al, BIOMEDICINES, vol. 5, no. 2, 2017 discloses that saponins, especially the saponin S01861, can be successfully used for enhancing the anti-tumoral activity of antibody drug conjugates with cytotoxic agents as active agent.

However, there still exists a need for improved delivery systems which further increase the potency, decrease the toxicity and/or reduce off-target effects of ASGPR targeted oligonucleotide systems (e.g. GalNAc-oligonucleotide conjugates).

### SUMMARY

The invention is defined by the claims. Any subject-matter falling outside the scope of the claims is for illustration purposes only.

A first aspect of the invention relates to a pharmaceutical combination comprising
- a conjugate of an effector molecule and a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)₃Tris; and
- a saponin, wherein the saponin is a monodesmosidic triterpene glycoside or a bidesmosidic triterpene glycoside as defined in the claims and optionally comprising a pharmaceutically acceptable excipient and/or pharmaceutically acceptable diluent.

An embodiment is the pharmaceutical combination of the invention in the form of at least two pharmaceutical compositions comprising:
- a first pharmaceutical composition comprising the conjugate and optionally comprising a pharmaceutically acceptable excipient and/or pharmaceutically acceptable diluent; and
- a second pharmaceutical composition comprising the saponin and optionally comprising a pharmaceutically acceptable excipient and/or pharmaceutically acceptable diluent.

An embodiment is the pharmaceutical combination of the invention in the form of a single pharmaceutical composition comprising the conjugate, the saponin and optionally a pharmaceutically acceptable excipient and/or pharmaceutically acceptable diluent.

A second aspect of the invention relates to the pharmaceutical combination of the invention, for use as a medicament.

A third aspect of the invention relates to the pharmaceutical combination of the invention, for use in the treatment or prophylaxis of a disease or health problem in which an expression product is involved of any one or more of genes: apoB, TTR, PCSK9, ALAS1, AT3, GO, CC5, X gene of HBV, S gene of HBV, AAT and LDH. An embodiment is the pharmaceutical combination for use according to the invention, wherein said use is in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, or an auto-immune disease.

A fourth aspect of the invention relates to an *in vitro* or *ex vivo* method for transferring an effector molecule of the invention from outside a cell to inside said cell, preferably into the cytosol of said cell, comprising the steps of:
a) providing a cell which expresses ASGPR, preferably ASGPR1, on its surface, preferably selected from a liver cell, a virally infected cell and a cancer cell;
b) providing the conjugate of the invention, the conjugate comprising the effector molecule to be transferred;
c) providing the saponin of the invention;
d) contacting the cell of step a) *in vitro* or *ex vivo* with the conjugate of step b) and the saponin of step c),
therewith effecting the transfer of said conjugate comprising the effector molecule from outside the cell to inside said cell, and by effecting the transfer of said conjugate effecting the transfer of the effector molecule from outside the cell to inside said cell, preferably into the cytosol of said cell.

A fifth aspect of the invention relates to an *in vitro* or *ex vivo* method for transferring the conjugate of the invention from outside a cell to inside said cell, preferably into the cytosol of said cell, comprising the steps of:
a) providing a cell which expresses ASGPR, preferably ASGPR1, on its surface, preferably selected from a liver cell, a virally infected cell and a cancer cell;
b) providing the conjugate of the invention;
c) providing the saponin of the invention;
d) contacting the cell of step a) *in vitro* or *ex vivo* with the conjugate of step b) and the saponin of step c),
therewith effecting the transfer of the conjugate from outside the cell to inside said cell.

A sixth aspect of the invention relates to a kit of parts, comprising the pharmaceutical combination of the invention or the second pharmaceutical composition of the invention, and instructions for use of said pharmaceutical combination or second pharmaceutical composition according to the invention or for use in an *in vitro* or *ex vivo* method according to the invention.

### DEFINITIONS

The term "GalNAc" has its regular scientific meaning and here refers to N-acetylgalactosamine and to the IUPAC name thereof: 2-(acetylamino)-2-deoxy-D-galactose.

The term "(GalNAc)₃Tris" has its regular scientific meaning in for example the field of siRNA-based therapy, and here refers to a moiety comprising three GalNAc units each separately covalently bound to the hydroxyl groups of tris(hydroxymethyl)aminomethane (Tris) (IUPAC name: 2-amino-2-(hydroxymethyl) propane-1,3-diol), preferably via at least one linker. (GalNAc)₃Tris can exist as a free amine comprising molecule or may be further functionalized via the remaining amine binding site, for example to form the (GalNAc)₃Tris-moiety comprising conjugates described herein.

The term "oligonucleotide" has its regular scientific meaning and here refers to a string of two or more nucleotides, *i.e.* an oligonucleotides is a short oligomer composed of ribonucleotides or deoxyribonucleotides. Examples are nucleic acids such as RNA and DNA, and any modified RNA or DNA, such as a string of nucleic acids comprising a nucleotide analogue such as a bridged nucleic acid (BNA), also known as locked nucleic acid (LNA) or a 2'-O,4'-C-aminoethylene or a 2'-O,4'-C-aminomethylene bridged nucleic acid (BNA^{NC}), wherein the nucleotide is a ribonucleotide or a deoxyribonucleotide.

The term "RNAi-mediated gene-targeting" has its regular scientific meaning and here refers to the *in vivo*, *ex vivo* or *in vitro* approach of influencing functioning of the gene in a cell by transferring into said cell an oligonucleotide, such as a small double-stranded RNA molecule, that targets mRNA involved in transcription of the gene: for example, small double-stranded RNA molecules can efficiently trigger RNAi silencing of specific genes.

The term "bridged nucleic acid", or "BNA" in short, or "locked nucleic acid" or "LNA" in short or 2'-0,4'-C-aminoethylene or 2'-0,4'-C-aminomethylene bridged nucleic acid (BNA^{NC}), has its regular scientific meaning and here refers to a modified RNA nucleotide. A BNA is also referred to as 'constrained RNA molecule' or 'inaccessible RNA molecule'. A BNA monomer can contain a five-membered, six-membered or even a seven-membered bridged structure with a "fixed" C₃'-endo sugar puckering. The bridge is synthetically incorporated at the 2', 4'-position of the ribose to afford a 2', 4'-BNA monomer. A BNA monomer can be incorporated into an oligonucleotide polymeric structure using standard phosphoramidite chemistry known in the art. A BNA is a structurally rigid oligonucleotide with increased binding affinity and stability.

The term antisense oligonucleotide has its regular scientific meaning and may be indicated in short in the description as "AON" or "ASO".

The term "BNA-based antisense oligonucleotide", or in short "BNA-AON", has its regular scientific meaning and here refers to a string of antisense nucleotides wherein at least one of said nucleotides is a BNA.

The term "proteinaceous" has its regular scientific meaning and here refers to a molecule that is protein-like, meaning that the molecule possesses, to some degree, the physicochemical properties characteristic of a protein, is of protein, relating to protein, containing protein, pertaining to protein, consisting of protein, resembling protein, or being a protein. The term "proteinaceous" as used in for example 'proteinaceous molecule' refers to the presence of at least a part of the molecule that resembles or is a protein, wherein 'protein' is to be understood to include a chain of amino-acid residues at least two residues long, thus including a peptide, a polypeptide and a protein and an assembly of proteins or protein domains. In the proteinaceous molecule, the at least two amino-acid residues are for example linked via (an) amide bond(s), such as (a) peptide bond(s). In the proteinaceous molecule, the amino-acid residues are natural amino-acid residues and/or artificial amino-acid residues such as modified natural amino-acid residues. In a preferred embodiment, a proteinaceous molecule is a molecule comprising at least two amino-acid residues, preferably between two and about 2.000 amino-acid residues. In one embodiment, a proteinaceous molecule is a molecule comprising from 2 to 20 (typical for a peptide) amino acids. In one embodiment, a proteinaceous molecule is a molecule comprising from 21 to 1.000 amino acids (typical for a polypeptide, a protein, a protein domain, such as an antibody, a single domain antibody, a Fab, an scFv, a ligand for a receptor such as EGF). Preferably, the amino-acid residues are (typically) linked via (a) peptide bond(s). According to the invention, said amino-acid residues are or comprise (modified) (non-)natural amino acid residues.

The term "effector molecule", or "effector moiety" when referring to the effector molecule as part of *e.g.* a covalent conjugate, has its regular scientific meaning and here refers to a molecule that can selectively bind to for example any one or more of the target molecules: a protein, a peptide, a carbohydrate, a saccharide such as a glycan, a (phospho)lipid, a nucleic acid such as DNA, RNA, an enzyme, and regulates the biological activity of such one or more target molecule(s). The effector molecule is for example a molecule selected from any one or more of a small molecule such as a drug molecule, a toxin such as a protein toxin, an oligonucleotide such as a BNA, a xeno nucleic acid or an siRNA, an enzyme, a peptide, a protein, or any combination thereof. Thus, for example, an effector molecule or an effector moiety is a molecule or moiety selected from any one or more of a small molecule such as a drug molecule, a toxin such as a protein toxin, an oligonucleotide such as a BNA, a xeno nucleic acid or an siRNA, an enzyme, a peptide, a protein, or any combination thereof, that can selectively bind to any one or more of the target molecules: a protein, a peptide, a carbohydrate, a saccharide such as a glycan, a (phospho)lipid, a nucleic acid such as DNA, RNA, an enzyme, and that upon binding to the target molecule regulates the biological activity of such one or more target molecule(s). Typically, an effector molecule can exert a biological effect inside a cell such as a mammalian cell such as a human cell, such as in the cytosol of said cell. Typical effector molecules are thus drug molecules, plasmid DNA, toxins such as toxins comprised by antibody-drug conjugates (ADCs), oligonucleotides such as siRNA, BNA, nucleic acids comprised by an antibody-oligonucleotide conjugate (AOC). For example, an effector molecule is a molecule which can act as a ligand that can increase or decrease (intracellular) enzyme activity, gene expression, or cell signalling.

The term "health problem" has its regular scientific meaning and here refers to any condition of the body of a subject such as a human patient, or of a part, organ, muscle, vein, artery, skin, limb, blood, cell, *etc.* thereof, that is suboptimal when compared to the condition of that body or part thereof in a healthy subject, therewith hampering the proper functioning and/or well-being of the subject, *e.g.* impairs normal functioning of a human body.

The term "GalNAc-decorated oligonucleotide drug" has its regular scientific meaning and here refers to an oligonucleotide for interfering in transcription of a gene, wherein one or more GalNAc units are coupled to the oligonucleotide, *e.g.* via at least one linker.

The term "locked nucleic acid", in short "LNA" has its regular scientific meaning and here refers to an oligonucleotide that contains one or more nucleotide building blocks in which an additional methylene bridge fixes the ribose moiety either in the C3'-endo conformation (beta-D-LNA) or C2'-endo (alpha-L-LNA) conformation, as is known in the art.

The term "bridged nucleic acid NC", in short "BNA^{NC}" has its regular scientific meaning and here refers to an oligonucleotide that contains one or more nucleotide building blocks in which an additional 2'-O,4'-C-aminoethylene bridged nucleic acid is comprised, with different substitutions at the N atom (of which a methyl group is the most commonly used to date), as is known in the art.

The term "chemically modified, metabolically stable siRNA" has its regular scientific meaning and here refers to an siRNA molecule comprising chemical modifications compared to the RNA oligonucleotide consisting of naturally occurring nucleotides, such that the modified siRNA molecule is more resistant towards metabolic degradation, digestion, enzymatic lysis, *etc.*, *i.e.* more stable.

The term "saponin" has its regular scientific meaning and here refers to a group of amphipatic glycosides which comprise one or more hydrophilic glycone moieties combined with a lipophilic aglycone core which is a sapogenin. The saponin may be naturally occurring or synthetic (*i.e.* non-naturally occurring). The term "saponin" includes naturally occurring saponins, derivatives of naturally occurring saponins as well as saponins synthesized *de novo* through chemical and/or biotechnological synthesis routes.

The term "saponin derivative" (also known as "modified saponin") has its regular scientific meaning and here refers to a compound corresponding to a naturally-occurring saponin which has been derivatised by one or more chemical modifications, such as the oxidation of a functional group, the reduction of a functional group and/or the formation of a covalent bond with another molecule (also referred to as "conjugation" or as "covalent conjugation"). Preferred modifications include derivatisation of an aldehyde group of the aglycone core; of a carboxyl group of a saccharide chain or of an acetoxy group of a saccharide chain. Typically, the saponin derivative does not have a natural counterpart, *i.e.* the saponin derivative is not produced naturally by *e.g.* plants or trees. The term "saponin derivative" includes derivatives obtained by derivatisation of naturally-occurring saponins as well as derivatives synthesized *de novo* through chemical and/or biotechnological synthesis routes resulting in a compound corresponding to a naturally occurring saponin which has been derivatised by one or more chemical modifications.

The term "aglycone core structure" has its regular scientific meaning and here refers to the aglycone core, or aglycone, of a saponin without the one or two carbohydrate antenna or saccharide chains (glycans, glycones) bound thereto. For example, quillaic acid is the aglycone core structure for SO1861, QS-7 and QS-21.

The term "saccharide chain" has its regular scientific meaning and here refers to any of a glycan, a carbohydrate antenna, a glycone, a single saccharide moiety (monosaccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

The term "antibody-drug conjugate" or "ADC" has its regular scientific meaning and here refers to any conjugate of an antibody such as an IgG, a Fab, an scFv, a single domain antibody, an immunoglobulin, an immunoglobulin fragment, one or multiple Vh domains, *etc.,* with any molecule that can exert a therapeutic effect when contacted with cells of a subject such as a human patient, such as an active pharmaceutical ingredient, a toxin, an oligonucleotide, an enzyme, a small molecule drug compound, *etc.*

The term "antibody-oligonucleotide conjugate" or "AOC" has its regular scientific meaning and here refers to any conjugate of an antibody such as an IgG, a Fab, a single domain antibody, an scFv, an immunoglobulin, an immunoglobulin fragment, one or multiple Vh domains, *etc.,* and any oligonucleotide molecule that can exert a therapeutic effect when contacted with cells of a subject such as a human patient, such as an oligonucleotide selected from a natural or synthetic string of nucleic acids encompassing DNA, modified DNA, RNA, modified RNA, synthetic nucleic acids, presented as a single-stranded molecule or a double-stranded molecule, such as a BNA, an allele-specific oligonucleotide, a short or small interfering RNA (siRNA; silencing RNA), an anti-sense DNA, anti-sense RNA, *etc.*

The term "conjugate" has its regular scientific meaning and here refers to at least a first molecule that is covalently bound through (a) chemical bond(s) to at least a second molecule, therewith forming an covalently coupled assembly comprising or consisting of the first molecule and the second molecule. Typical conjugates are (GalNAc)₃Tris - siRNA, an ADC, an AOC, and SO1861-EMCH (EMCH linked to the aldehyde group of the aglycone core structure of the saponin).

The term 'S' as used such as in a GalNAc - effector molecule conjugate or construct comprising a linker, represents 'stable linker' which remains intact in the endosome and in the cytosol.

The term 'L' as used such as in an antibody-saponin conjugate or construct comprising a linker, represents 'labile linker' which is cleaved under slightly acid conditions in the endosome.

The term "moiety" has its regular scientific meaning and here refers to a molecule that is bound, linked, conjugated to a further molecule, linker, assembly of molecules, *etc.*, and therewith forming part of a larger molecule, conjugate, assembly of molecules. Typically, a moiety is a first molecule that is covalently bound to a second molecule, involving one or more chemical groups initially present on the first molecule and present on the second molecule. For example, saporin is a typical molecule, and is an example of an effector molecule. As part of an antibody-drug conjugate, the saporin is a typical effector moiety in the ADC. As part of an antibody-oligonucleotide conjugate, a BNA or an siRNA is a typical effector moiety in the AOC.

The term "DAR" normally stands for *Drug Antibody Ratio* and refers to the average *drug* to *antibody ratio* for a given preparation of *antibody drug* conjugate (ADC) and here refers to a ratio of the amount of bound SO1861 moieties, or SPT001, or bound ApoBBNA with respect to the conjugate molecule.

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments of the invention described herein can operate in combination and cooperation, unless specified otherwise.

Furthermore, the various embodiments, although referred to as "preferred" or *"e.g."* or "for example" or "in particular" and the like are to be construed as exemplary manners in which the invention may be implemented rather than as limiting the scope of the invention.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

### BRIEF DESCRIPTION OF THE DRAWINGS

**Figure 1****:** synthesis of trivalent-GalNAc.
**Figure 2****:** synthesis of SO1861-DBCO.
**Figure 3****:** synthesis of monovalent-GalNAc-SO1861.
**Figure 4****:** synthesis of trivalent-GalNAc-SO1861.

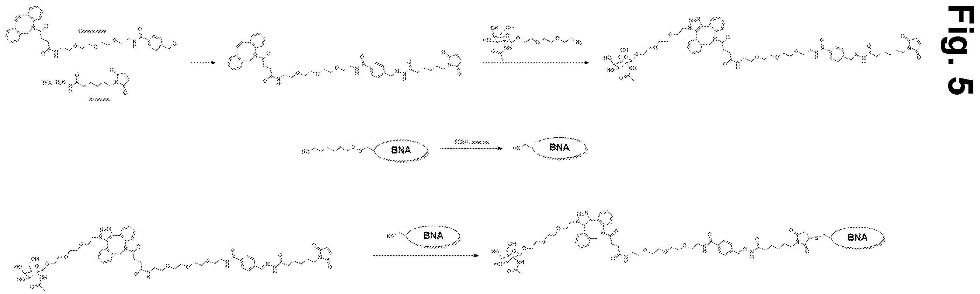

**Figure 5****:** synthesis of monovalent-GalNAc-BNA.
**Figure 6****:** synthesis of trivalent-GalNAc-BNA.
**Figure 7****:** synthesis of trivalent-GalNAc-BNA.
**Figure 8A** **and** **8B****:** synthesis of trivalent GalNAc.
**Figure 9A and 9B****:** Cell viability (MTS) of GalNAc-SO1861 and trivalent-GalNAc-SO1861 on HepG2 (A) and Huh7 (B) cell lines. The legend displayed next to Figure 9B also applies for Figure 9A.
**Figure 10A and 10B****:** Cell viability assay (MTS) HepG2 (A) and Huh7 (B) cell lines. The legend displayed next to Figure 10B also applies for Figure 10A.
**Figure 11A and 11B****:** Gene expression analysis on HepG2 (A) and Huh7 (B) cell lines.
**Figure 12A and 12B****:** Gene expression analysis on HepG2 (A) and Huh7 (B) cell lines. The legend displayed next to Figure 12B also applies for Figure 12A.
**Figure 13A and 13B****:** Cell viability assay on HepG2 (A) and Huh7 (B) cell lines. The legend displayed next to Figure 13B also applies for Figure 13A.
**Figure 14A** **and B:** Gene expression analysis on HepG2 (A) and Huh7 (B) cell lines. The legend displayed next to Figure 14B also applies for Figure 14A.
**Figure 15A** **and B:** Cell viability assay on HepG2 (A) and Huh7 (B) cell lines. The legend displayed next to Figure 15B also applies for Figure 15A.
**Figure 16A** **and B:** Gene expression analysis on HepG2 (A) and Huh7 (B) cell lines. The legend displayed next to Figure 16B also applies for Figure 16A.
**Figure 17A** **and B:** Gene expression analysis on HepG2 (A) and Huh7 (B) cell lines. The legend displayed next to Figure 17B also applies for Figure 17A.
**Figure 18A** **and B:** ApoB RNA expression analysis and cell viability assay on human primary hepatocytes.
**Figure 19A** **and B:** ApoB RNA expression analysis and cell viability assay on human primary hepatocytes.
**Figure 20A and B:** ApoB RNA expression analysis assay on mouse primary hepatocytes.
**Figure 21****:** (GalNAc)3-Ls-SO1861 synthesis, intermediate 22, 23.
**Figure 22****:** (GalNAc)3-Ls-BNA synthesis, intermediate 25, 26.
**Figure 23****:** (GalNAc)3-Ls-BNA synthesis.

### DETAILED DESCRIPTION

It is a first goal of the present invention to provide an improved therapeutic combination or an improved pharmaceutical composition for delivery of an effector molecule coupled to a ligand for ASGPR, in particular ASGPR1, such as a GalNAc comprising ligand, when for example the delivery from outside a target cell into said cell is considered, or more in particular when the delivery of the effector molecule in the cytosol of said target cell is considered. It is a second goal of the present invention to provide an improved method of treatment of a (human) patient suffering from a disease to be treated with a conjugate comprising an effector molecule and a ligand for ASGPR.

It is an objective of the current invention to provide a therapeutic composition or a therapeutic combination of *e.g.* two therapeutic compositions, comprising a conjugate of a ligand for ASGPR covalently linked to an effector molecule that is capable of exerting a biological effect once delivered inside a target cell which comprises the molecular target for the effector molecule, which conjugate, when administered to a (human) patient in need thereof, bearing the target cell, has an improved therapeutic effect or a sufficient effect at a lower dose than the currently required dose for the conjugate. This way, the therapeutic window of the conjugate is widened effectively.

At least one of the above objectives is achieved by providing the therapeutic combination of at least two pharmaceutical compositions or the pharmaceutical composition of the invention.

The present invention will be described with respect to particular embodiments but the invention is not limited thereto but only by the claims.

A first aspect of the invention relates to a pharmaceutical combination comprising:
- a conjugate of an effector molecule and a ligand for asialoglycoprotein receptor (ASGPR), wherein the ligand for ASGPR comprises at least one *N*-acetylgalactosamine (GalNAc) moiety, preferably three or four GalNAc moieties, more preferably the ligand for ASGPR comprises or consists of (GalNAc)₃Tris; and
- a saponin, wherein the saponin is a monodesmosidic triterpene glycoside or a bidesmosidic triterpene glycoside, as defined in the claims and optionally comprising a pharmaceutically acceptable excipient and/or pharmaceutically acceptable diluent.

An embodiment is the pharmaceutical combination of the invention in the form of at least two pharmaceutical compositions comprising:
- a first pharmaceutical composition comprising the conjugate and optionally comprising a pharmaceutically acceptable excipient and/or pharmaceutically acceptable diluent; and
- a second pharmaceutical composition comprising the saponin and optionally comprising a pharmaceutically acceptable excipient and/or pharmaceutically acceptable diluent.

An embodiment is the pharmaceutical combination of the invention in the form of a single pharmaceutical composition comprising the conjugate, the saponin and optionally a pharmaceutically acceptable excipient and/or pharmaceutically acceptable diluent.

The inventors surprisingly found that cells which express ASGPR, in particular ASGPR1 at their surface, exhibit improved uptake of a conjugate comprising a ligand for ASGPR and an effector molecule, when said cells are contacted with both the conjugate and a saponin of the triterpene glycoside type, typically a bidesmosidic terpenoid type of saponin. For example, GalNAc based ligands for ASGPR1, such as a (GalNAc)₃Tris conjugate, covalently conjugated with an oligonucleotide, such as a gene silencing BNA, for example for silencing HSP27 exhibit such improved uptake in the presence of a saponin. Herewith, the inventors now provide a solution for the long-felt need for improving the efficacy of delivery of *e.g.* oligonucleotides from outside a target cell to be treated with such an oligonucleotide, to inside said cell, in particular for improving the intracellularly delivery into the cytosol of a target cell that expresses ASGPR1 on its surface, of an oligonucleotide such as a BNA and/or an siRNA. The applied saponins according to the invention are known for their ability to enhance the endosomal escape of e.g. protein toxins once a target cell is contacted with such a saponin and the toxin, wherein the toxin is for example part of an antibody-drug conjugate (ADC). The inventors now also established that certain derivatives of such saponins maintain their capability of enhancing the escape of effector molecules from endosomes and lysosomes into the cytosol, when the saponins and the effector molecule co-localize inside the cell, *i.e.* in the endosome. Surprisingly, such saponin derivatives are capable of enhancing the delivery of the effector molecule comprised by the conjugate into a target cell bearing the ASGPR1, while the cytotoxicity and the haemolytic activity of such saponin derivates are lower than observed for the (naturally occurring) non-modified saponin. Such saponin derivatives are described in more detail herein.

### Conjugate - introduction

The pharmaceutical combination and certain pharmaceutical compositions of the present invention comprise a conjugate of an effector molecule and a ligand for asialoglycoprotein receptor (ASGPR) such as ASGPR1, and preferably ASGPR1. When the effector molecule is bound to the remainder of the conjugate it is referred to herein as an "effector moiety". The ASGPR ligand comprises at least one N-acetylgalactosamine (GalNAc) moiety. In accordance with embodiments of the present invention each GalNAc moiety is bound to the remainder of the ASGPR ligand or - in case an ASGPR ligand consists of a single GalNAc moiety - to the effector moiety, via a covalent bond to the oxygen on position "1" as indicated in formula (I):

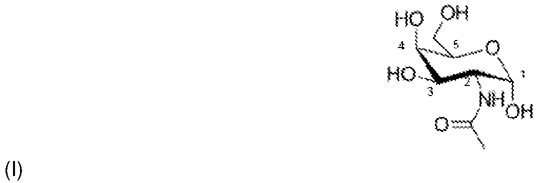

As shown in formula (II), the ASGPR ligand consists of a single GalNAc moiety bound to the effector moiety E, preferably via an effector moiety linker L_{E}.

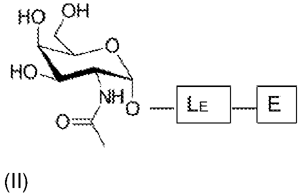

The ASGPR ligand may comprise more than one GalNAc moiety, such as 2, 3, 4, 5 or 6 GalNAc moieties, preferably 3 or 4 GalNAc moieties, more preferably 3 GalNAc moieties. In such case, it is preferred that the GalNAc moieties are each separately covalently bound via the oxygen on position "1" to a central bridging moiety B, which effectively forms a bridge between the GalNAc moieties and the effector moiety, preferably via an effector moiety linker L_{E}. The GalNAc moieties may be directly bound to the bridging moiety B as shown in formula (III): 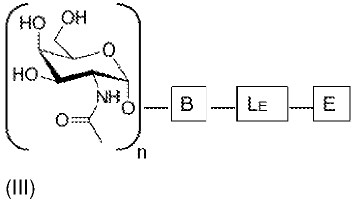 wherein n is an integer larger than or equal to 3 or 4, preferably n is 3, L_{E} is an effector moiety linker and E is the effector moiety. More preferably, the GalNAc moieties are bound to the bridging moiety B via GalNAc linkers L_{GAL} as shown in formula (IV): 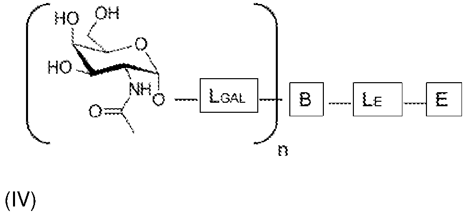 wherein n is an integer larger than or equal to 3 or 4, preferably n is 3, L_{E} is an effector moiety linker and E is the effector moiety. While each occurrence of L_{GAL} may be independently chosen, the skilled person will appreciate that the synthesis of the conjugate is simplified in case each occurrence of L_{GAL} represents the same moiety.

### Conjugate - Linker L_{E}

The effector moiety linker L_{E} represents any chemical moiety suitable for covalently binding an effector moiety to GalNAc as in formula (II) or to the bridging moiety B as in formula (III) and (IV). The identity and size of the linker is not particularly limited and covalently binding an effector molecule to GalNAc as in formula (II) or to the bridging moiety B as in formula (III) and (IV) may be effected by means of a 'regular' chemical functional group (*e.g.* an ether bond) as well as through "click-chemistry" type linkers which typically have a long chain length, resulting in a linker E_{L} comprising *e.g.* more than 10 or more than 20 carbon atoms. Suitable effector moiety linkers L_{E} and associated coupling reactions are described in the handbook Hermanson, Greg T. Bioconjugate techniques. Academic press, 2013.

As will be understood by the skilled person, the effector moiety linker L_{E} will typically be the result of a coupling reaction (*e.g.* "click-chemistry" type) between at least a first precursor L_{E1} covalently bound to GalNAc or the bridging moiety B and a second precursor L_{E2} covalently bound to the effector moiety. This principle is illustrated in the following reaction scheme for the compound of formula (II): 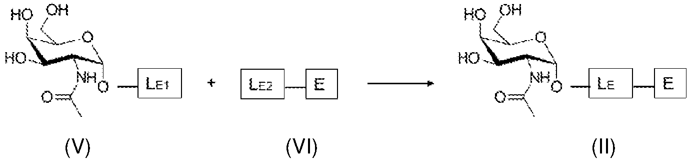 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety and E is the effector moiety.

The corresponding reaction scheme for the compound of formula (III) is as follows: 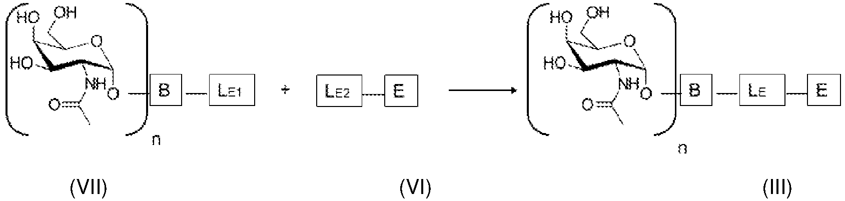 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety, n is an integer larger than or equal to 3 or 4, preferably n is 3, B is a bridging moiety and E is the effector moiety.

The corresponding reaction scheme for the compound of formula (IV) is as follows: 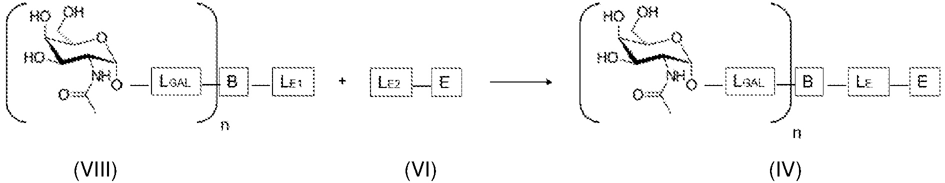 wherein L_{E} is an effector moiety linker, L_{E1} is a precursor of the effector moiety linker L_{E} which is covalently bound to GalNAc and L_{E2} is a precursor of the effector moiety linker L_{E} which is covalently bound to the effector moiety, n is an integer larger than or equal to 3 or 4, preferably n is 3, E is the effector moiety, and L_{GAL} is a GalNAc linker and B is a bridging moiety.

The effector moiety linker L_{E} can be the result of a coupling reaction between at least a first precursor L_{E1} covalently bound to GalNAc or the bridging moiety and a second precursor L_{E2} covalently bound to the effector moiety, wherein the coupling reaction is for example an azide-alkyne cycloaddition, a thiol maleimide coupling, a staudinger reaction, a nucleophilic ring-opening of strained heterocyclic electrophiles (such as aziridines, epoxides, cyclic sulfates, aziridinium ions, episulfonium ions), a carbonyl reaction of the non-aldol type (such as urea, thiourea, hydrazon, oxime ether, amide or aromatic heterocycle formation) or an addition to a carbon-carbon double bond (such as epoxidation, aziridination, dihydroxylation, sulfentyl halide addition, nitrosyl halide addition or micheal addition), preferably wherein the coupling reaction is an azide-alkyne cycloaddition, a thiol maleimide coupling, a staudinger reaction, a nucleophilic ring-opening of strained heterocyclic electrophiles, more preferably wherein the coupling reaction is an azide-alkyne cycloaddition or a thiol maleimide coupling.

In accordance with some embodiments of the invention, the effector moiety linker L_{E} comprises a succinimide thioether moiety. Such a succinimide thioether is *e.g.* the result of a thiol maleimide coupling between an N-substituted maleimide and a thiol or sulfhydryl containing compound. This thiol maleimide coupling is a commonly known 'click'-chemistry tool in the field of bioconjugation and is for example described in Hermanson, Greg T. Bioconjugate techniques. Academic press, 2013 page 289. Consequently, it is preferred that the effector moiety linker L_{E} is the result of a coupling reaction between a first precursor L_{E1} covalently bound to GalNAc or the bridging moiety, the first precursor L_{E1} comprising an N-substituted maleimide; and a second precursor L_{E2} covalently bound to the effector moiety, the second precursor L_{E2} comprising a thiol or a precursor thereof. A suitable thiol precursor is a disulfide, which may be cleaved (e.g. in-situ) via reduction to the corresponding thiol.

A preferred structure for the precursor L_{E1} present in the compounds of formula (V), (VII) or (VIII) is the following terminal N-substituted maleimide: 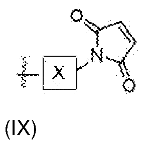 wherein X represents any linker suitable for covalently bonding the terminal N-substituted maleimide to GalNAc or the bridging moiety B. As will be understood by the skilled person, X may be the result of a coupling reaction between a first moiety covalently bound to GalNAc or the bridging moiety and a second moiety covalently bound to the maleimide. X can comprise a hydrazone and/or a 1, 2, 3-triazole. Whenever reference is made to a 1, 2, 3-triazole in the context of the linkers of the present application, this preferably means a 1*H*-1, 2, 3-triazole.

Embodiments of the structure for the precursor L_{E1} present in the compounds of formula (V), (VII) or (VIII) are the following terminal N-substituted maleimides: 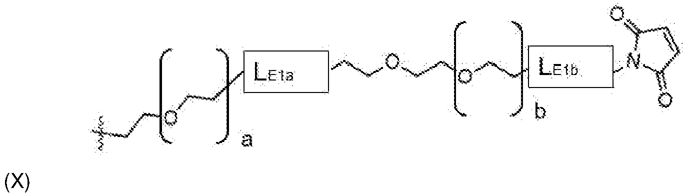 wherein a and b each independently represent an integer larger than or equal to 0, preferably a and b each independently represent an integer selected from 0, 1, 2, and 3, more preferably a and b represent 2, and wherein L_{E1a} represents a hydrazone and/or a 1, 2, 3-triazole, preferably a 1, 2, 3-triazole and wherein L_{E1b} represents a hydrazone and/or a 1, 2, 3-triazole, preferably a hydrazone; 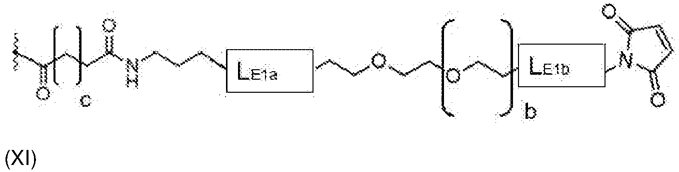 wherein b and c each independently represent an integer larger than or equal to 0, preferably b represents an integer selected from 0, 1, 2, and 3 and c represents an integer in the range of 5-15, more preferably b represents 2, and c represents 9, wherein L_{E1a} represents a hydrazone and/or a 1, 2, 3-triazole, preferably a 1, 2, 3-triazole and wherein L_{E1b} represents a hydrazone and/or a 1, 2, 3-triazole, preferably a hydrazone;
and 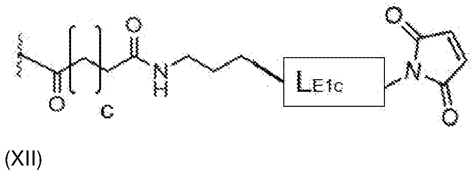 wherein c represents an integer larger than or equal to 0, preferably c represents an integer in the range of 5-15, more preferably c represents 9, wherein L_{E1c} represents a hydrazone and/or a 1, 2, 3-triazole, preferably a 1, 2, 3-triazole.

L_{E1a}, L_{E1b} and L_{E1c} each preferably comprise less than 20 carbon atoms, more preferably L_{E1a}, L_{E1b} and L_{E1c} each independently represent a moiety according to formula (XIII), (XIV) or (XV), preferably L_{E1a} is the 1,2,3-triazole of formula (XIII), L_{E1b} is the hydrazone of formula (XIV) and L_{E1c} is the 1,2,3-triazole of formula (XV) :

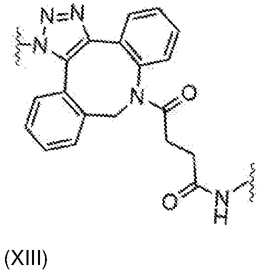

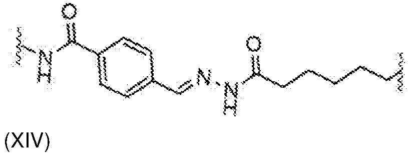

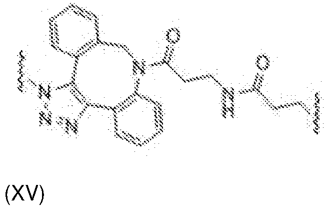

Hence, in some embodiments the conjugate is a compound of formula (II), (III) or (IV) as described herein, wherein the effector moiety linker L_{E} is the result of a coupling reaction between a compound of formula (V), (VII) or (VIII) with a compound of formula (VI), wherein the first precursor L_{E1} is a terminal N-substituted maleimide of formula (X), (XI) or (XII), wherein L_{E1a} is for example the 1,2,3-triazole of formula (XIII), L_{E1b} is for example the hydrazone of formula (XIV) and L_{E1c} is for example the 1,2,3-triazole of formula (XV).

### Conjugate - Bridging moiety B

The bridging moiety B present in compounds of formula (III) or (IV) represents any moiety suitable for covalently binding 2 or more GalNAc moieties, preferably 3 GalNac moieties, and the effector moiety linker L_{E}.

According to preferred embodiments, the bridging moiety B is a compound of formula (XV): 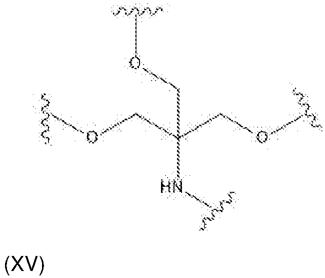 wherein the oxygen atoms of the compound of formula (XV) are bound to GalNAc (corresponding to compounds of formula (III)) or to the GalNAc linkers L_{GAL} (corresponding to compounds of formula (IV)), and the nitrogen atom of the compound of formula (XV) is bound to the effector moiety linker L_{E}.

The skilled person will understand that these compounds of formula (III) or (IV) wherein the bridging moiety B is a compound of formula (XV) correspond to conjugates wherein the ASGPR ligand consists of (GalNAc)₃Tris.

### Conjugate - Linker L_{GAL}

The GalNAc linkers L_{GAL} represent any chemical moiety suitable for covalently binding GalNAc to the bridging moiety as in formula (IV). The identity and size of the linker is not particularly limited.

According to embodiments, the GalNAc linkers L_{GAL} each comprise 2-25 carbon atoms, preferably 7-15 carbon atoms, more preferably 11 carbon atoms. Preferably the GalNAc linkers L_{GAL} comprise at least one, preferably two amide moieties. A particularly preferred GalNAc linker L_{GAL} is a compound according to formula (XVI):

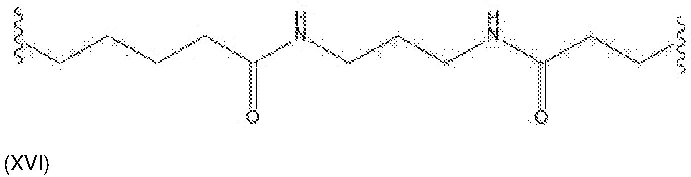

### Conjugate - Effector moiety

An embodiment is the pharmaceutical combination of the invention, wherein the effector molecule comprises or consists of at least one of a small molecule such as a drug molecule, a toxin such as a protein toxin, an oligonucleotide such as a BNA, a xeno nucleic acid or an siRNA, an enzyme, a peptide, a protein, or any combination thereof, preferably, the effector molecule is a toxin, an enzyme or an oligonucleotide. According to the invention, the effector molecule is a molecule capable of exerting a biological effect inside a cell that expresses the ASGPR on its surface and that comprises the binding partner for the effector molecule inside the cell, *e.g.* in the cytosol of said cell, such that the effector molecule can exert its intracellular biological effect once delivered inside the cell and preferably inside the cytosol, i.e. at the location inside the cell where the target for the effector molecule resides.

An embodiment is the pharmaceutical combination of the invention, wherein the ligand for ASGPR, in particular a ligand for ASGPR1, and the effector molecule, preferably a toxin or an oligonucleotide, are conjugated via a covalent bond, preferably via at least one linker. Such linkers suitable for coupling of the ligand for ASGPR, such as (GalNAc)₃Tris, to an effector molecule, such as a BNA or an siRNA or a protein toxin, *etc.,* are detailed here above.

An embodiment is the pharmaceutical combination of the invention, wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), mRNA, DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON). As said, any effector molecule for which the target binding partner is present inside a cell that expresses the receptor for the ligand comprised by the conjugate of the invention, i.e. ASGPR, is suitably selected for incorporation in the conjugate of the invention. Thus, for example an ASO, an siRNA or a BNA capable for targeting a gene, mRNA, *etc.,* present in the cell which exposes ASGPR at its surface, are suitable candidates for incorporation in the conjugate of the invention.

An embodiment is the pharmaceutical combination of the invention, wherein the effector molecule is an oligonucleotide selected from any one of: an anti-miRNA, a BNA-AON or an siRNA, such as BNA-based siRNA, preferably selected from chemically modified siRNA, metabolically stable siRNA and chemically modified, metabolically stable siRNA. Such chemically modified and/or metabolically stable siRNA moieties are known in the art, and are suitable for coupling to a ligand for ASGPR.

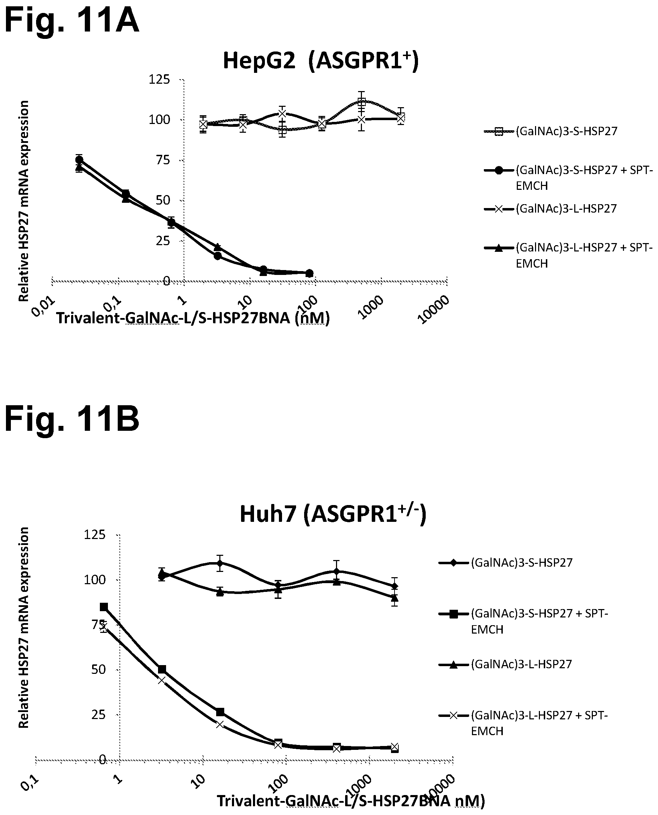

An embodiment is the pharmaceutical combination of the invention, wherein the effector molecule is an oligonucleotide that is capable of silencing any one of genes: HSP27, apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is capable of targeting an aberrant miRNA. An embodiment is the pharmaceutical combination of the invention, wherein the effector molecule, when present inside a cell, is an oligonucleotide that is capable of silencing any one of genes: apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or wherein the effector molecule, when present inside a cell, is capable of targeting an aberrant miRNA. According to the invention, any gene or mRNA present in a cell that expresses ASGPR at its cell surface can effectively be targeted by an effector molecule comprised by the conjugate of the invention, wherein the effector molecule is *e.g.* a BNA, an siRNA, an ASO, *etc.,* when for example co-administered together with a saponin according to the invention, to a subject in need of modification of *e.g.* a gene, (level of) mRNA expression, silencing of a gene, *etc.* Under influence of the saponin, the therapeutic window of the conjugate comprising the effector molecule and the ligand for ASGPR is widened, resulting in a stronger biological effect of the effector molecule inside the target cell, and/or resulting in a biological effect of the effector molecule at a lower effective dose than what would be achieved when the target cells are exposed to the conjugate in the absence of the saponin or the saponin derivative. According to the invention, contacting the target cell which expresses ASGPR with a conjugate comprising a ligand for ASGPR and an effector molecule together with a saponin or a saponin derivative, can result in overcoming a threshold with regard to intracellular activity of the effector molecule. A dose of the conjugate that for example would be required for reaching a sufficiently high amount of effector molecule in the cytosol of a target cell in the absence of saponin, could be too high for safe administration to a patient in need of such treatment with the effector molecule. In the presence of the saponin, the effector molecule already can exert its biological effect in the cytosol of a target cell expressing the ASGPR at lower dose, which lower dose would not exert any effect inside the cell when target cells are exposed to such lower dose in the absence of saponin. For example, BNA for silencing HSP27 in (liver) cells did not silence HSP27 inside ASGPR bearing cells when a conjugate of GalNAc ligand for ASGPR and the BNA was contacted with ASGPR bearing cells, whereas when the same cells were contacted with the same or lower dose of the conjugate in the presence of saponin or saponin derivative such as a saponin with linker EMCH conjugated to the aldehyde group in the aglycone of the saponin, HSP27 was effectively silenced in the target cell (as shown in the appended example 2 and Figure 11). Thus, the inventors now provide molecules, pharmaceutical combinations, pharmaceutical compositions and a method for treating a (human) subject with such molecules, combinations and compositions, such that effector molecules can be administered to a patient in need thereof at lower dose, or such that effector molecules with an improved therapeutic window can be administered, resulting in an improved therapeutic effect once the effector molecule reaches its intracellular target in a target cell for the conjugates according to the invention.

An embodiment is the pharmaceutical combination of the invention, wherein the effector molecule is an oligonucleotide that, for example when present inside a mammalian cell, is capable of targeting an mRNA involved in expression of any one of proteins: HSP27, apoB, TTR, PCSK9, ALAS1, AT3, GO, CC5, expression product of X gene of HBV, expression product of S gene of HBV, AAT and LDH, or is capable of antagonizing or restoring an miRNA function such as inhibiting an oncogenic miRNA (onco-miR) or suppression of expression of an onco-miR, for example when present inside a mammalian cell. Such proteins are typically involved in diseases for which *e.g.* silencing of the gene involved in expression of such a protein would result in relief from disease or disease symptoms to the benefit of the patient treated with the combination of the saponin and the conjugate comprising the ligand for ASGPR and the effector molecule capable of targeting mRNA involved in expression of any one of these proteins. Thus, it is part of the invention that the combinations and compositions of the invention, comprising saponin, are suitable for including in RNAi based treatment regimens. RNAi enables target gene silencing by cleaving mRNA or repressing mRNA translation. The saponin aids in potentiating the gene silencing effector molecule by improving the cytosolic delivery of the effector molecule, when a target cell selected for RNAi therapy and expressing the ASGPR is contacted with both the saponin and the conjugate as described herein.

An embodiment is the pharmaceutical combination of the invention, wherein the effector molecule is a toxin which comprises or consists of at least one proteinaceous molecule, preferably selected from any one or more of a peptide, a protein, an enzyme such as urease and Cre-recombinase, a proteinaceous toxin, a ribosome-inactivating protein, a protein toxin selected from Table A5 and/or a bacterial toxin, a plant toxin, more preferably selected from any one or more of a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin, and/or comprises or consists of at least one of a toxin targeting ribosome, a toxin targeting elongation factor, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof. The inventors established that the combination of the invention and the compositions of the invention are equally suitable for improved delivery of nucleic acid based effector molecules, when comprised by the conjugate of the ASGPR ligand and the effector molecule, as for cytosolic delivery of effector molecules such as peptides and proteins, when such an effector molecule as part of the conjugate is contacted with target cell expressing the ASGPR, in the presence of saponin or a saponin derivative. For example, the therapeutic window for protein toxins is widened when contacting target cells with the conjugate comprising the effector molecule, *i.e.* the protein toxin, and the ligand for ASGPR, and with the saponin, according to the invention. The toxin exerts its intracellular effect to a higher extent or at a lower dose, when the target cells are exposed to the saponin and to the conjugate comprising the toxin, compared to the intracellular effect achieved at the same toxin dose or at the same lower toxin dose in the absence of the saponin, or a saponin derivative.

### Saponin

The saponin is a monodesmosidic triterpene glycoside or a bidesmosidic triterpene glycoside comprising an aglycone core structure selected from quillaic acid and gypsogenin, preferably the saponin comprises an aglycone core structure selected from quillaic acid and gypsogenin or derivatives thereof, more preferably the saponin aglycone core structure is quillaic acid or a derivative thereof. Such preferred aglycones comprise an aldehyde group at the C-4 atom. Without wishing to be bound by any theory, such an aldehyde group, or a derivative thereof as described herein elsewhere, contributes to the endosomal escape enhancing activity of saponins of the triterpene glycoside type, comprising an aglycone selected from Group C, especially quillajic acid and gypsogenin.

An embodiment is the saponin conjugate of the invention, wherein the at least one saponin is a monodesmosidic or bi-desmosidic triterpene saponin belonging to the type of a 12,13-dehydrooleanane with the aldehyde group in position C-4 and optionally comprising a glucuronic acid group in a carbohydrate substituent at the C-3beta-OH group of the saponin, preferably a bi-desmosidic triterpene saponin belonging to the type of a 12,13-dehydrooleanane with the aldehyde group in position C-4 and comprising a glucuronic acid group in a carbohydrate substituent at the C-3beta-OH group of the saponin.

An embodiment is the pharmaceutical combination of the invention, wherein
- the saponin comprises a saccharide chain bound to the aglycone core structure, which is selected from group A:
   GlcA-,
   Glc-,
   Gal-,
   Rha-(1→2)-Ara-,
   Gal-(1→2)-[Xyl-(1→3)]-GlcA-,
   Glc-(1→2)-[Glc-(1→4)]-GlcA-,
   Glc-(1→2)-Ara-(1→3)-[Gal-(1→2)]-GlcA-,
   Xyl-(1→2)-Ara-(1→3)-[Gal-(1→2)]-GlcA-,
   Glc-(1→3)-Gal-(1→2)-[Xyl-(1→3)]-Glc-(1→4)-Gal-,
   Rha-(1→2)-Gal-(1→3)-[Glc-(1→2)]-GlcA-,
   Ara-(1→4)-Rha-(1→2)-Glc-(1→2)-Rha-(1→2)-GlcA-,
   Ara-(1→4)-Fuc-(1→2)-Glc-(1→2)-Rha-(1→2)-GlcA-,
   Ara-(1→4)-Rha-(1→2)-Gal-(1→2)-Rha-(1→2)-GlcA-,
   Ara-(1→4)-Fuc-(1→2)-Gal-(1→2)-Rha-(1→2)-GlcA-,
   Ara-(1→4)-Rha-(1→2)-Glc-(1→2)-Fuc-(1→2)-GlcA-,
   Ara-(1→4)-Fuc-(1→2)-Glc-(1→2)-Fuc-(1→2)-GlcA-,
   Ara-(1→4)-Rha-(1→2)-Gal-(1→2)-Fuc-(1→2)-GlcA-,
   Ara-(1→4)-Fuc-(1→2)-Gal-(1→2)-Fuc-(1→2)-GlcA-,
   Xyl-(1→4)-Rha-(1→2)-Glc-(1→2)-Rha-(1→2)-GlcA-,
   Xyl-(1→4)-Fuc-(1→2)-Glc-(1→2)-Rha-(1→2)-GlcA-,
   Xyl-(1→4)-Rha-(1→2)-Gal-(1→2)-Rha-(1→2)-GlcA-,
   Xyl-(1→4)-Fuc-(1→2)-Gal-(1→2)-Rha-(1→2)-GlcA-,
   Xyl-(1→4)-Rha-(1→2)-Glc-(1→2)-Fuc-(1→2)-GlcA-,
   Xyl-(1→4)-Fuc-(1→2)-Glc-(1→2)-Fuc-(1→2)-GlcA-,
   Xyl-(1→4)-Rha-(1→2)-Gal-(1→2)-Fuc-(1→2)-GlcA-,
   Xyl-(1→4)-Fuc-(1→2)-Gal-(1→2)-Fuc-(1→2)-GlcA-, and
   derivatives thereof,
   or
- the saponin comprises a saccharide chain bound to the aglycone core structure, which is selected from group B:
   Glc-,
   Gal-,
   Rha-(1→2)-[Xyl-(1→4)]-Rha-,
   Rha-(1→2)-[Ara-(1→3)-Xyl-(1→4)]-Rha-,
   Ara-,
   Xyl-,
   Xyl-(1→4)-Rha-(1→2)-[R1-(→4)]-Fuc- wherein R1 is 4E-Methoxycinnamic acid,
   Xyl-(1→4)-Rha-(1→2)-[R2-(→4)]-Fuc- wherein R2 is 4Z-Methoxycinnamic acid,
   Xyl-(1→4)-[Gal-(1→3)]-Rha-(1→2)-4-OAc-Fuc-,
   Xyl-(1→4)-[Glc-(1→3)]-Rha-(1→2)-3,4-di-OAc-Fuc-,
   Xyl-(1→4)-[Glc-(1→3)]-Rha-(1→2)-[R3-(→4)]-3-OAc-Fuc- wherein R3 is 4E-Methoxycinnamic acid,
   Glc-(1→3)-Xyl-(1→4)-[Glc-(1→3)]-Rha-(1→2)-4-OAc-Fuc-,
   Glc-(1→3)-Xyl-(1→4)-Rha-(1→2)-4-OAc-Fuc-,
   (Ara- or Xyl-)(1→3)-(Ara- or Xyl-)(1→4)-(Rha- or Fuc-)(1 --->2)-[4-OAc-(Rha- or Fuc-)(1→4)]-(Rha- or Fuc-),
   Xyl-(1→3)-Xyl-(1→4)-Rha-(1→2)-[Qui-(1→4)]-Fuc-,
   Api-(1→3)-Xyl-(1→4)-[Glc-(1→3)]-Rha-(1→2)-Fuc-,
   Xyl-(1→4)-[Gal-(1→3)]-Rha-(1→2)-Fuc-,
   Xyl-(1→4)-[Glc-(1→3)]-Rha-(1→2)-Fuc-,
   Ara/Xyl-(1→4)-Rha/Fuc-(1→4)-[Glc/Gal-(1→2)]-Fuc-,
   Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[Rha-(1→3)]-4-OAc-Fuc-,
   Api-(1→3)-Xyl-(1→4)-[Glc-(1→3)]-Rha-(1→2)-[Rha-(1→3)]-4-OAc-Fuc-,
   6-OAc-Glc-(1→3)-Xyl-(1→4)-Rha-(1→2)-[3-OAc-Rha-(1→3)]-Fuc-,
   Glc-(1→3)-Xyl-(1→4)-Rha-(1→2)-[3-OAc--Rha-(1→3)]-Fuc-,
   Xyl-(1→3)-Xyl-(1→4)-Rha-(1→2)-[Qui-(1→4)]-Fuc-,
   Glc-(1→3)-[Xyl-(1→4)]-Rha-(1→2)-[Qui-(1→4)]-Fuc-,
   Glc-(1→3)-Xyl-(1→4)-Rha-(1→2)-[Xyl-(1→3)-4-OAc-Qui-(1→4)]-Fuc-,
   Xyl-(1→3)-Xyl-(1→4)-Rha-(1→2)-[3,4-di-OAc-Qui-(1→4)]-Fuc-,
   Glc-(1→3)-[Xyl-(1→4)]-Rha-(1→2)-Fuc-,
   6-OAc-Glc-(1→3)-[Xyl-(1→4)]-Rha-(1→2)-Fuc-,
   Glc-(1→3)-[Xyl-(1→3)-Xyl-(1→4)]-Rha-(1→2)-Fuc-,
   Xyl-(1→3)-Xyl-(1→4)-Rha-(1→2)-[Xyl-(1→3)-4-OAc-Qui-(1→4)]-Fuc-,
   Api/Xyl-(1→3)-Xyl-(1→4)-[Glc-(1→3)]-Rha-(1→2)-[Rha-(1→3)]-4OAc-Fuc-,
   Api-(1→3)-Xyl-(1→4)-[Glc-(1→3)]-Rha-(1→2)-[Rha-(1→3)]-4OAc-Fuc-,
   Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
   Glc-(1→3)-[Glc-(1→6)]-Gal-, and
   derivatives thereof,
   or
- the saponin is a bidesmosidic triterpene glycoside comprising a first saccharide chain selected from the group A bound to the aglycone core structure and comprising a second saccharide chain selected from the group B bound to the aglycone core structure.

Such saponins display the typical endosomal escape enhancing effect when a target cell is contacted with the saponin and an effector molecule, such as an effector molecule that is part of the conjugate according to the invention, comprising the effector molecule and the ligand for ASGPR1, e.g. a (GalNAc)₃Tris. Under influence of such a selected saponin comprising the one or two saccharides selected from Group C, the effector molecule in the conjugate reaches an intracellular threshold level such that the biological activity of the effector molecule inside a target cell is either established, or is increased, or the activity of the effector molecule can be established at a lower dose than the dose required for the same level of activity in the absence of saponin. In Table 1, saponins with such endosomal escape enhancing activity are summarized. These saponins comprise the listed aglycone core structures of Group C and comprise the monosaccharides or polysaccharides of Group A and/or Group B, and have been shown to potentiate effector molecules when target cells are exposed to both the effector molecule and the saponin, or have high structural similarity with saponins for which such endosomal escape enhancing activity has been determined.

Therefore, an embodiment is the pharmaceutical combination of the invention, wherein the saponin is selected from the group consisting of: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, beta-Aescin, Aescin la, Teaseed saponin I, Teaseedsaponin J, Assamsaponin F, Digitonin, Primula acid 1 and AS64R, stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741,a GE1741 derivative and combinations thereof, more preferably the saponin is selected from the group consisting of a QS-21 derivative, a S01861 derivative and combinations thereof, most preferably the saponin is a SO1861 derivative. The present inventors have found that these derivatives have improved potentiating effects when employed in conjunction with a conjugate comprising an effector molecule as described herein. That is to say, the derivatives lead to comparable endosomal escape enhancing effects as the non-derivatised saponins, while displaying lower cytotoxicity and lower haemolytic activity. More structural details of saponin derivatives suitable in the context of the present invention are provided below.

An embodiment is the pharmaceutical combination of the invention, wherein the saponin is a saponin derivative wherein either:
i. the saponin derivative comprises an aglycone core structure comprising an aldehyde group which has been derivatised; or
ii. the saponin derivative comprises a saccharide chain, preferably a saccharide chain selected from group A as defined here above, the saccharide chain comprising a carboxyl group which has been derivatised; or
iii. the saponin derivative comprises a saccharide chain, preferably a saccharide chain selected from group B as defined here above, the saccharide chain comprising an acetoxy (Me(CO)O-) group which has been derivatised; or
iv. any combination of derivatisations i., ii. and iii. is present, optionally any combination of two derivatisations of derivatisations i., ii. and iii.

The inventors established that in particular saponins which have been derivatised with regard to a single chemical group present in the naturally occurring saponin or with regard to two of such chemical groups, maintain sufficient effector molecule potentiating activity, while both cytotoxicity and haemolytic activity of such mono-derivatised and bi-derivatised saponins is strongly reduced compared to cytotoxicity and haemolytic activity of the naturally occurring non-modified counterparts of such saponin derivatives. Also suitable according to the invention are the naturally occurring saponins for which the effector molecule potentiating effect is established when the saponin and the conjugate comprising the effector molecule are both contacted with a target cell exposing ASGPR1 at its surface.

An embodiment is the pharmaceutical combination of the invention, wherein the saponin is selected from the group consisting of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, OS1861, QS1862, Ouillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

An embodiment is the pharmaceutical combination of the invention, wherein the saponin is a saponin derivative of the quillaic acid or gypsogenin saponin of Group C as listed here above and represented by Molecule 1: 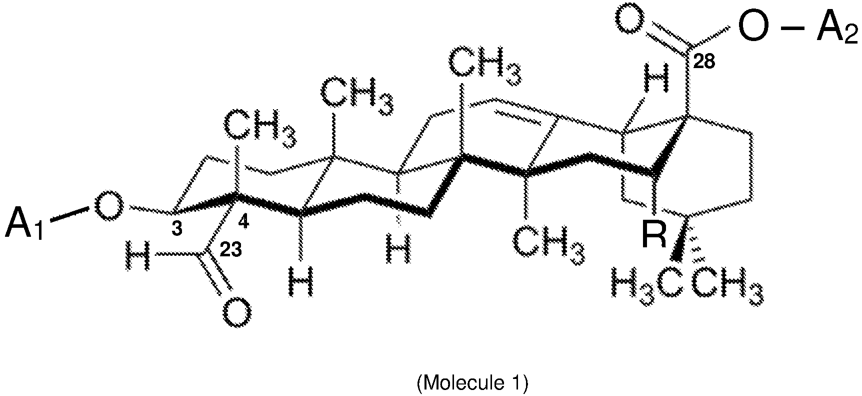 wherein
A₁ represents hydrogen, a monosaccharide or a linear or branched oligosaccharide, preferably
A₁ represents a saccharide chain selected from group A as defined here above, more preferably
A₁ represents a saccharide chain selected from group A as defined here above and A₁ comprises or consists of a glucuronic acid moiety;
A₂ represents hydrogen, a monosaccharide or a linear or branched oligosaccharide, preferably
A₂ represents a saccharide chain selected from group B as defined here above, more preferably
A₂ represents a saccharide chain selected from group B as defined here above and A₂ comprises at least one acetoxy (Me(CO)O-) group, such as one, two, three or four acetoxy groups, wherein at least one of A₁ and A₂ is not hydrogen, preferably both A₁ and A₂ are an oligosaccharide chain;
and R is hydrogen in gypsogenin or hydroxyl in quillaic acid;
wherein the saponin derivative corresponds to the saponin represented by Molecule 1 wherein at least one of the following derivatisations is present:
i. the aldehyde group at position C₂₃ of the quillaic acid or gypsogenin has been derivatised;
ii. the carboxyl group of a glucuronic acid moiety of A₁, when A₁ represents a saccharide chain selected from group A as defined here above and A₁ comprises or consists of a glucuronic acid moiety, has been derivatised; and
iii. one or more, preferably all, of acetoxy group(s) of one saccharide moiety or of two or more saccharide moieties of A₂, when A₂ represents a saccharide chain selected from group B as defined here above and A₂ comprises at least one acetoxy group, has/have been derivatised.

An embodiment is the pharmaceutical combination of the invention, wherein A₁ represents a saccharide chain selected from group A as defined here above and comprises or consists of a glucuronic acid moiety and wherein the carboxyl group of a glucuronic acid moiety of A₁ has been derivatised and/or wherein A₂ represents a saccharide chain selected from group B as defined here above and A₂ comprises at least one acetoxy group and wherein at least one acetoxy group of A₂ has been derivatised.

An embodiment is the pharmaceutical combination of the invention, wherein the saponin represented by Molecule 1 is a bidesmosidic triterpene saponin, *i.e.* a saponin of the bidesmosidic triterpene glycoside type.

An embodiment is the pharmaceutical combination of the invention, wherein the saponin derivative corresponds to the saponin represented by Molecule 1 wherein at least one of the following derivatisations is present:
i. the aldehyde group at position C₂₃ of the quillaic acid or gypsogenin has been derivatised by;
   - reduction to an alcohol; or
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
   - transformation into a hydrazone bond through reaction with N-[β-maleimidopropionic acid] hydrazide (BMPH) wherein the maleimide group of the BMPH is optionally derivatised by formation of a thioether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the carboxyl group of a glucuronic acid moiety of A₁, when A₁ represents a saccharide chain selected from group A as defined here above and A₁ comprises or consists of a glucuronic acid moiety, has been derivatised by transformation into an amide bond through reaction with 2-amino-2-methyl-1,3-propanediol (AMPD) or *N*-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of A₂, when A₂ represents a saccharide chain selected from group B as defined here above and A₂ comprises at least one acetoxy group, has/have been derivatised by transformation into a hydroxyl group (HO-) by deacetylation.

An embodiment is the pharmaceutical combination of the invention, wherein A₁ is Gal-(1→2)-[Xyl-(1→3)]-GlcA and/or A₂ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, more preferably SO1861, GE1741, SA1641 and/or QS-21, or a derivative thereof, most preferably SO1861 or a derivative thereof.

An embodiment is the pharmaceutical combination of the invention, wherein the saponin is a saponin derivative wherein either:
i. the saponin derivative comprises an aglycone core structure comprising an aldehyde group which has been derivatised by:
   - reduction to an alcohol; or
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
   - transformation into a hydrazon bond through reaction with N-[β-maleimidopropionic acid] hydrazide (BMPH) wherein the maleimide group of the BMPH is optionally derivatised by formation of a thioether bond with mercaptoethanol; or
   - transformation into a hydrazon bond through reaction with N-[κ-maleimidoundecanoic acid] hydrazide (KMUH) wherein the maleimide group of the KMUH is optionally derivatised by formation of a thioether bond with mercaptoethanol; or
ii. the saponin derivative comprises a saccharide chain, preferably a saccharide chain selected from group A as defined here above, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM; or
iii. the saponin derivative comprises a saccharide chain, preferably a saccharide chain selected from group B as defined here above, the saccharide chain comprising an acetoxy (Me(CO)O-) group which has been derivatised by transformation into a hydroxyl group (HO-) by deacetylation; or
iv. the saponin derivative comprises any combination of derivatisations i., ii. and iii., optionally any combination of two derivatisations i., ii. and iii;
preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thioether bond with mercaptoethanol.

An embodiment is the pharmaceutical combination of the invention, wherein the saponin is a saponin derivative wherein either:
i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH; or
ii. the saponin comprises a saccharide chain, preferably a saccharide chain selected from group A as defined here above, the saccharide chain comprising a carboxyl group, preferably a carboxyl group of a glucuronic acid moiety which has been derivatised by transformation into an amide bond through reaction with N-(2-aminoethyl)maleimide (AEM) , therewith providing a saponin-Glu-AEM such as a QS-21-Glu-AEM or a SO1861-Glu-AEM; or
iii. the saponin derivative comprises a combination of derivatisations i. and ii.

An embodiment is the pharmaceutical combination of the invention, wherein the saponin derivative comprises an aglycone core structure wherein the aglycone core structure comprises an aldehyde group and wherein the saponin derivative comprises a saccharide chain, preferably a saccharide chain selected from group A as defined here above, the saccharide chain comprising a carboxyl group, preferably a carboxyl group of a glucuronic acid moiety, which glucuronic acid moiety has been derivatised by transformation into an amide bond through reaction with N-(2-aminoethyl)maleimide (AEM).

An embodiment is the pharmaceutical combination of the invention, wherein the saponin is a saponin derivative represented by Molecule 2: 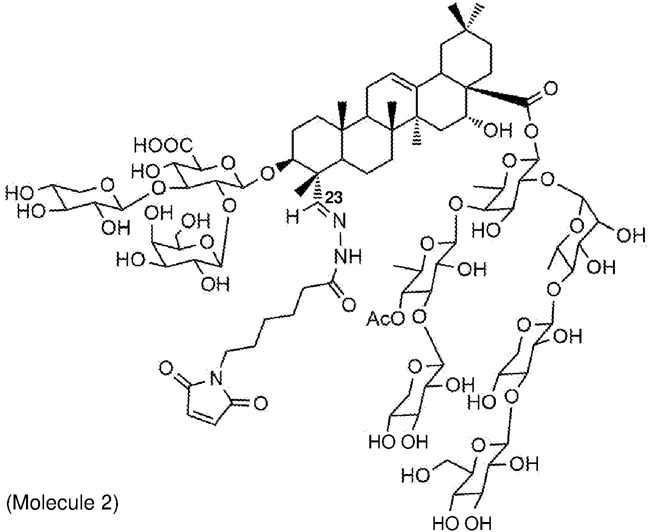 or wherein the saponin derivative is the saponin derivative represented by Molecule 3

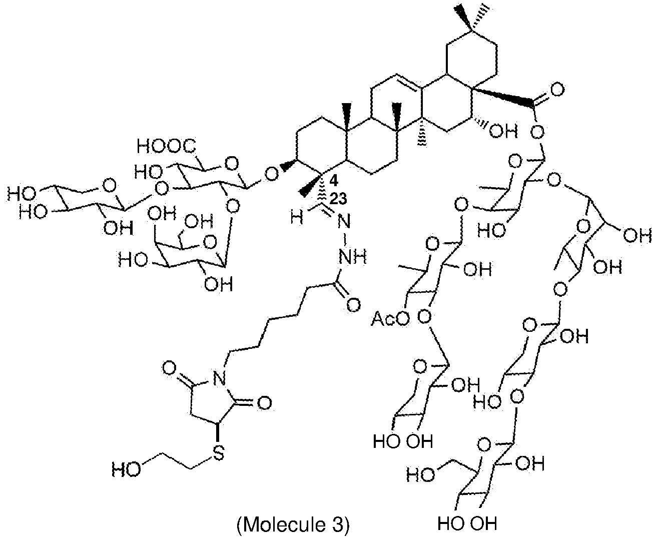

| TABLE A1. Saponins displaying (late) endosomal/lysosomal escape enhancing activity, and saponins comprising a structure reminiscent to such saponins displaying (late) endosomal/lysosomal escape enhancing activity | | | |
|---|---|---|---|
| **Saponin Name** | **Aglycone core** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | - |
| NP-012672 | 16alpha,23-Dihydroxyoleanolic acid | Ara/Xyl-(1→4)-Rha/Fuc-(1→2)-Glc/Gal-(1→2)-Rha/Fuc-(1→2)-GlcA- | Ara/Xyl- |
| NP-017777 | Gypsogenin | Gal-(1→2)-[Xyl-(1→3)]-GlcA- | Xyl-(1→4)-Rha-(1→2)-[R-(→4)]-Fuc- (R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110^{c}, NP-017772^{d} | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-01 8109 | Gypsogenin | Gal-(1→2)-[Xyl-(1→3)]-GlcA- | Xyl-(1→4)-[Glc-(1→3)]-Rha-(1→2)-[R-(→4)]-3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641^{a}, AE X55^{b} | Gypsogenin | Gal-(1→2)-[Xyl-(1→3)]-GlcA- | Xyl-(1→3)-Xyl-(1→4)-Rha-(1→2)-[Qui-(1→4)]-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc--Rha-(1→3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| SO1861 | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | Glc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1832 | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| beta-Aescin (described: Aescin la) | Protoaescigenin-21 (2-methylbut-2-enoate)-22-acetat | Glc-(1→2)-[Glc-(1→4)]-GlcA- | - |
| Teaseed saponin I | 23-Oxo-barringtogenol C - 21,22-bis(2-methylbut-2-enoate) | Glc-(1→2)-Ara-(1→3)-[Gal-(1→2)]-GlcA- | - |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | - |
| Assamsaponin F | 23-Oxo-barringtogenol C - 21 (2-methylbut-2-enoate)-16,22-diacetat | Glc-(1→2)-Ara-(1→3)-[Gal-(1→2)]-GlcA- | - |
| Digitonin | Digitogenin | Glc-(1→3)-Gal-(1→2)-[Xyl-(1→3)]-Glc-(1→4)-Gal- | - |
| Primula acid 1 | 3,16,28-Trihydroxyoleanan-12-en | Rha-(1→2)-Gal-(1→3)-[Glc-(1→2)]-GlcA- | - |
| AS64R | Gypsogenic acid | - | Glc-(1→3)-[Glc-(1→6)]-Gal- |
| | | **Carbohydrate substituent at the C-23-OH group** | |
| AS6.2 | Gypsogenic acid | Gal- | Glc-(1→3)-[Glc-(1→6)]-Gal- |
| a, b: Different names refer to different isolates of the same structure | | | |
| c, d: Different names refer to different isolates of the same structure | | | |

A second aspect of the invention relates to the pharmaceutical combination of the invention for use as a medicament. To the knowledge of the inventors they are the first to provide the pharmaceutical combination of the invention for improving the efficacy of e.g. siRNA or BNA based therapies. Use of the pharmaceutical combination of the invention potentiates the intracellular effect of the effector molecule comprised by the conjugate comprising the ligand for ASGPR1. The medicament is particularly suitable for treatment of diseases or abberancies involving aberrant liver cells which express ASGPR1. Aberrant liver cells are cells of patients who suffer for example from any disease or health problem in which an expression product is involved of any one or more of genes: HSP27, apoB, TTR, PCSK9, ALAS1, AT3, GO, CC5, X gene of HBV, S gene of HBV, AAT and LDH. Such diseases are for example any of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, a disease or disorder relating to HSP27 expression, or an auto-immune disease.

A third aspect of the invention relates to the pharmaceutical combination of the invention, for use in the treatment or prophylaxis of a disease or health problem in which an expression product is involved of any one or more of genes: HSP27, apoB, TTR, PCSK9, ALAS1, AT3, GO, CC5, X gene of HBV, S gene of HBV, AAT and LDH.

An embodiment is the pharmaceutical combination of the invention, or the pharmaceutical combination for use of the invention, for use in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, a disease or disorder relating to HSP27 expression, or an auto-immune disease.

An embodiment is the pharmaceutical combination for use according to the invention, wherein the pharmaceutical combination comprises:
- a saponin derivative, wherein the saponin derivative corresponds to the saponin represented by Molecule 1 wherein at least one of the following derivatisations is present:
   i. the aldehyde group at position C₂₃ of the quillaic acid or gypsogenin has been derivatised by;
      - reduction to an alcohol; or
      - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
      - transformation into a hydrazone bond through reaction with N-[β-maleimidopropionic acid] hydrazide (BMPH) wherein the maleimide group of the BMPH is optionally derivatised by formation of a thioether bond with mercaptoethanol; or
      - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
   ii. the carboxyl group of a glucuronic acid moiety of A₁, when A₁ represents a saccharide chain selected from group A and A₁ comprises or consists of a glucuronic acid moiety, has been derivatised by transformation into an amide bond through reaction with 2-amino-2-methyl-1,3-propanediol (AMPD) or *N*-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
   iii. one or more, optionally all, of acetoxy group(s) of one saccharide moiety or of two or more saccharide moieties of A₂, when A₂ represents a saccharide chain selected from group B and A₂ comprises at least one acetoxy group, has/have been derivatised by transformation into a hydroxyl group (HO-) by deacetylation; or
- a saponin according to Molecule 1, wherein A₁ is Gal-(1→2)-[Xyl-(1→3)]-GlcA and/or A₂ is Glc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAcbeta-D-quinovopyranosyl-(1→4)]-beta-D-fucopyranoside, more preferably SO1861, GE1741, SA1641 and/or QS-21, or a derivative thereof, most preferably SO1861 or a derivative thereof; or
- a saponin derivative wherein
   i. the saponin derivative comprises an aglycone core structure comprising an aldehyde group which has been derivatised by:
      - reduction to an alcohol; or
      - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
      - transformation into a hydrazon bond through reaction with N-[β-maleimidopropionic acid] hydrazide (BMPH) wherein the maleimide group of the BMPH is optionally derivatised by formation of a thioether bond with mercaptoethanol; or
      - transformation into a hydrazon bond through reaction with N-[κ-maleimidoundecanoic acid] hydrazide (KMUH) wherein the maleimide group of the KMUH is optionally derivatised by formation of a thioether bond with mercaptoethanol;
   ii. the saponin derivative comprises a saccharide chain, preferably a saccharide chain selected from group A, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or *N*-(2-aminoethyl)maleimide (AEM), therewith providing a saponin-Glu-AMPD such as a QS-21-Glu- AMPD or a SO1861-Glu-AMPD or a saponin-Glu-AEM such as a QS-21-Glu-AEM or a SO1861-Glu-AEM;
   iii. the saponin derivative comprises a saccharide chain, preferably a saccharide chain selected from group B, the saccharide chain comprising an acetoxy (Me(CO)O-) group which has been derivatised by transformation into a hydroxyl group (HO-) by deacetylation; or
   iv. the saponin derivative comprises any combination of derivatisations i., ii. and iii., optionally any combination of two derivatisations i., ii. and iii.;
   preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thioether bond with mercaptoethanol; or
- a saponin derivative wherein
   i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH;
   ii. the saponin comprises a saccharide chain, preferably a saccharide chain selected from group A, the saccharide chain comprising a carboxyl group, preferably a carboxyl group of a glucuronic acid moiety which has been derivatised by transformation into an amide bond through reaction with *N*-(2-aminoethyl)maleimide (AEM) , therewith providing a saponin-Glu-AEM such as a QS-21-Glu-AEM or a SO1861-Glu-AEM; or
   iii. the saponin derivative comprises a combination of derivatisations i. and ii.; or
- a saponin derivative that comprises an aglycone core structure wherein the aglycone core structure comprises an aldehyde group and wherein the saponin derivative comprises a saccharide chain, preferably a saccharide chain selected from group A, the saccharide chain comprising a carboxyl group, preferably a carboxyl group of a glucuronic acid moiety, which glucuronic acid moiety has been derivatised by transformation into an amide bond through reaction with *N*-(2-aminoethyl)maleimide (AEM); or
- a saponin derivative represented by Molecule 2: 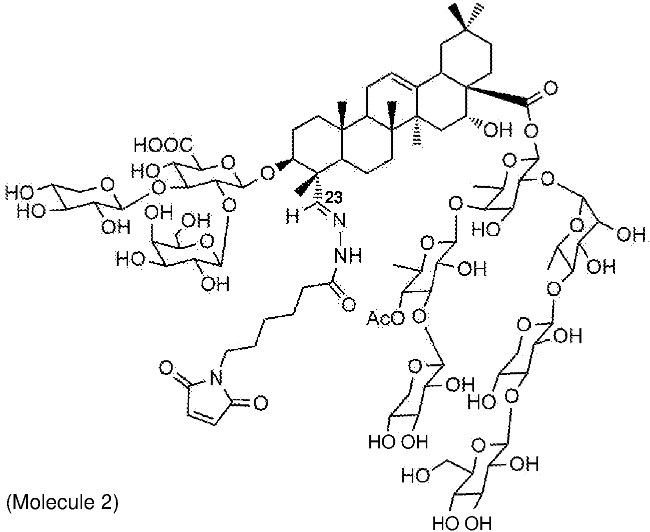 or a saponin derivative represented by Molecule 3:

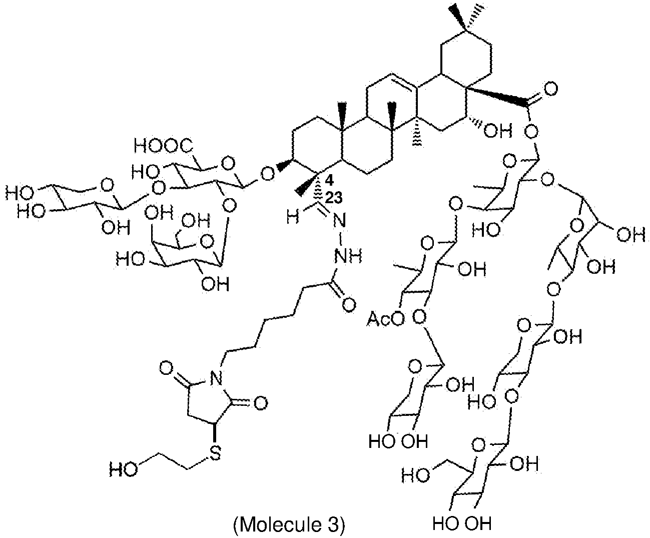

A fourth aspect of the invention relates to an *in vitro* or *ex vivo* method for transferring the effector molecule comprised by the conjugate comprising the ligand for ASGPR according to the invention, from outside a cell to inside said cell, preferably into the cytosol of said cell, comprising the steps of:
a) providing a cell which expresses ASGPR, preferably ASGPR1, on its surface, preferably selected from a liver cell, a virally infected cell and a cancer cell;
b) providing the conjugate of the invention, the conjugate comprising the effector molecule to be transferred;
c) providing the saponin according to the invention;
d) contacting the cell of step a) *in vitro* or *ex vivo* with the conjugate of step b) and the saponin of step c), therewith effecting the transfer of said conjugate comprising the effector molecule from outside the cell to inside said cell, and by effecting the transfer of said conjugate effecting the transfer of the effector molecule from outside the cell to inside said cell, preferably into the cytosol of said cell.

A fifth aspect of the invention relates to an *in vitro* or *ex vivo* method for transferring the conjugate of the invention from outside a cell to inside said cell, comprising the steps of:
a) providing a cell which expresses ASGPR, preferably ASGPR1, on its surface, preferably selected from a liver cell, a virally infected cell and a cancer cell;
b) providing the conjugate of the invention;
c) providing the saponin of the invention;
d) contacting the cell of step a) *in vitro* or *ex vivo* with the conjugate of step b) and the saponin of step c), therewith effecting the transfer of the conjugate from outside the cell to inside said cell.

An embodiment is any of the two methods of the invention, wherein the ligand for ASGPR comprises at least one N-acetylgalactosamine (GalNAc) moiety, preferably three or four GalNAc moieties, more preferably the ligand for ASGPR comprises or consists of (GalNAc)₃Tris and/or wherein the effector molecule is an oligonucleotide selected from any one of an anti-miRNA, a BNA-AON or an siRNA, such as BNA-based siRNA, preferably selected from chemically modified siRNA, metabolically stable siRNA and chemically modified, metabolically stable siRNA.

An embodiment is any of the two methods of the invention, wherein the effector molecule comprises or consists of at least one of a small molecule such as a drug molecule, a toxin such as a protein toxin, an oligonucleotide such as a BNA, a xeno nucleic acid or an siRNA, an enzyme, a peptide, a protein, or any combination thereof, preferably, the effector molecule is a toxin, an enzyme or an oligonucleotide, preferably the toxin is saporin or dianthin.

An embodiment is any of the two methods of the invention, wherein the saponin is derivatised SO1861 and/or derivatised QS-21, preferably SO1861-Glu-AEM or SO1861-Ald-EMCH or QS-21-Glu-AEM or QS-21-Ald-EMCH according to previous embodiments of the invention.

An embodiment is any of the two methods of the invention, wherein the saponin is a saponin derivative corresponding to the saponin represented by Molecule 1 wherein at least one of the following derivatisations is present:
i. the aldehyde group at position C₂₃ of the quillaic acid or gypsogenin has been derivatised by;
   - reduction to an alcohol; or
   - transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
   - transformation into a hydrazone bond through reaction with N-[β-maleimidopropionic acid] hydrazide (BMPH) wherein the maleimide group of the BMPH is optionally derivatised by formation of a thioether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the carboxyl group of a glucuronic acid moiety of A₁, when A₁ represents a saccharide chain selected from group A and A₁ comprises or consists of a glucuronic acid moiety, has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of A₂, when A₂ represents a saccharide chain selected from group B and A₂ comprises at least one acetoxy group, has/have been derivatised by transformation into a hydroxyl group (HO-) by deacetylation.

An embodiment is any of the two methods of the invention, wherein the effector molecule is an oligonucleotide which, for example when present inside a mammalian cell, can silence any one of genes: apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or, for example when present inside a mammalian cell, can target an aberrant miRNA and/or, for example when present inside a mammalian cell, wherein the oligonucleotide can target an mRNA involved in expression of any one of proteins: apoB, TTR, PCSK9, ALAS1, AT3, GO, CC5, expression product of X gene of HBV, expression product of S gene of HBV, AAT and LDH, or, for example when present inside a mammalian cell, can antagonize or restore an miRNA function such as inhibiting an oncogenic miRNA (onco-miR) or suppression of expression of an onco-miR.

A sixth aspect of the invention relates to a kit of parts, comprising the pharmaceutical combination of the invention or the second pharmaceutical composition of the invention, and instructions for use of said pharmaceutical combination or second pharmaceutical composition according to the invention or for use in a method according to the invention.

The present invention has been described above with reference to a number of exemplary embodiments. The invention is further illustrated by the following examples.

### EXAMPLES AND EXEMPLARY EMBODIMENTS

### Example 1. CD71-saporin + 4000 nM trivalent GalNAc-L-SO1861

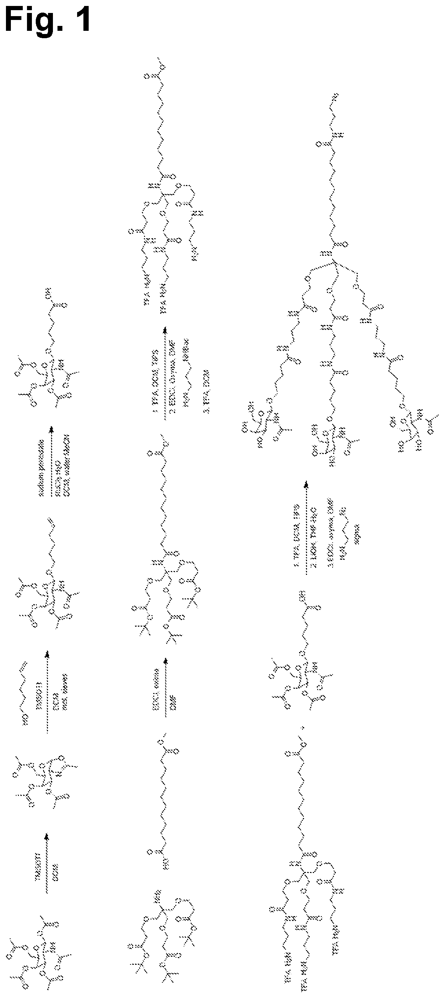

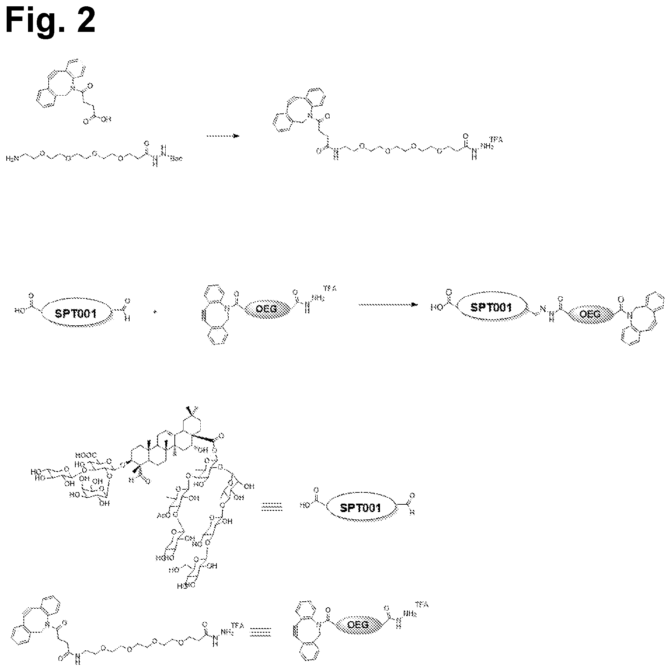

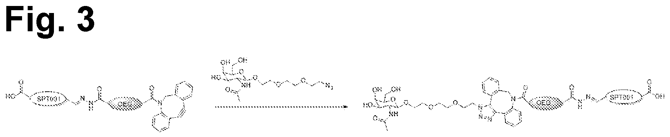

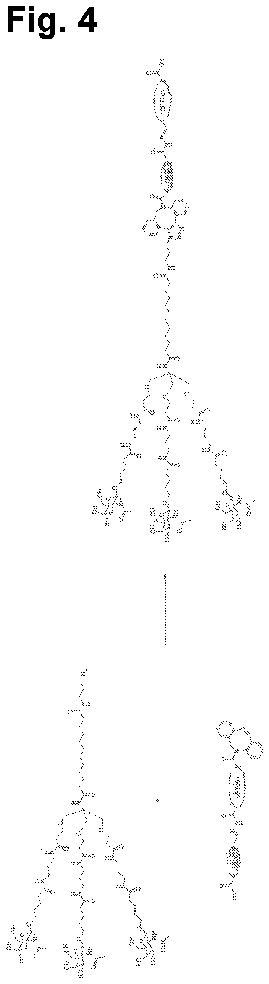

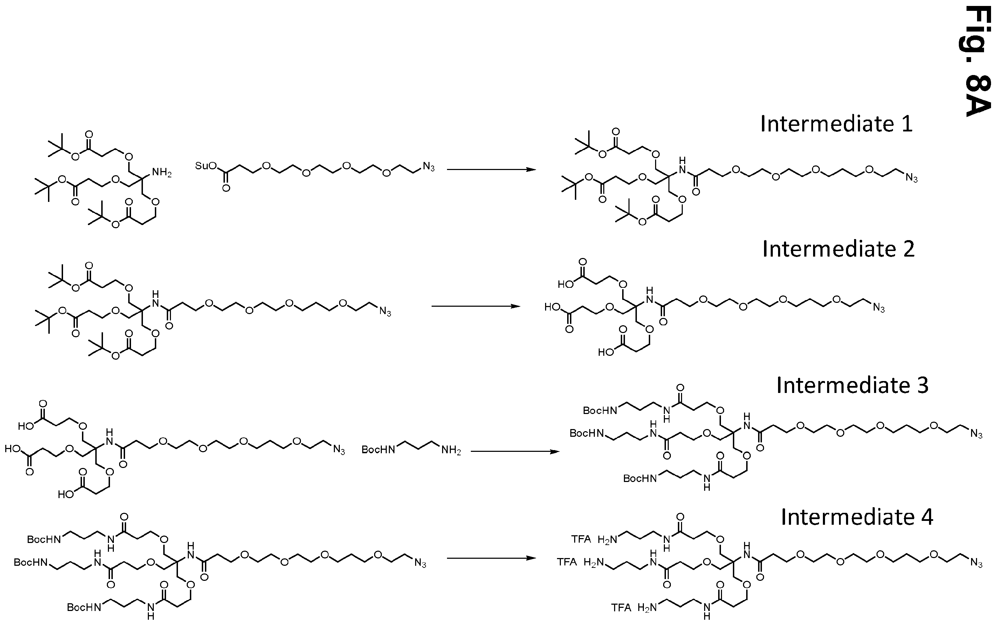

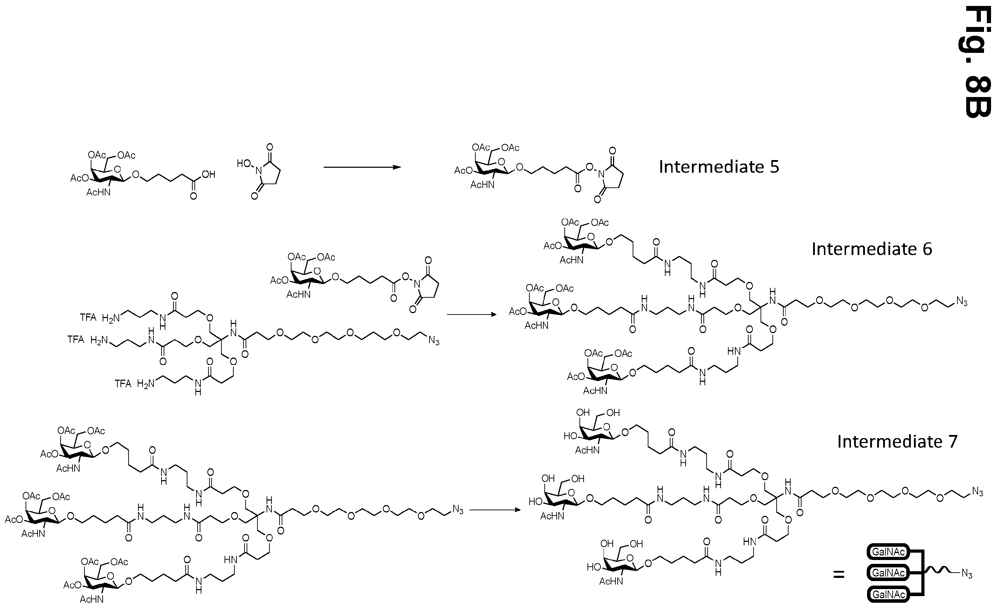

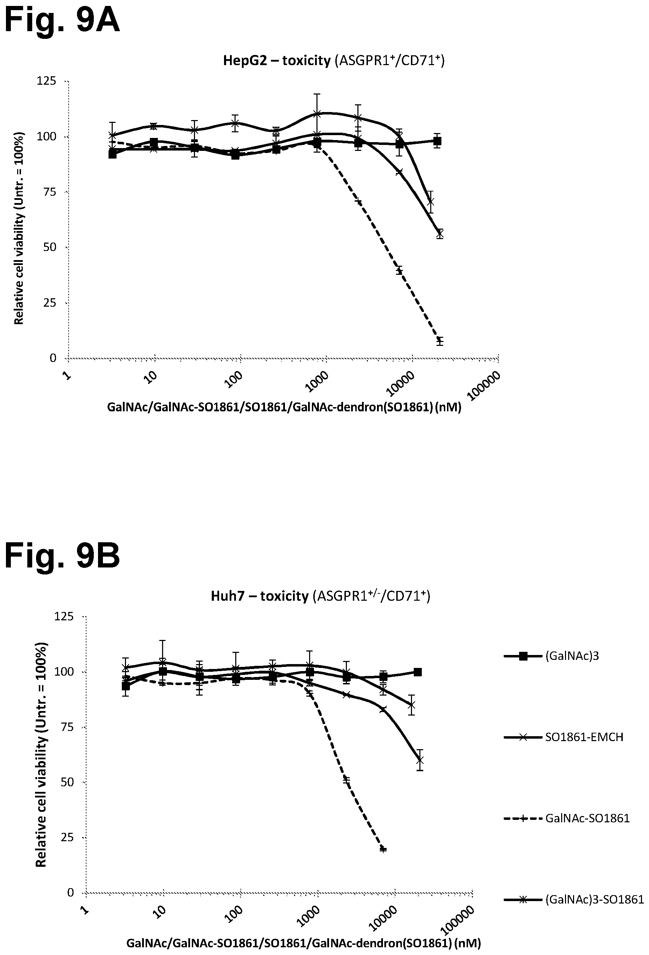

Trivalent-GalNAc is a targeting ligand that recognizes and binds the ASGPR1 receptor on hepatocytes. Trivalent GalNAC was produced (Figure 1 and Figure 8) and SO1861-EMCH was conjugated (labile, in a similar manner as described in Figure 2 and Figure 4 for underivatised SO1861, wherein SPT001 is synonymous to SO1861) to trivalent-GalNAc, with a DAR = 1 for the bound SO1861, (trivalent-GalNAc-SO1861), also referred to as (GalNAc)3-SO1861). SO1861-EMCH refers to SO1861 wherein the aldehyde group is derivatised by transformation into a hydrazone bond through reaction with N-ε-maleimidocaproic acid hydrazide (EMCH), therewith providing SO1861-Ald-EMCH. The 'trivalent-GalNAc' as depicted in Figures 1 and 8 is a typical (GalNAc)₃Tris conjugate suitable for coupling to an effector molecule or to saponin. As a control SO1861-EMCH was also conjugated (labile) to monovalent-GalNAc (Figure 3), with a DAR = 1 for the bound SO1861, (GalNAc-SO1861). HepG2 (ASGPR1⁺; CD71⁺, Table A3) and Huh7 (ASGPR1 ⁺¹⁻; CD71⁺, Table A3) cells were treated with a concentration range of trivalent-GalNAc-L-SO1861 and GalNAc-L-SO1861 in the presence and absence of 10 pM CD71-saporin (monoclonal antibody OKT-9 targeting CD71 conjugated to the protein toxin saporin). Cell treatment in absence of CD71-saporin show that the trivalent-GalNAc-L-SO1861 (or trivalent-GalNAc, also referred to as (GaINAc)3) as single compound is not toxic up to 15000 nM (Figure 9).

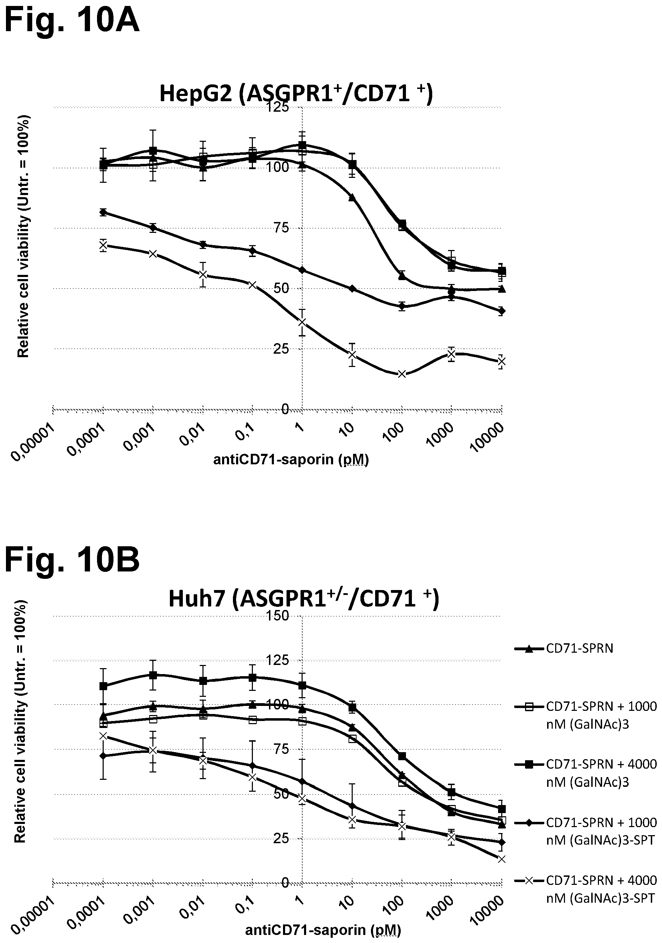

HepG2 and Huh7 cells were also treated with a range of concentrations of CD71-saporin, also referred to as CD71-SPRN, in combination with a fixed concentration of 1000 nM or 4000 nM trivalent-GalNAc-SO1861 (DAR = 1 for the bound SO1861), also referred to as (GalNAc)3-SPT. Targeted protein toxin mediated cell killing of ASGPR1/CD71 expressing cells (HepG2 and Huh7) was determined. This revealed strong cell killing at low concentrations of CD71-saporin (IC50 = 1 pM) in both cell lines, whereas equivalent concentrations CD71-saporin, CD71-saporin *+* 1000 nM trivalent-GalNAc or CD71-saporin *+* 4000 nM trivalent-GalNAc could only induce cell killing at high concentrations CD71-saporin (IC50 = 100 pM) in both cell lines (Figure 10).

All this shows that trivalent-GalNAc-SO1861 in combination with low concentrations of CD71-saporin effectively induce cell killing in ASGPR1/CD71 expressing cells. Thus trivalent-GalNAc-SO1861 effectively induces endosomal escape of a protein toxin in ASGPR1 expressing cells.

### Example 2 trivalent-GalNAc-L/S-BNA + SO1861-EMCH

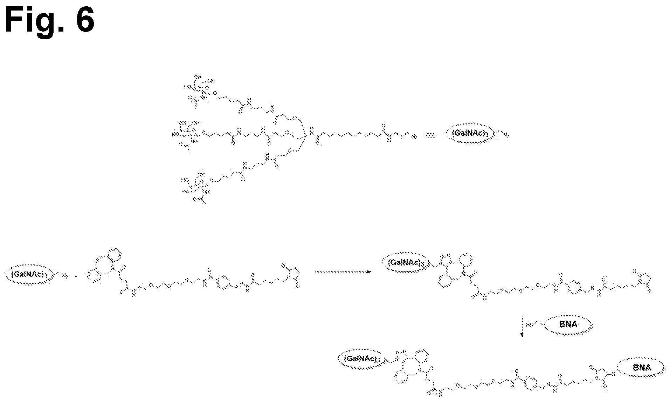

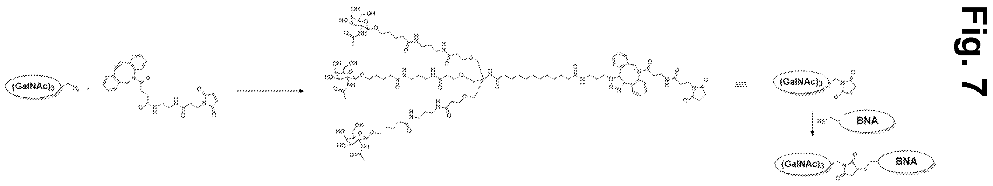

HSP27BNA was conjugated to trivalent-GalNAc (Figure 6-7) and HepG2 (ASGPR1⁺) cells or Huh7 (ASPGR1^{+/-}) were treated with a range of concentrations of trivalent-GalNAc-L-HSP27BNA (labile conjugation, Figure 6) or trivalent-GalNAc-S-HSP27BNA (stable conjugation, Figure 7) in combination with a fixed concentration of 4000 nM SO1861-EMCH, also referred to as SPT-EMCH. HSP27 gene silencing in HepG2 and Huh7 cells was determined. Only in combination with 4000 nM SO1861-EMCH effective gene silencing was observed at low concentrations of trivalent-GalNAc-L-HSP27BNA (HepG2: IC50 = 0,1 nM; Huh7: IC50 = 3 nM) or trivalent-GalNAc-S-HSP27BNA (HepG2: IC50 = 0,1 nM; Huh7: IC50 = 3 nM) in both HEPG2 and Huh7 cells, whereas equivalent concentrations trivalent-GalNAc-L-HSP27BNA (HepG2/Huh7: IC50> 2000 nM) or trivalent-GalNAc-S-HSP27BNA (HepG2/Huh7: IC50> 2000nM) did not show gene silencing activity in HepG2 and Huh7 cell lines (Figure 11).

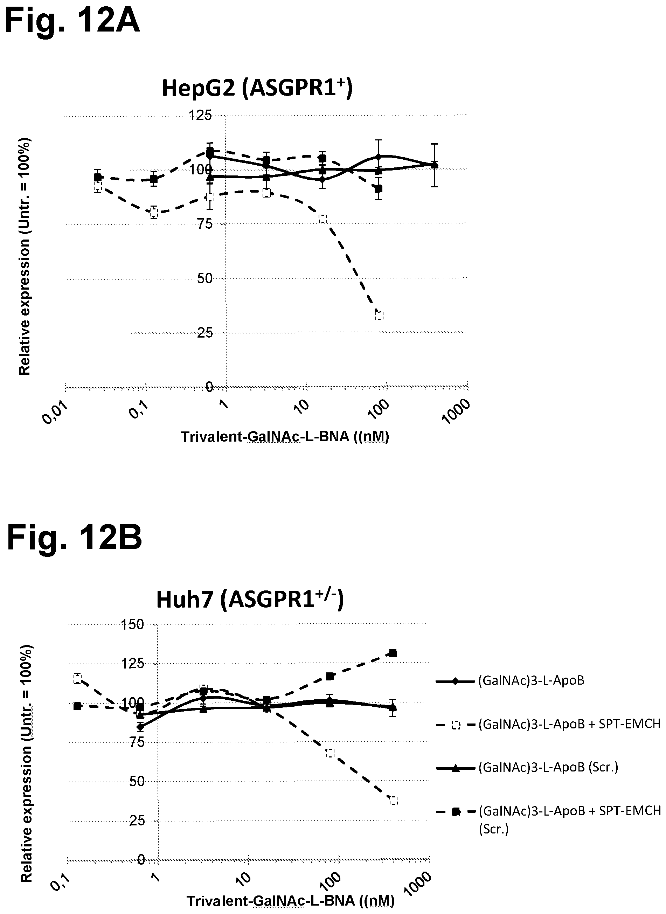

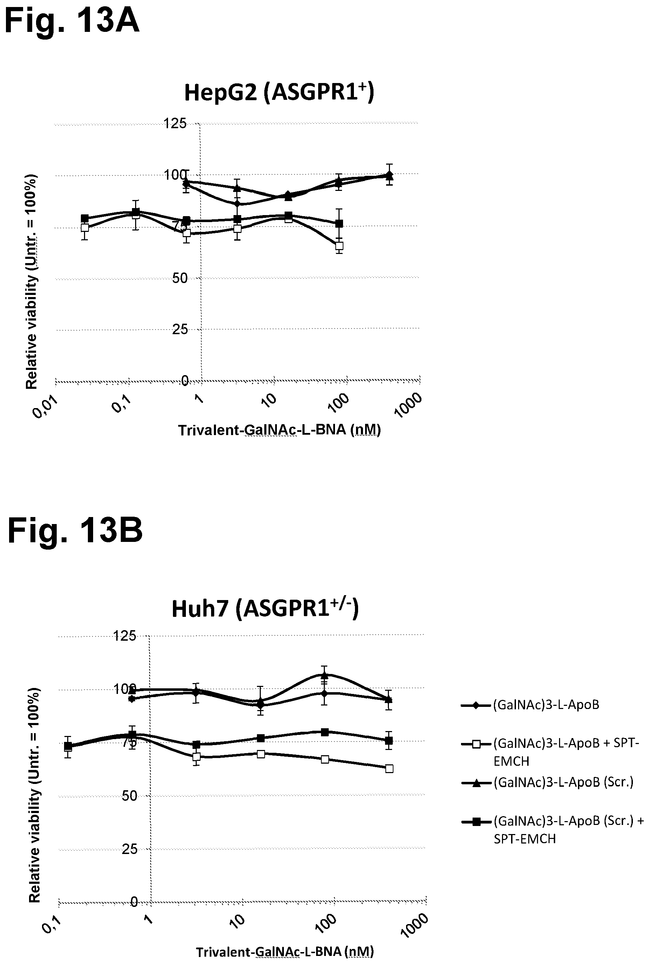

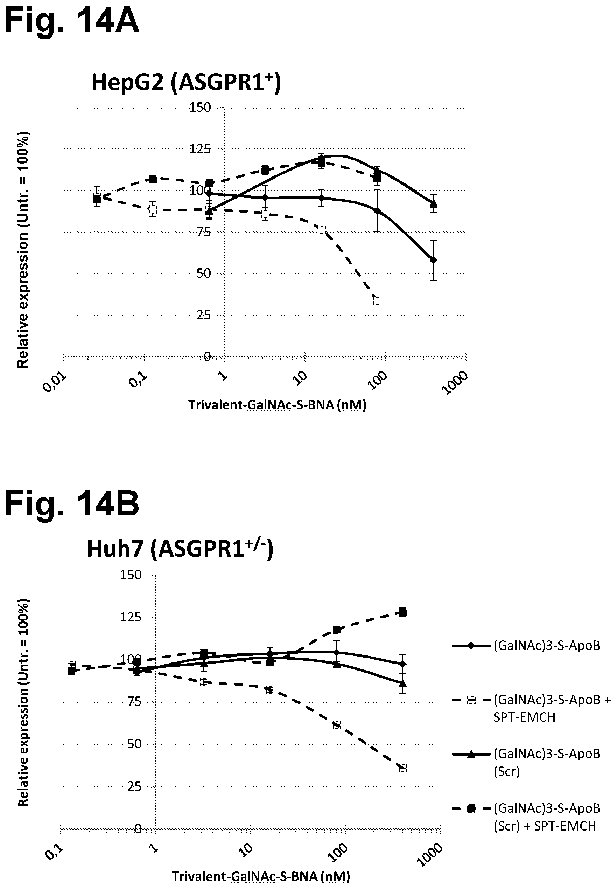

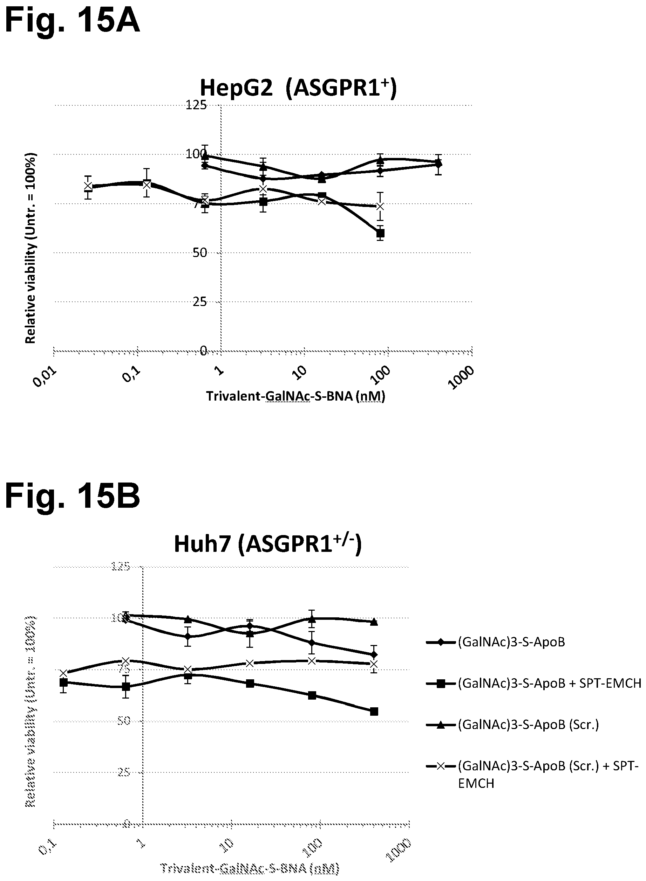

Next, ApoBBNA was conjugated in a similar manner (Figure 6-7) to trivalent-GalNAc and HepG2 (ASGPR1⁺) cells or Huh7 (ASPGR1^{+/-}) were treated with a range of concentrations of trivalent-GalNAc-L-ApoBBNA (labile conjugation, Figure 6, Figure 12, Figure 13) or trivalent GalNAc-S-ApoBBNA (stable conjugation, Figure 7, Figure 14, Figure 15) or a trivalent GalNAc-L-ApoB_{scrambled}BNA or trivalent GalNAc-S-ApoB_{scrambled}BNA (where the scrambled ApoBBNA is an antisense scrambled oligo sequence that does not bind the ApoB mRNA; off-target control BNA) in combination with a fixed concentration of 4000 nM SO1861-EMCH. ApoB gene silencing in HepG2 (ASGPR1⁺) cells or Huh7 (ASPGR1^{+/-}) was determined. Only in combination with 4000 nM SO1861-EMCH effective gene silencing was observed at low concentrations of trivalent-GalNAc-L-ApoBBNA (IC50 = 10 nM) or trivalent-GalNAc-S-ApoBBNA (IC50 = 10 nM) in HepG2 cells, whereas equivalent concentrations trivalent-GalNAc-L-ApoBBNA (IC50 > 2000 nM) or trivalent-GalNAc-S-ApoBBNA (IC50 > 2000 nM) did not show gene silencing activity in HepG2 cells (Figure 12, Figure 14). The treatments did not affect the viability of the cell lines (Figure 13, Figure 15). The 'L' refers to a 'labile' linker, which indicates that the linker is cleaved in the cell, *i.e.* at pH as apparent in the endosome and the lysosome. In contrast, the 'S' refers to a 'stable' linker, which indicates that the linker is not cleaved in the cell, *i.e.* at pH as apparent in the endosome and the lysosome. Thus, a conjugate comprising a labile bond between any two molecules forming the conjugate or comprised by the conjugate, will be cleaved at a position in the labile linker, therewith dissociating the two linked or bound molecules. An example is the cleavage of a hydrazone bond under acidic conditions as apparent in the endosome and lysosome of mammalian cells such as human cells.

All this shows that SO1861-EMCH can enhance endosomal escape and target gene silencing of trivalent-GalNAc-L-BNA or trivalent-GalNAc-S-BNA for two independent gene targets. The gene silencing is more efficient in HepG2 cells compared to Huh7 cells, suggesting that higher levels of ASGPR1 expression at the plasma membrane of the cell facilitates increased uptake of the GalNAc-BNA conjugates.

### Example 3 trivalent-GalNAc-L/S-BNA + trivalent-GalNAc-L-SO1861

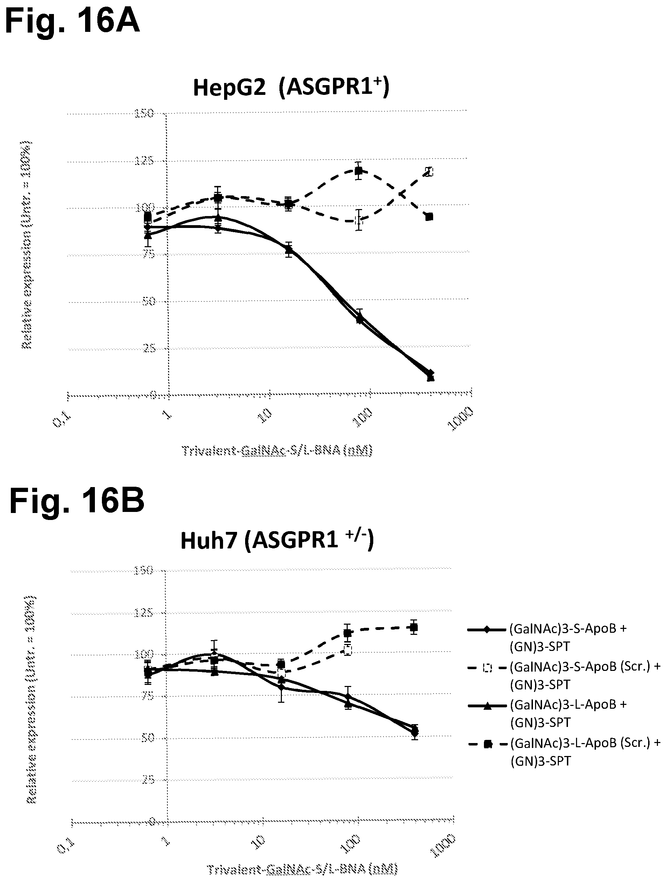

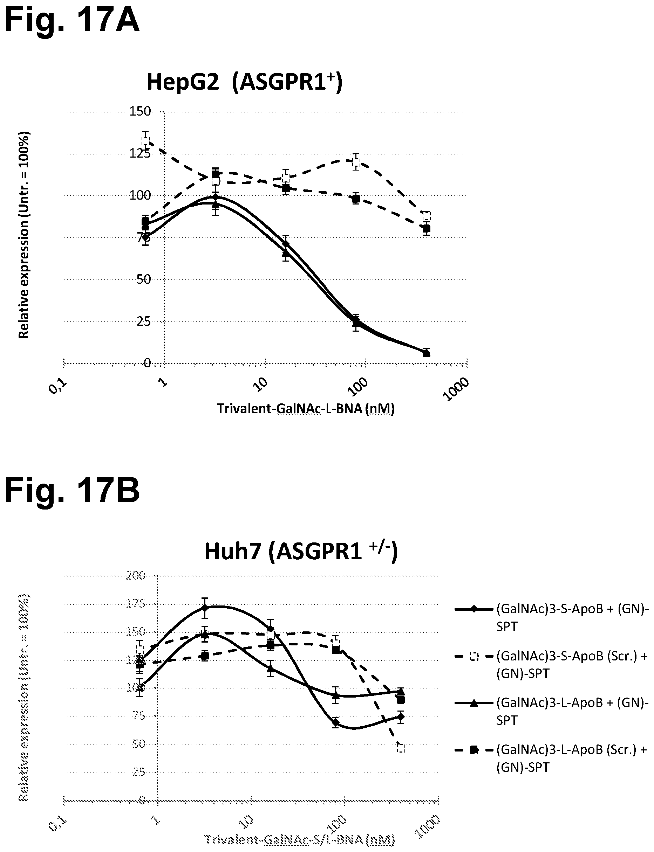

SO1861-EMCH was conjugated (labile, in a similar manner as described in Figure 2 and Figure 4 for underivatised SO1861) to trivalent-GalNAc, with a DAR = 1 for the bound SO1861, (trivalent-GalNac-SO1861). HepG2 (ASGPR1⁺) cells or Huh7 (ASPGR1^{+/-}) cells were treated with a range of concentrations of trivalent-GalNAc-L-ApoBBNA (labile conjugation Figure 16), trivalent-GalNAc-S-ApoBBNA (stable conjugation (Figure 17) or the scrambled off-target control conjugates (trivalent-GalNAc-L-ApoB_{scaambled}BNA or trivalent-GalNAc-S-ApoB_{scrambled}BNA) in combination with a fixed concentration of 4000 nM trivalent-GalNAc-L-SO1861 (DAR = 1 for the bound SO1861). ApoB gene silencing HepG2 (ASGPR1⁺) cells and Huh7 (ASPGR1^{+/-}) cells was determined. Only in combination with 4000 nM trivalent-GalNAc-L-SO1861 effective gene silencing was observed at low concentrations of trivalent-GalNAc-L-ApoBBNA (IC50 = 50 nM) or trivalent-GalNAc-S-ApoBBNA (IC50 = 50 nM) in HepG2 cells (ASGPR1⁺). Both combination treatments in Huh7 cells (ASGPR1^{+/-}) showed less effective gene silencing (trivalent-GalNAc-L-ApoBBNA: IC50 = 700 nM; trivalent-GalNAc-S-ApoBBNA: IC50 = 700 nM). Equivalent concentrations of trivalent-GalNAc-L-ApoBBNA (HepG2/Huh7: IC50> 1000nM) or trivalent-GalNAc-S-ApoBBNA (HepG2/Huh7: IC50 > 1000 nM) did not show gene silencing activity in HepG2 and Huh7 cell lines, nor did 4000 nM trivalent-GalNAc-L-SO1861 induce/enhance gene silencing of scrambled trivalent-GalNAc-L-ApoB_{scrambled}BNA (HepG2/Huh7: IC50 > 1000 nM) or scrambled trivalent-GalNAc-S-ApoB_{scrambled}BNA (HepG2/Huh7: IC50 > 1000 nM) (Figure 16). The treatments did not affect the viability of both cell lines (Figure 17).

All this shows that in combination with trivalent-GalNAc-L-SO1861, very low concentrations of trivalent-GalNAc-L-HSP27BNA or trivalent-GalNAc-S-HSP27BNA effectively induce gene silencing in ASGPR1 expressing cells, and again stronger effects are seen in cells with a higher ASGPR1 expression.

### Example 4 (GalNAc)3-BNA + SO1861-EMCH in human primary hepatocytes

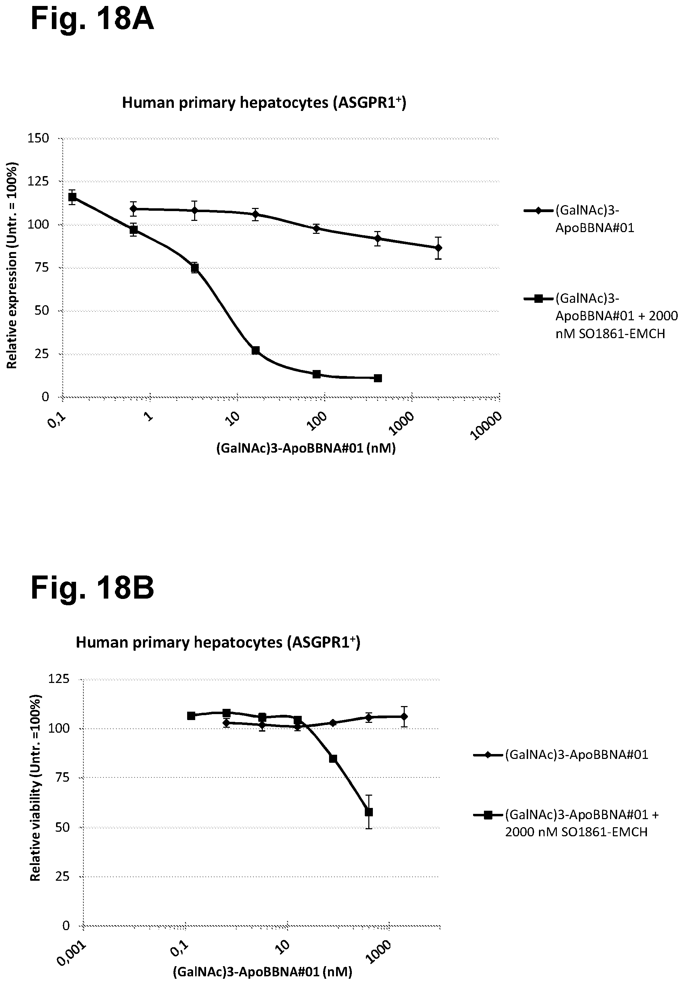

ApoBBNA (ApoBBNA#01, targeting human ApoB mRNA) was conjugated to trivalent-GalNAc ((GalNAc)3) (Figure 6) and primary hepatocytes (ASGPR1⁺) cells were treated with a range of concentrations of (GalNAc)3-ApoBBNA#01 with and without addition of 2000 nM SO1861. ApoB gene silencing in primary hepatocytes (ASGPR1⁺) cells was determined. Only in combination with 2000 nM, SO1861-EMCH effective gene silencing was observed at low concentrations of (GalNAc)3-ApoBBNA#01 (IC50 = 8 nM) in primary hepatocytes, whereas (GaINAc)3-ApoBBNA#01 alone revealed an IC50> 1000 nM (Figure 18A). Next the effect on cell viability of these treatments was determined and this revealed, (GalNAc)3-ApoBBNA#01: IC50> 10000 nM and (GalNAc)3-ApoBBNA + SO1861-EMCH: IC50 ca 500 nM (Figure 18B). All this shows that SO1861-EMCH can strongly enhance (GalNAc)3-targeted delivery of an oligonucleotide payload in human primary hepatocytes.

### Example 5 (GalNAc)3-BNA + SO1861-EMCH in human primary hepatocytes

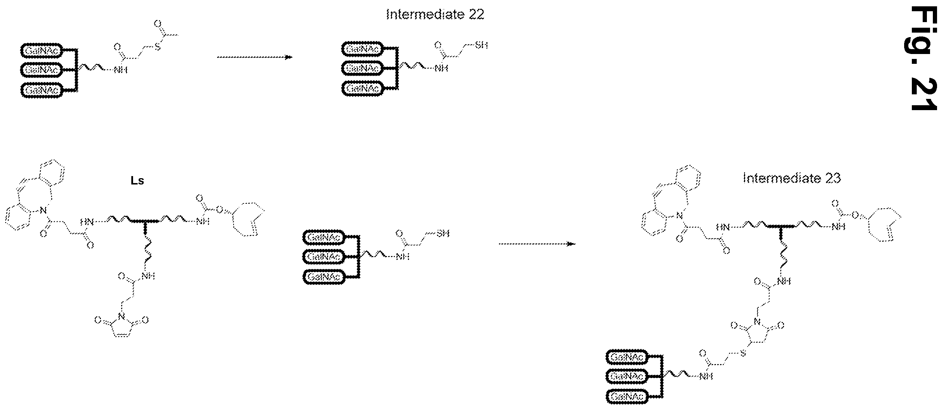

An ApoB-sequence (targeting both, mouse and human ApoB RNA) BNA, more specifically ApoB#02 BNA^{NC}, or ApoB#02 or ApoBBNA#02 or thiol13-ApoB BNA, was conjugated to a linker ('Ls') revealing (GaINAc)3-ApoB#02 with a DAR = 1 with respect to bound ApoB#02 (FIG 21-23).

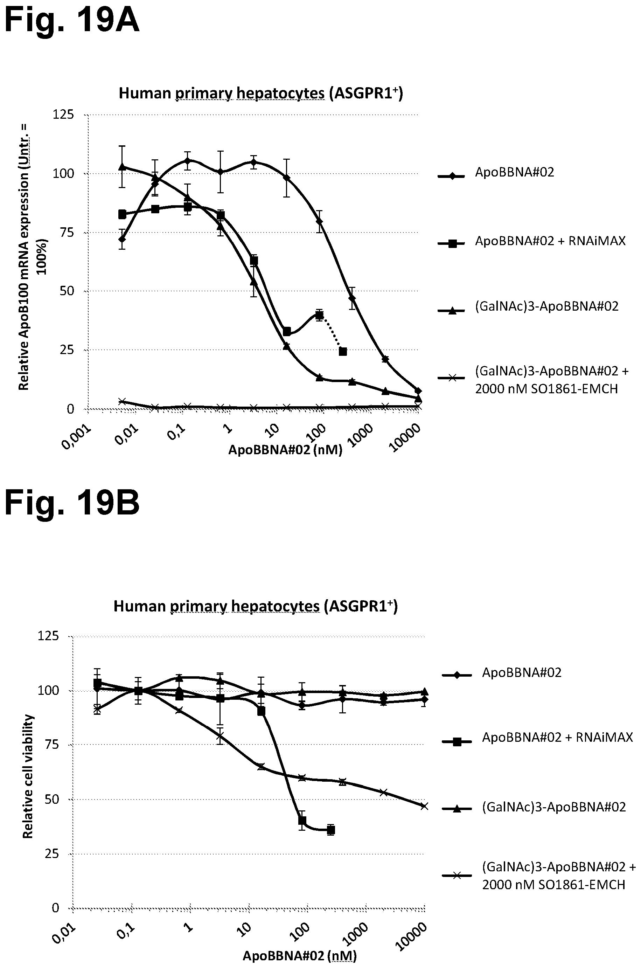

Human primary hepatocytes (ASGPR1⁺) were treated with a range of concentrations (GaINAc)3-ApoB#02 BNA with and without 2000 nM SO1861-EMCH. ApoB gene silencing in primary hepatocytes (ASGPR1⁺) cells was determined. Only in combination with 2000 nM SO1861-EMCH effective gene silencing was observed at very low concentrations of (GalNAc)3-ApoBBNA#02 (IC50 < 0.01 nM), whereas equivalent concentrations ApoBBNA#02 (IC50 = 200 nM), (GalNAc)3-ApoBBNA#02 (IC50 = 5 nM), or ApoBBNA#02 RNAiMAX transfection (IC50 = 5 nM) where less potent (Figure 19A). The dotted line in the ApoBBNA#02 RNAiMAX indicates concentrations at which toxicity and cell death occurs. Next the effect on cell viability of these treatments was determined and this revealed, ApoBBNA#02: IC50 > 10000; (GalNAc)3-ApoBBNA#02: IC50 > 10000 nM; (GalNAc)3-ApoBBNA#02 *+* SO1861-EMCH: IC50 = 5000 nM; ApoBBNA#02 *+* RNAiMAX: IC50 = 100 nM (Figure 19B). All this shows that SO1861-EMCH can strongly enhance (GalNAc)3-targeted delivery of an oligonucleotide payload in human primary hepatocytes.

### Example 6 (GalNAc)3-BNA + SO1861-EMCH in primary mouse hepatocytes

A murine ApoB-sequence targeting BNA, more specifically ApoB#02 BNA^{NC}, or ApoB#02 or ApoBBNA#02 or thiol13-ApoB BNA, was conjugated to a linker ('Ls') revealing (GaINAc)3-ApoB#02 with a DAR = 1 with respect to bound ApoB#02 (Figure 21-23).

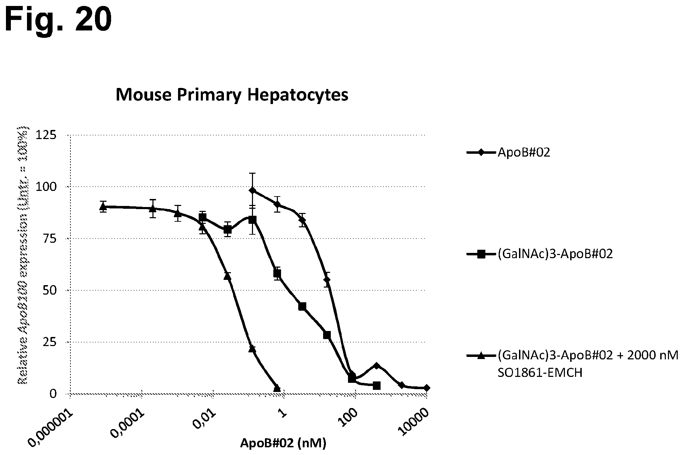

Primary mouse hepatocytes were treated with a range of concentrations of either ApoB#02 BNA, (GalNAc)3-ApoB#02, or (GalNAc)3-ApoB#02 *+* 2000 nM SO1861-EMCH and *ApoB* RNA expression was determined by qPCR after 72 hrs incubation. Gene expression inhibition, was observed at low concentrations of (GaINAc)3-ApoB#02 BNA (IC50 ca 0.03 nM) in combination with 2000 nM SO1861-EMCH (Figure 20). Treatment with ApoB#02 alone was approximately 100 - 667-fold less potent in *ApoB* RNA downmodulation (ApoB#02: IC50 = 20 nM), while (GalNAc)3-ApoB#02 with an ApoB#02 IC50 of ca. 2 nM was about 10 - 67-fold less potent (Figure 20).

This shows that only in combination with (GalNAc)3-SO1861 or SO1861-EMCH, very low concentrations of (GaINAc)3-ApoB#02 BNA are effectively inducing *ApoB* RNA downmodulation, i.e., gene silencing in murine primary hepatocytes.

### Materials and methods

### Abbreviations

- AEM: *N*-(2-Aminoethyl)maleimide trifluoroacetate salt
- AMPD: 2-Amino-2-methyl-1,3-propanediol
- BOP: (Benzotriazol-1-yloxy)tris(dimethylamino)phosphonium hexafluorophosphate
- DIPEA: N,N-diisopropylethylamine
- DMF: N,N-dimethylformamide
- EDCl.HCl: 3-((Ethylimino)methyleneamino)-N,N-dimethylpropan-1-aminium chloride
- EMCH.TFA: N-(ε-maleimidocaproic acid) hydrazide, trifluoroacetic acid salt
- HATU: 1-[Bis(dimethylamino)methylene]-1H-1,2,3-triazolo[4,5-b]pyridinium 3-oxid hexafluorophosphate
- min: minutes
- NMM: 4-Methylmorpholine
- r.t.: retention time
- TCEP: tris(2-carboxyethyl)phosphine hydrochloride
- Temp: temperature
- TFA: trifluoroacetic acid

### Analytical methods

### LC-MS method 1

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: neg or neg/pos within in a range of 1500-2400 or 2000-3000; ELSD: gaspressure 40 psi, drift tube temp: 50°C; column: Acquity C18, 50×2.1 mm, 1.7 µm Temp: 60ºC, Flow: 0.6 mL/min, lin. Gradient depending on the polarity of the product:
^{A}t₀ = 2% A, t_{5.0min} = 50% A, t_{6.0min} = 98% A
^{B}t₀ = 2% A, t_{5.0min} = 98% A, t_{6.0min} = 98% A
Posttime: 1.0 min, Eluent A: acetonitrile, Eluent B: 10 mM ammonium bicarbonate in water (pH=9.5).

### LC-MS method 2

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gaspressure 40 psi, drift tube temp: 50°C; column: Waters XSelect^{™} CSH C18, 50×2.1 mm, 2.5 µm, Temp: 25°C, Flow: 0.5 mL/min, Gradient: t₀ₘᵢₙ = 5% A, t_{2.0min} = 98% A, t_{2.7min} = 98% A, Posttime: 0.3 min, Eluent A: acetonitrile, Eluent B: 10 mM ammonium bicarbonate in water (pH=9.5).

### LC-MS method 3

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product pos/neg 105-800, 500-1200 or 1500-2500; ELSD: gaspressure 40 psi, drift tube temp: 50°C; column: Waters XSelect^{™} CSH C18, 50×2.1 mm, 2.5µm, Temp: 40°C, Flow: 0.5 mL/min, Gradient: t₀ₘᵢₙ = 5% A, t_{2.0min} = 98% A, t_{2.7min} = 98% A, Posttime: 0.3 min, Eluent A: 0.1% formic acid in acetonitrile, Eluent B: 0.1% formic acid in water.

### LC-MS method 4

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: pos/neg 100-800 or neg 2000-3000; ELSD: gaspressure 40 psi, drift tube temp: 50°C column: Waters Acquity Shield RP18, 50×2.1 mm, 1.7 µm, Temp: 25ºC, Flow: 0.5 mL/min, Gradient: t₀ₘᵢₙ = 5% A, t_{2.0min} = 98% A, t_{2.7min} = 98% A, Posttime: 0.3 min, Eluent A: acetonitrile, Eluent B: 10 mM ammonium bicarbonate in water (pH=9.5).

### Preparative methods

### Preparative MP-LC method 1

Instrument type: Reveleris^{™} prep MPLC; column: Waters XSelect^{™} CSH C18 (145×25 mm, 10 µm); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient:
^{A}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
^{A}t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B
; Detection UV: 210, 235, 254 nm and ELSD.

### Preparative MP-LC method 2

Instrument type: Reveleris^{™} prep MPLC; Column: Phenomenex LUNA C18(3) (150×25 mm, 10 µm); Flow: 40 mL/min; Column temp: room temperature; Eluent A: 0.1% (v/v) Formic acid in water, Eluent B: 0.1% (v/v) Formic acid in acetonitrile; Gradient:
^{A}t₀ₘᵢₙ = 5% B, t₁ₘᵢₙ = 5% B, t₂ₘᵢₙ = 20% B, t₁₇ₘᵢₙ = 60% B, t₁₈ₘᵢₙ = 100% B, t₂₃ₘᵢₙ = 100% B
^{B}t₀ₘᵢₙ = 2% B, t₁ₘᵢₙ = 2% B, t₂ₘᵢₙ = 2% B, t₁₇ₘᵢₙ = 30% B, t₁₈ₘᵢₙ = 100% B, t₂₃ₘᵢₙ = 100% B
^{C}t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B
^{D}t₀ₘᵢₙ = 5% B, t₁ₘᵢₙ = 5% B, t₂ₘᵢₙ = 5% B, t₁₇ₘᵢₙ = 40% B, t₁₈ₘᵢₙ = 100% B, t₂₃ₘᵢₙ = 100% B
; Detection UV : 210, 235, 254 nm and ELSD.

### Preparative LC-MS method 3

MS instrument type: Agilent Technologies G6130B Quadrupole; HPLC instrument type: Agilent Technologies 1290 preparative LC; Column: Waters XSelect^{™} CSH (C18, 150×19 mm, 10 µm); Flow: 25 ml/min; Column temp: room temperature; Eluent A: 100% acetonitrile; Eluent B: 10 mM ammonium bicarbonate in water pH = 9.0; Gradient:
^{A}t₀ = 20% A, t_{2.5min} = 20% A, t₁₁ₘᵢₙ = 60% A, t₁₃ₘᵢₙ = 100% A, t₁₇ₘᵢₙ = 100% A
^{B}t₀ = 5% A, t_{2.5min} = 5% A, t₁₁ₘᵢₙ = 40% A, t₁₃ₘᵢₙ = 100% A, t₁₇ₘᵢₙ = 100% A
; Detection: DAD (210 nm); Detection: MSD (ESI pos/neg) mass range: 100 - 800; Fraction collection based on DAD.

### Preparative LC-MS method 4

MS instrument type: Agilent Technologies G6130B Quadrupole; HPLC instrument type: Agilent Technologies 1290 preparative LC; Column: Waters XBridge Protein (C4, 150×19 mm, 10 µm); Flow: 25 ml/min; Column temp: room temperature; Eluent A: 100% acetonitrile; Eluent B: 10 mM ammonium bicarbonate in water pH=9.0; Gradient:
^{A}t₀ = 2% A, t_{2.5min} = 2% A, t₁₁ₘᵢₙ = 30% A, t₁₃ₘᵢₙ = 100% A, t₁₇ₘᵢₙ = 100% A
^{B}t₀ = 10% A, t_{2.5min} = 10% A, t₁₁ₘᵢₙ = 50% A, t₁₃ₘᵢₙ = 100% A, t₁₇ₘᵢₙ = 100% A
^{C}t₀ = 5% A, t_{2.5min} = 5% A, t₁₁ₘᵢₙ = 40% A, t₁₃ₘᵢₙ = 100% A, t₁₇ₘᵢₙ = 100% A
; Detection: DAD (210 nm); Detection: MSD (ESI pos/neg) mass range: 100 - 800; Fraction collection based on DAD

### Flash chromatography

Grace Reveleris X2^{®} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50bar (725psi); Detection: UV 200-400nm, combination of up to 4 UV signals and scan of entire UV range, ELSD; Column sizes: 4-330g on instrument, luer type, 750g up to 3000g with optional holder.

### S01861-EMCH synthesis (SO1861-EMCH is also referred to as SO1861-Ald-EMCH)

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC. Fractions corresponding to the product were immediately pooled together, frozen and lyophilized overnight to give the title compound (120 mg, 90%) as a white fluffy solid. Purity based on LC-MS 96%.
LRMS (m/z): 2069 [M-1]¹⁻
LC-MS r.t. (min): 1.08⁴

### S01861-EMCH-mercaptoethanol (SO1861-EMCH (blocked))

To SO1861-EMCH (0.1 mg, 48 nmol) 200 µL mercaptoethanol (18 mg, 230 µmol) was added and the solution was shaken for 1 h at 800 rpm and room temperature on a ThermoMixer C (Eppendorf). After shaking for 1 h, the solution was diluted with methanol and dialyzed extensively for 4 h against methanol using regenerated cellulose membrane tubes (Spectra/Por 7) with a MWCO of 1 kDa. After dialysis, an aliquot was taken out and analyzed via MALDI-TOF-MS.

MALDI-TOF-MS (RP mode): *m*/*z* 2193 Da ([M+K]⁺, SO1861-EMCH-mercaptoethanol), *m*/*z* 2185 Da ([M+K]⁺, SO1861-EMCH-mercaptoethanol), *m*/*z* 2170 Da ([M+Na]⁺, SO1861-EMCH-mercaptoethanol).

### Trivalent GalNAc-azide synthesis

### Intermediate 1:

### tert-butyl 1-azido-17,17-bis((3-(tert-butoxy)-3-oxopropoxy)methyl)-15-oxo-3,6,9,12,19-pentaoxa-16-azadocosan-22-oate

To di-tert-butyl 3,3'-((2-amino-2-((3-(tert-butoxy)-3-oxopropoxy)methyl)propane-1,3-diyl)bis(oxy))dipropionate (1.27 g, 2.51 mmol) was added a solution of 3-Azido(peg4)propionic acid N-hydroxysuccinimide ester (977 mg, 2.51 mmol) in DMF (10 mL). Next, DIPEA (657 µL, 3.77 mmol) was added and the reaction mixture was stirred overnight at room temperature. The reaction mixture was evaporated *in vacuo* and the residue was dissolved in ethyl acetate (100 mL). The resulting solution was washed with 0.5 N potassium bisulphate solution (2 × 100 mL) and brine (100 mL), dried over Na₂SO₄, filtered and evaporated in vacuo. The residue was purified by flash chromatography (DCM - 10% methanol in DCM (v/v) gradient 100:0 rising to 0:100) to give the title compound (1.27 g, 65%) as a colorless oil. Purity based on LC-MS 100% (ELSD).
LRMS (m/z): 780 [M+1]¹⁺
LC-MS r.t. (min): 2.10²

### Intermediate 2:

### 1-azido-17,17-bis((2-carboxyethoxy)methyl)-15-oxo-3,6,9,12,19-pentaoxa-16-azadocosan-22-oic acid

To a solution of tert-butyl 1-azido-17,17-bis((3-(tert-butoxy)-3-oxopropoxy)methyl)-15-oxo-3,6,9,12,19-pentaoxa-16-azadocosan-22-oate (1.27 g, 1.63 mmol) in DCM (5.0 mL) was added TFA (5.0 mL, 65 mmol). The reaction mixture was stirred at room temperature. After 1.5 hours the reaction mixture was evaporated *in vacuo,* co-evaporated with toluene (3 × 10 mL) and DCM (3 × 10 mL) to give the crude title product as a colorless oil.
LRMS (m/z): 611 [M+1]¹⁺

### Intermediate 3:

### di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate

1-azido-17,17-bis((2-carboxyethoxy)methyl)-15-oxo-3,6,9,12,19-pentaoxa-16-azadocosan-22-oic acid (997 mg, 1.63 mmol), Oxyma Pure (1.04 g, 7.35 mmol) and EDCl.HCl (1.17 g, 6.12 mmol) were dissolved in DMF (10.0 mL). Next, DIPEA (1.99 mL, 11.4 mmol) was added, followed directly by the addition of a solution of N-BOC-1,3-propanediamine (1.07 g, 6.12 mmol) in DMF (10.0 mL). The reaction mixture was stirred overnight at room temperature. The reaction mixture was evaporated *in vacuo* and the residue was dissolved in ethyl acetate (100 mL). The resulting solution was washed with 0.5 N potassium bisulphate solution (100 mL), saturated sodium bicarbonate solution (2 × 100 mL) and brine (100 mL), dried over Na₂SO₄, filtered and evaporated *in vacuo.* The residue was purified by flash chromatography (DCM - 10% methanol in DCM (v/v) gradient 0:100 rising to 100:0, staying at 100:0 until the product eluted ) to give the title compound (1.16 g, 66%) as a yellowish viscous oil. LC-MS 99% (ELSD).
LRMS (m/z): 1080 [M+1]¹⁺
LC-MS r.t. (min): 1.51³

### Intermediate 4:

### 3,3'-((2-((3-((3-aminopropyl)amino)-3-oxopropoxy)methyl)-2-(1-azido-3,6,9,12-tetraoxapentadecan-15-amido)propane-1,3-diyl)bis(oxy))bis(N-(3-aminopropyl)propanamide) tris(2,2,2-trifluoroacetate) synthesis

To a solution of di-tert-butyl (10-(1-azido-3,6,9,12-tetraoxapentadecan-15-amido)-10-(13,13-dimethyl-5,11-dioxo-2,12-dioxa-6,10-diazatetradecyl)-5,15-dioxo-8,12-dioxa-4,16-diazanonadecane-1,19-diyl)dicarbamate (1.16 g, 1.08 mmol) in DCM (10 mL) was added TFA (10 mL, 131 mmol). The reaction mixture was stirred at room temperature. After 2 hours the reaction mixture was evaporated *in vacuo,* co-evaporated with toluene (3 × 10 mL) and DCM (3 × 10 mL) to give the crude title product as a yellowish viscous oil.
LRMS (m/z): 260 [M+3]³⁺, 390 [M+2]²⁺, 780 [M+1]¹⁺,

### Intermediate 5:

### (2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate

5-(((2R,3R,4R,5R,6R)-3-acetamido-4,5-diacetoxy-6-(acetoxymethyl)tetrahydro-2H-pyran-2-yl)oxy)pentanoic acid (obtain according J. Am. Chem Soc., 2014, 136, 16958-16961, 3.00 g, 6.70 mmol) and *N*-Hydroxysuccinimide (926 mg, 8.05 mmol) were dissolved in DCM (50 mL). Next, EDCl.HCl (1.54 g, 8.05 mmol) and 4-(Dimethylamino)pyridine (82 mg, 0.67 mmol) were added and the reaction mixture was stirred overnight at room temperature. The reaction mixture was diluted with DCM and the resulting solution was washed with 0.5 N potassium bisulphate solution (150 mL), saturated sodium bicarbonate solution (150 mL) and brine (150 mL), dried over Na₂SO₄, filtered and evaporated *in vacuo* to give the title compound (3.60 g, 99%) as a white foam. Purity based on LC-MS 99% (ELSD).
LRMS (m/z): 545 [M+1]¹⁺
LC-MS r.t. (min): 1.07³

### Intermediate 6:

### [(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)-2-[(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl ]butoxy}-5-acetamidooxan-2-yl]methyl acetate

3,3'-((2-((3-((3-aminopropyl)amino)-3-oxopropoxy)methyl)-2-(1-azido-3,6,9,12-tetraoxapentadecan-15-amido)propane-1,3-diyl)bis(oxy))bis(N-(3-aminopropyl)propanamide) tris(2,2,2-trifluoroacetate) (1.21 g, 1.08 mmol) was dissolved in a mixture of DMF (10 mL) and DIPEA (1.69 mL, 9.70 mmol). Next, (2R,3R,4R,5R,6R)-5-acetamido-2-(acetoxymethyl)-6-((5-((2,5-dioxopyrrolidin-1-yl)oxy)-5-oxopentyl)oxy)tetrahydro-2H-pyran-3,4-diyl diacetate (2.20 g, 4.04 mmol) was added and the reaction mixture was stirred over the weekend at room temperature. Next, the reaction mixture was evaporated *in vacuo* and the residue was purified by flash chromatography (DCM - 30% methanol in DCM (v/v) gradient 0:100 rising to 100:0) to give the title compound (1.84 g, 83%) as a yellowish foam. LC-MS 95% (ELSD).
LRMS (m/z): 2068 [M+1]¹⁺
LC-MS r.t. (min): 1.18³

### Intermediate 7:

### Trivalent GalNAc-azide

[(3R,6R)-3,4-bis(acetyloxy)-6-{4-[(3-{3-[2-(1-azido-3,6,9,12-tetraoxapentadecan-15-amido)-3-(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)-2-[(2-{[3-(5-{[(2R,5R)-4,5-bis(acetyloxy)-6-[(acetyloxy)methyl]-3-acetamidooxan-2-yl]oxy}pentanamido)propyl]carbamoyl}ethoxy)methyl]propoxy]propanamido}propyl)carbamoyl]butoxy}-5-acetamidooxan-2-yl]methyl acetate (300 mg, 0.145 mmol) was dissolved in a mixture of triethylamine (2.00 mL, 14.4 mmol), methanol (2.00 mL) and water (2.00 mL) and the reaction mixture was stirred at room temperature. After 2 hours the reaction mixture was evaporated *in vacuo.* The residue was purified by preparative MP-LC.^{2B} Fractions corresponding to the product were immediately pooled together, frozen and lyophilized overnight to give the title compound (164 mg, 67%) as a white solid. Purity based on LC-MS 97%.
LRMS (m/z): 1688 [M-1]¹⁻
LC-MS r.t. (min): 1.99^{1A}

### Trivalent-GalNAc-SO1861 and GalNAc-SO1861 synthesis (Figure 2, 4)

### Intermediate 8:

### SO1861-NH₂

SO1861-azide (6.89 mg, 3.20 µmol) was dissolved in mixture of 32 mM potassium carbonate (150 µL, 4.80 µmol) and acetonitrile (150 µL). Directly afterwards, a 1.0 M trimethylphosphine solution in THF (32 µL, 32 µmol) was added and the resulting mixture was shaken for 1 min and left standing at room temperature. After 30 min. the reaction mixture was subjected to preparative MP-LC. Fractions corresponding to the product were immediately pooled together, frozen and lyophilized overnight to give the title compound (5.30 mg, 78%) as a white fluffy solid. Purity based on LC-MS 94%.
LRMS (m/z): 1062 [M-2]²-
LC-MS r.t. (min): 2.51^{1B}

### Intermediate 9:

### SO1861-DBCO

To SO1861-amine (48.6 mg, 22.9 µmol) and DBCO-NHS (13.0 mg, 32.4 µmol) was added to a solution of DIPEA (5.98 µL, 34.3 µmol) and DMF (2.0 mL). The reaction mixture was shaken for 1 min and left standing at room temperature. After 4 hours the reaction mixture was diluted with a solution of 50 mM sodium bicarbonate (1.00 mL, 50 µmol). The resulting mixture was shaken for 1 min and left standing at room temperature. After 30 min the reaction mixture was subjected to preparative MP-LC.^{1B} Fractions corresponding to the product were immediately pooled together, frozen and lyophilized overnight to give the title compound (16.9 mg, 31%) as a white fluffy solid. Purity based on LC-MS 94%.
LRMS (m/z): 2412 [M-1]¹-
LC-MS r.t. (min): 2.45^{1B}

### S01861-L-trivalent GalNAc synthesis

SO1861-DBCO (7.50 mg, 3.11 µmol) and trivalent GalNAc-azide (5.31 mg, 3.14 µmol) were dissolved in a mixture of water/acetonitrile (2:1, v/v, 0.90 mL). The reaction mixture was shaken for 1 min and left standing at room temperature. After 2 hours the reaction mixture was subjected to preparative LC-MS.^{3B} Fractions corresponding to the product were immediately pooled together, frozen and lyophilized overnight to give the title compound (9.60 mg, 75%) as a white fluffy solid. Purity based on LC-MS 99%.
LRMS (m/z): 2050 [M-2]²⁻
LC-MS r.t. (min): 2.04^{1B}

### SO1861-L-GaINAc synthesis

SO1861-DBCO (1.75 mg, 0.73 µmol) and GalNAc-PEG3-azide (0.82 mg, 2.18 µmol) were dissolved in a mixture of water/acetonitrile (1:1, v/v, 1.00 mL). The reaction mixture was shaken for 1 min and left standing at room temperature. After 2 hours the reaction mixture was subjected to preparative LC-MS.^{3B} Fractions corresponding to the product were immediately pooled together, frozen and lyophilized overnight to give the title compound (1.63 mg, 81%) as a white fluffy solid. Purity based on LC-MS 100%.
LRMS (m/z): 2790 [M-1]¹⁻
LC-MS r.t. (min): 2.15^{1B}

### Trivalent GaINAc-BNA oligo synthesis (Figure 6, 7)

### Intermediate 10:

### 4-{2-azatricyclo[10.4.0.0^{4,9}]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide

4-{2-azatricyclo[10.4.0.0^{4,9}]hexadeca-1(12),4(9),5,7,13,15-hexaen-10-yn-2-yl}-N-{2-[2-(2-{2-[(4-formylphenyl)formamido]ethoxy}ethoxy)ethoxy]ethyl}-4-oxobutanamide (25.0 mg, 40.9 µmol) and EMCH.TFA (20.8 mg, 61.3 µmol) were dissolved in methanol (extra dry, 2.00 mL). Next, TFA (9.39 µL, 123 µmol) was added. The reaction mixture was shaken for 1 min and left standing overnight at room. The reaction mixture was subjected to preparative MP-LC.^{1B} Fractions corresponding to the product were immediately pooled together, frozen and lyophilized overnight to give the title compound (16.2 mg, 48%) as a white solid. Purity based on LC-MS 91%.
LRMS (m/z): 410.2 [M+2]²⁺
LC-MS r.t. (min): 1.41²

### Intermediate 11:

### Trivalent GalNAc-L-maleimide

Trivalent GalNAc-azide (20.3 mg, 12.0 µmol) and 4-{2-azatricyclo[10.4.0.0^{4,9}]hexadeca-1(12),4(9),5,7,13,15-hexaen-10-yn-2-yl}-N-[2-(2-{2-[2-({4-[(E)-{[6-(2,5-dioxo-2,5-dihydro-1H-pyrrol-1-yl)hexanamido]imino}methyl]phenyl}formamido)ethoxy]ethoxy}ethoxy)ethyl]-4-oxobutanamide (11.8 mg, 14.4 µmol) were dissolved in a mixture of water/acetonitrile (2:1, v/v, 0.90 mL). The reaction mixture was shaken for 1 min and left standing at room temperature. After 2 hours the reaction mixture was subjected to preparative MP-LC.^{2C} Fractions corresponding to the product were immediately pooled together, frozen and lyophilized overnight to give the title compound (25.6 mg, 85%) as a white solid. Purity based on LC-MS 88%.
LRMS (m/z): 2101 [M-405]¹⁻, 2304 [M-202]¹⁻, 2507 [M-1]¹⁻
LC-MS r.t. (min): 1.90^{1B} (double peaks due to isomers)

### Intermediate 12:

### Trivalent GalNAc-S-maleimide

Trivalent GalNAc-azide (20.3 mg, 12.0 µmol) and DBCO-maleimide (10.3 mg, 24.0 µmol) were dissolved in a mixture of water/acetonitrile (2:1, v/v, 0.90 mL). The reaction mixture was shaken for 1 min and left standing at room temperature. After 2 hours the reaction mixture was subjected to preparative MP-LC.^{2D} Fractions corresponding to the product were immediately pooled together, frozen and lyophilized overnight to give the title compound (22.2 mg, 87%) as a white solid. Purity based on LC-MS 85%. Contains 10% of the hydrolysed maleimide.
LRMS (m/z): 2115 [M-1]¹-
LC-MS r.t. (min): 1.60^{1B} (double peaks due to isomers)

### Trivalent GalNAc-S-ApoB (or HSP27) BNA oligo synthesis

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-S-maleimide (3.02 mg, 1.43 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (6.75 mg, quant.) as a white fluffy solid. Purity based on LC-MS 94% (very broad peak).
LRMS (m/z): 2318 [M-4]⁴-
LC-MS r.t. (min): 1.65^{1A}

### Trivalent GalNAc-S-ApoB (or HSP27) scrambled BNA oligo synthesis

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-S-maleimide (3.09 mg, 1.46 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (5.91 mg, 93%) as a white fluffy solid. Purity based on LC-MS 88% (very broad peak).
LRMS (m/z): 2311 [M-4]⁴-
LC-MS r.t. (min): 1.60^{1A}

### Trivalent GalNAc-L-ApoB (or HSP27) BNA oligo synthesis

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.63 mg, 1.45 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (6.68 mg, quant.) as a white fluffy solid. Purity based on LC-MS 99% (very broad peak).
LRMS (m/z): 2416 [M-4]⁴-
LC-MS r.t. (min): 1.97^{1A}

### Trivalent GalNAc-L-ApoB (or HSP27) scrambled BNA oligo synthesis

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate/acetonitrile (3:1, v/v, 1.00 mL) and the resulting solution was directly added to trivalent GalNAc-L-maleimide (3.68 mg, 1.47 µmol). The reaction mixture was shaken for 1 min and left standing at room temperature. After 1.5 hours the reaction mixture was subjected to preparative LC-MS.^{4A} Fractions corresponding to the product were immediately pooled together, frozen and lyophilized overnight to give the title compound (4.71 mg, 71%) as a white fluffy solid. Purity based on LC-MS 96% (very broad peak).
LRMS (m/z): 2409 [M-4]⁴⁻
LC-MS r.t. (min): 1.93^{1A}

### Dendron(-L-SO1861)₄-trivalent GalNAc and dendron(-L-SO1861)₈-trivalent GalNAc synthesis

### Intermediate 13:

### Trivalent GalNAc-amine formate

Trivalent GalNAc-azide (36.5 mg, 21.6 µmol) was dissolved in a solution of potassium carbonate (5.97 mg, 43.2 µmol) in water (1.00 mL) and acetonitrile (1.00 mL). Next, a 1.0 M trimethylphosphine solution in THF (216 µL, 216 µmol) was added and the resulting mixture was shaken for 1 min and left standing at room temperature. After 45 min the reaction mixture was evaporated *in vacuo* and the residue was dissolved in water/acetonitrile (9:1, v/v, 1 mL). The resulting solution was directly subjected to preparative MP-LC.^{2B} Fractions corresponding to the product were immediately pooled together, frozen and lyophilized overnight to give the title compound (36.1 mg, 98%) as a white solid. Purity based on LC-MS 100%.
LRMS (m/z): 1662 [M-1]¹-
LC-MS r.t. (min): 1.62^{1A}

### Intermediate 14:

### Trivalent GalNAc-DBCO

Trivalent GalNAc-amine formate (17.4 mg, 10.2 µmol) and DBCO-NHS (6.14 mg, 15.3 µmol) were dissolved in a solution of NMM (2.24 µL, 20.3 µmol) in DMF (0.50 mL). The reaction mixture was shaken for 1 min and left standing at room temperature. After 2 hours the reaction mixture was evaporated *in vacuo* and the residue was dissolved in water/acetonitrile (8:2, v/v, 1 mL). The resulting solution was directly subjected to preparative MP-LC.^{2C} Fractions corresponding to the product were immediately pooled together, frozen and lyophilized overnight to give the title compound (14.2 mg, 72%) as a white solid. Purity based on LC-MS 96%.
LRMS (m/z): 1950 [M-1]¹
LC-MS r.t. (min): 1.86^{1B}

### Intermediate 15:

### dendron(-L-SO1861)₈-azide

Dendron(-L-SO1861)s-amine (19.6 mg, 1.08 µmol) and 2,5-dioxopyrrolidin-1-yl 1-azido-3,6,9,12-tetraoxapentadecan-15-oate (4.17 mg, 10.8 µmol) were dissolved in DMF (1.50 mL). Next, DIPEA (1.87 µL, 10.8 µmol) was added and the mixture was shaken for 1 min and left standing overnight at room temperature. The reaction mixture was subjected to preparative LC-MS.^{4B} Fractions corresponding to the product were immediately pooled together, frozen and lyophilized overnight to give the title compound (11.7 mg, 59%) as a white fluffy solid. Purity based on LC-MS 92% (very broad peak).
LRMS (m/z): 2316 [M-8]⁸⁻, ): 2647 [M-7]⁷-
LC-MS r.t. (min): 4.29^{1A}

### Dendron(-L-SO1861)₄-trivalent GalNAc synthesis

Dendron(-L-SO1861 )₄-azide (2.50 mg, 0.266 µmol) and trivalent GalNAc-DBCO (1.56 mg, 0.799 µmol) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.74 mg, 91%) as a white fluffy solid. Purity based on LC-MS 86% (very broad peak).
LRMS (m/z): 2832 [M-4]⁴-
LC-MS r.t. (min): 4.07^{1A}

### Dendron(-L-SO1861)₈-trivalent GalNAc synthesis

Dendron(-L-SO1861)₈-azide (2.50 mg, 0.135 µmol) and trivalent GalNAc-DBCO (0.79 mg, 0.405 µmol) were dissolved in a mixture of water/acetonitrile (3:1, v/v, 1.00 mL). The reaction mixture was shaken for 1 min and left standing at room temperature. After 2 hours the reaction mixture was subjected to preparative LC-MS.^{4B} Fractions corresponding to the product were immediately pooled together, frozen and lyophilized overnight to give the title compound (2.03 mg, 74%) as a white fluffy solid. Purity based on LC-MS 100% (very broad peak).
LRMS (m/z): 2559 [M-8]⁸⁻, 2925 [M-7]⁷⁻
LC-MS r.t. (min): 4.18^{1A}

### Intermediate 16:

### Trivalent GalNAc-thioacetate

Trivalent GalNAc-amine formate (18.7 mg, 11.0 µmol) and 4-nitrophenyl 3-(acetylthio)propanoate (5.90 mg, 21.9 µmol) were dissolved in a solution of NMM (2.41 µL, 21.9 µmol) in DMF (0.50 mL). The reaction mixture was shaken for 1 min and left standing at room temperature. After 2 hours the reaction mixture was evaporated *in vacuo* and the residue was dissolved in a mixture of 0.1% formic acid in water and 0.1% formic acid in acetonitrile (9:1, v/v, 1 mL). The resulting solution was directly subjected to preparative MP-LC.^{2B} Fractions corresponding to the product were immediately pooled together, frozen and lyophilized overnight to give the title compound (13.0 mg, 66%) as a white solid. Purity based on LC-MS 100%.
LRMS (m/z): 1749 [M-43]¹⁻, 1792 [M-1]¹
LC-MS r.t. (min): 1.24^{1B}

### Intermediate 17:

### DBCO-TCO-trivalent GalNAc

Trivalent GalNAc-thioacetate (13.0 mg, 7.25 µmol) was dissolved in methanol (0.50 mL). Next, a 1 M solution of sodium hydroxide (7.98 µL, 7.98 µmol) was added. The reaction mixture was shaken for 1 min and left standing at room temperature. After 30 min the reaction mixture was added to a freshly prepared solution of trifunctional linker (Ls-t) (*i.e.,* is an example of a saponin moiety linker Ls) (7.39 mg, 6.13 µmol) in 20 mM ammonium bicarbonate/acetonitrile (3:1, v/v, 2.00 mL).

The trifunctional linker has the IUPAC name: 5,8,11,18,21,24,27-Heptaoxa-2,14,30-triazatritriacontanoic acid, 14-[16-(11,12-didehydrodibenz[*b*,*f*]azocin-5(6*H*)-yl)-13,16-dioxo-3,6,9-trioxa-12-azahexadec-1-yl]-33-(2,5-dihydro-2,5-dioxo-1*H*-pyrrol-1-yl)-15,31-dioxo-, (1*R*,4*E*)-4-cycloocten-1-yl ester. The trifunctional linker has the following formula (XVII):

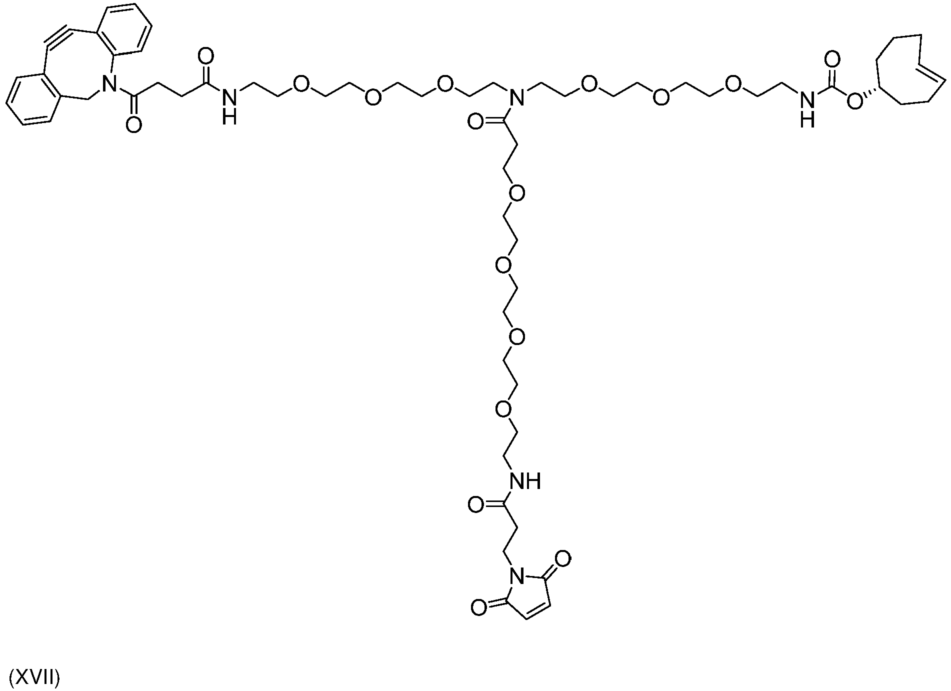

The resulting mixture was shaken for 1 min and left standing at room temperature. After 2 hours the reaction mixture was subjected to preparative MP-LC.^{2A} Fractions corresponding to the product were immediately pooled together, frozen and lyophilized overnight to give the title compound (3.83 mg, 21%) as a white solid. Purity based on LC-MS 89%.
LRMS (m/z): 2409 [M-546]¹⁻, 2955 [M-1]¹⁻
LC-MS r.t. (min): 2.66^{1B}

### Intermediate 18:

### methyltetrazine-L-ApoB BNA oligo

To ApoB BNA oligo disulfide (5.00 mg, 0.686 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

(1.36 mg, 1.73 µmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 µm syringe filter. The filtrate was lyophilized overnight to yield the title product (5.44 mg, quant) as a pink fluffy solid. Purity based on LC-MS 90% (very broad peak).
LRMS (m/z): 2648 [M-3]³⁻
LC-MS r.t. (min): 0.62⁴

### Intermediate 19:

### methyltetrazine-L-ApoB scrambled BNA oligo

To ApoB scrambled BNA oligo disulfide (5.00 mg, 0.688 µmol) was added a solution of 20 mM ammonium bicarbonate with 2.5 mM TCEP (1.00 mL, 2.5 µmol). The reaction mixture was shaken for 1 min and left standing at room temperature. After 1 hour the reaction mixture was filtered by using a centrifugal filter with a molecular weight cut-off of 3000 Da (5000 × g for 30 min, 2 × 0.50 mL). Next, the residue solution was washed twice with a solution of 20 mM ammonium bicarbonate with 2.5 mM TCEP (0.50 mL), each time filtered under the same conditions described above. As next, the residue solution was diluted with 20 mM ammonium bicarbonate (1.50 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (1.34 mg, 1.70 µmol) in acetonitrile (0.5 mL). The reaction mixture was shaken for 1 min and left standing at room temperature. After 30 min the reaction mixture was frozen and lyophilized overnight to yield the crude title product as a pink fluffy solid. To the crude product was added a solution of 20 mM ammonium bicarbonate (1.50 mL) and the resulting suspension was filtered over a 0.45 µm syringe filter. The filtrate was lyophilized overnight to yield the title product (5.48 mg, quant) as a pink fluffy solid. Purity based on LC-MS 84% (very broad peak).
LRMS (m/z): 2639 [M-3]³⁻
LC-MS r.t. (min): 0.58⁴

### Intermediate 20:

### DBCO-L-ApoB BNA oligo-trivalent GalNAc

To methyltetrazine-L-ApoB BNA oligo (5.44 mg, 0.684 µmol) and DBCO-TCO-trivalent GalNAc (2.03 mg, 0.687 µmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.^{4C} Fractions corresponding to the product were immediately pooled together, frozen and lyophilized overnight to give the title compound (2.87 mg, 39%) as a white fluffy solid. LC-MS shows multiple broad peaks with the correct m/z values.
LRMS (m/z): 2175 [M-5]⁵⁻, 2718 [M-6]⁴⁻
LC-MS r.t. (min): 2.60-3.00^{1A}

### Intermediate 21:

### DBCO-L-ApoB scrambled BNA oligo-trivalent GalNAc

To methyltetrazine-L-ApoB scrambled BNA oligo (5.48 mg, 0.692 µmol) and DBCO-TCO-trivalent GalNAc (1.80 mg, 0.609 µmol) was added 20 mM ammonium bicarbonate/acetonitrile (3:1, v/v, 1.00 mL). The reaction mixture was shaken for 1 min and left standing at room temperature. After 2 hours the reaction mixture was subjected to preparative LC-MS.^{4C} Fractions corresponding to the product were immediately pooled together, frozen and lyophilized overnight to give the title compound (2.15 mg, 33%) as a white fluffy solid. LC-MS shows multiple broad peaks with the correct m/z values.
LRMS (m/z): 2169 [M-5]⁵⁻, 2711 [M-6]⁴⁻
LC-MS r.t. (min): 2.58-3.20^{1A}

### Intermediate 22 (Figure 21)

### Trivalent GalNAc-thiol

Trivalent GalNAc-thioacetate (16.2 mg, 9.02 µmol) was dissolved in methanol (500 µL). Next, a 1.00 M solution of sodium hydroxide (11.0 µL, 11.0 µmol) was added. The reaction mixture was shaken for 1 min and left standing at room temperature. After 30 min the reaction mixture was submitted to preparative MP-LC.^{1A} Fractions corresponding to the product were immediately pooled together, frozen and lyophilized overnight to give the title compound (13.1 mg, 83%) as a white solid. Purity based on LC-MS 98%.
LRMS (m/z): 1748 [M-H]¹⁻
LC-MS r.t. (min): 1.78^{1A}

### Intermediate 23: (Figure 21)

### DBCO-TCO-trivalent GalNAc

Trifunctional linker (Ls) (15.0 mg, 12.4 µmol) was dissolved in acetonitrile (0.50 mL). Next, a solution of 20 mM ammonium bicarbonate (1.50 mL) was added and the resulting solution was directly transferred to trivalent GalNAc-thiol (22.7 mg, 13.0 µmol). The reaction mixture was shaken for 1 min and left standing at room temperature. After 1 hour the reaction mixture was frozen and lyophilized overnight to yield the crude title product as a white solid. Purity based on LC-MS 84%.
LRMS (m/z): 2409 [M-546]¹⁻, 2955 [M-1]¹⁻
LC-MS r.t. (min): 2.63^{1B}

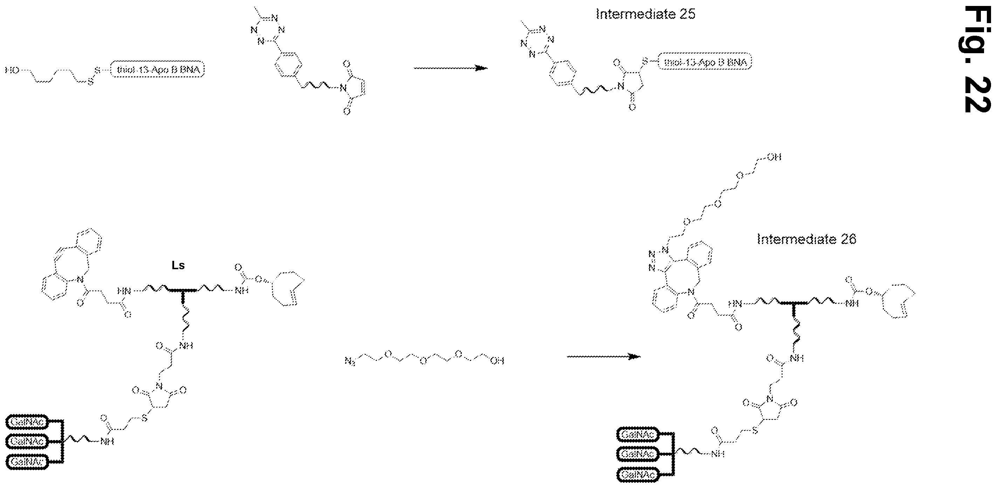

### Intermediate 25 (Figure 22):

### methyltetrazine-BNA oligo

To Thiol-13-ApoB BNA oligo disulfide (20 mg, 4.17 µmol) was added a solution of 20 mM ammonium bicarbonate with 5.0 mM TCEP (2.00 mL, 10.0 µmol). The reaction mixture was shaken for 1 min and left standing at room temperature. After 1 hour the reaction mixture was filtered by using a centrifugal filter with a molecular weight cut-off of 3000 Da (5000 × g for 30 min, 4× 0.50 mL). Next, the residue solution was diluted with 20 mM ammonium bicarbonate (3.00 mL) and the resulting mixture was directly added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-,6,9,12-tetraoxapentadecan-15-amide (7.22mg, 9.16 µmol) in acetonitrile (1.0 mL). The reaction mixture was shaken for 1 min and left standing at room temperature. After 1 hour the reaction mixture was frozen and lyophilized overnight to yield the crude title product as a pink fluffy solid. To the crude product was added a 20 mM ammonium bicarbonate/acetonitrile (2:1, v/v, 2.00 mL) and the resulting solution was subjected to preparative LC-MS.^{1A} Fractions corresponding to the product were immediately pooled together, frozen and lyophilized overnight to give the title compound (20.3 mg, 89%) as a pink fluffy solid. Purity based on LC-MS 93% (broad peak).
LRMS (m/z): 1817 [M-3]³⁻
LC-MS r.t. (min): 0.59³

### Intermediate 26 (Figure 22):

### (blocked DBCO)-TCO-trivalent GalNAc

A solution of 1-azido-3,6,9-trioxaundecane-11-ol (2.17 mg, 9.88 µmol) in DMF (0.50 mL) was added to DBCO-TCO-trivalent GalNAc (14.6 mg, 4.94 µmol. The reaction mixture was shaken for 1 min and left standing at room temperature. After 2 hours the reaction mixture was submitted to preparative MP-LC.^{1C} Fractions corresponding to the product were immediately pooled together, frozen and lyophilized overnight to give the title compound (10.00 mg, 64%) as a white solid. Purity based on LC-MS 98%.
LRMS (m/z): 1750 [fragment]
LC-MS r.t. (min): 2.33^{1B}

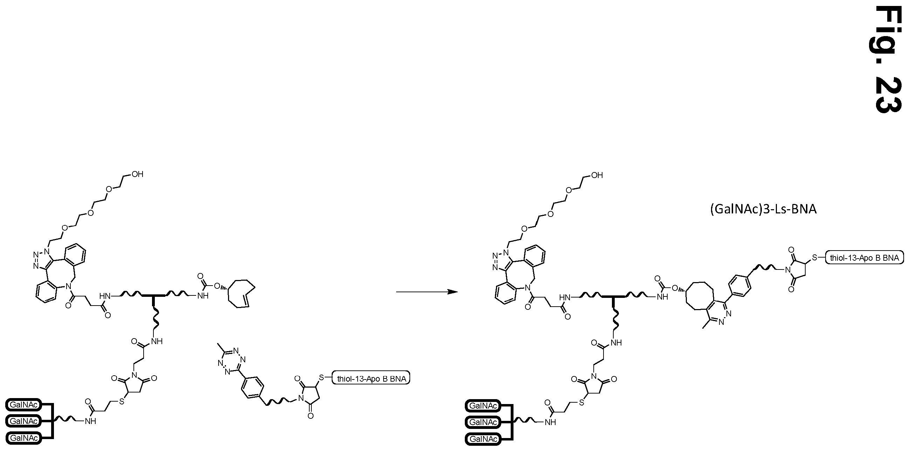

### Ls-(blocked DBCO)-BNA oligo-trivalent GalNAc (Figure 23)

(blocked DBCO)-TCO-trivalent GalNAc (10.0 mg, 3.15 µmol) and Methyltetrazine-BNA oligo (10.0 mg, 1.83 µmol) were dissolved in a solution of 20 mM ammonium bicarbonate/acetonitrile (3:1, v/v, 1.00 mL). The reaction mixture was shaken for 1 min and left standing at room temperature. After 3 hours the reaction mixture was frozen and lyophilized overnight. The crude product was dissolved in 20 mM ammonium bicarbonate (1 mL) and the resulting solution was directly subjected to preparative LC-MS.^{1B} Fractions corresponding to the product were immediately pooled together, frozen and lyophilized overnight to give the title compound (GalNAc)3-Ls-BNA) ((11.3 mg, 72%) as a white fluffy solid. Purity based on LC-MS 95% (multiple (broad) peaks due to regioisomers).
LRMS (m/z): 2149 [M-4]⁴⁻, 2866 [M-3]³⁻
LC-MS r.t. (min): 2.92^{1A}

### antiCD71-saporin synthesis

Custom CD71mab-saporin conjugate was produced and purchased from Advanced Targeting Systems (San Diego, CA). CD71 antibody (anti-CD71, clone OKT-9, InVivoMab) was purchased from BioXCell.

### HSP27BNA, ApoB and ApoB_{scrambled} oligo sequences

HSP27 (5'-GGCacagccagtgGCG-3') [SEQ ID NO: 1] (The antisense BNA (HSP27) was BNA, more specifically BNA^{NC}, with oligo nucleic acid sequence 5'-GGCacagccagtgGCG-3' according to Zhang et *al.* (2011) [Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333*]),* ApoB, also referred to as ApoB#01 (5'-GCCTCagtctgcttcGCACC-3') [SEO ID NO: 2] and ApoB_{scrambled} (5'-GGCCTctctacaccgCTCGT-3') [SEQ ID NO: 3] BNA oligos, and murine and human sequence-compatible ApoB#02 (5'-GCattggtatTCA-3') [SEQ ID NO: 12] BNA^{NC} oligonucleotides were ordered with 5'-Thiol C6 linker at Bio-Synthesis Inc., with BNA bases in capitals, and fully phosphorothioated backbones (Lewisville, Texas).

### RNA isolation and gene expression analysis cell lines

RNA from cells was isolated and analysed according to standard protocols (Biorad). qPCR primers that were used are indicated in Table A2.

**Table A2. Primers used in qPCR (human cells) are shown below:**

| Gene | Primer | Sequence (5'-3') |
|---|---|---|
| HSP27 | Forward | GCAGTCCAACGAGATCACCA [SEQ ID NO: 4] |
| | Reverse | TAAGGCTTTACTTGGCGGCA [SEQ ID NO: 5] |
| ApoB | Forward | AGGGTCCGGGAATCTGATGA [SEQ ID NO: 6] |
| | Reverse | TGGGCACGTTGTCTTTCAGAG [SEQ ID NO: 7] |
| HBMS | Forward | CACCCACACACAGCCTACTT [SEQ ID NO: 8] |
| | Reverse | GTACCCACGCGAATCACTCT [SEQ ID NO: 9] |
| GUSB | Forward | GAAAATACGTGGTTGGAGAGCT [SEQ ID NO: 10] |
| | Reverse | CCGAGTGAAGATCCCCTTTTTA [SEQ ID NO: 11] |

### Cell viability assay

After treatment the cells were incubated for 72 hr at 37°C before the cell viability was determined by a MTS-assay, performed according to the manufacturer's instruction (CellTiter 96^{®} AQueous One Solution Cell Proliferation Assay, Promega). Briefly, the MTS solution was diluted 20× in DMEM without phenol red (PAN-Biotech GmbH) supplemented with 10% FBS. The cells were washed once with 200 µL/PBS well, after which 100 µL diluted MTS solution was added/well. The plate was incubated for approximately 20-30 minutes at 37°C. Subsequently, the OD at 492 nm was measured on a Thermo Scientific Multiskan FC plate reader (Thermo Scientific). For quantification the background signal of 'medium only' wells was subtracted from all other wells, before the cell viability percentage of treated/untreated cells was calculated, by dividing the background corrected signal of treated wells over the background corrected signal of the untreated wells (x 100).

### FACS analysis

Cells were seeded in DMEM (PAN-Biotech GmbH) supplemented with 10% fetal bovine serum (PAN-Biotech GmbH) and 1% penicillin/streptomycin (PAN-Biotech GmbH), in T75 flasks at appropriate density for each cell-line and incubated for 72-96 hrs (5% CO₂, 37°C), until a confluency of 90% was reached. Next, the cells were trypsinized (TryplE Express, Gibco Thermo Scientific) to single cells, transferred to a 15 mL falcon tube, and centrifuged (1,400 rpm, 3 min). The supernatant was discarded while leaving the cell pellet submerged. 500.000 Cells were transferred to round bottom FACS tubes and the washed with 3 mL cold DPBS (Mg²⁺ and Ca²⁺ free, 2% FBS). The cells were centrifuged at 1800 rpm, 3 min 4°C and resuspended in 200 µL cold DPBS (Mg²⁺ and Ca²⁺ free, 2% FBS) or 200 µL antibody solution; containing 5 µL antibody in 195 µL cold DPBS (Mg²⁺ and Ca²⁺ free, 2% FBS). PE anti-human CD71 (#334106, Biolegend ) was used to stain the transferrin receptor, PE Mouse IgG2a, κ Isotype Ctrl FC (#400212, Biolegend) was used as its matched isotype control. PE anti-human ASGPR1 (#130-122-963, Miltenyi) was used to stain the ASGPR1 receptor and PE Mouse IgG1, Isotype Ctrl (#130-113-762, Miltenyi) was used as its matched isotype control. Samples were incubated for 30 min. at 4°C. Afterwards, the cells were washed 2x with cold DPBS (Mg²⁺ and Ca²⁺ free, 2% FBS) and fixated for 20 min at room temperature using a 2% PFA solution in DPBS (Mg²⁺ and Ca²⁺ free, 2% FBS). Cells were washed 1x with cold DPBS, and resuspended in 1000 µL cold DPBS for FACS analysis. Samples were analyzed with a Sysmex Cube 8 flow cytometry system (Sysmex) and FCS Express 7 Research edition software. Results of the FACS analyses are summarized in Table A3.

**Table A3. Cell membrane receptor expression levels of ASPGR1 and CD71 of HepG2 and Huh7 cells**

| **Cell line** | **ASPGR1 expression level (MFI)** | **CD71 expression level (MFI)** |
|---|---|---|
| **HepG2** | 8.8 | 74.7 |
| **Huh7** | 1.6 | 109 |

### RNA isolation and gene expression analysis from primary mouse and human hepatocytes

RNA from cells was isolated using TRI Reagent^{®} Solution (Thermo Scientific) according to the manufacturer's instruction. Conversion into cDNA was performed using iScript^{™} cDNA Synthesis Kit (BioRad) using standard protocols. *ApoB* expression levels and levels of specific hepatocyte housekeeping genes were determined using quantitative real-time PCR assays (qRT-PCR) using iTag^{™} Universal SYBR^{®} Green Supermix (BioRad) and the Light Cycler 480 (Roche Diagnostics, Rotkreuz, Switzerland) with specific DNA primers, listed in Table A4 (murine) and Table A2 (human). Analysis was done by the ΔCt method to determine ApoB expression relative to 2 hepatocyte-specific housekeeping control mRNAs. Each analysis reaction was performed in triplicate.

**Table A4. Primers used in qPCR analysis of mouse primary hepatocytes**

| | | |
|---|---|---|
| ApoB#02 | Forward | GCTAACACTAAGAACCAGAAGATC [SEQ ID NO: 13] |
| | Reverse | TGTCCGTCTAAGGATCCTGC [SEQ ID NO: 14] |
| Mm PPIA | Forward | GCGGCAGGTCCATCTACG [SEQ ID NO: 15] |
| | Reverse | GCCATCCAGCCATTCAGTC [SEQ ID NO: 16] |
| Mm SDHA | Forward | GAGGAAGCACACCCTCTCAT [SEQ ID NO: 17] |
| | Reverse | GGAGCGGATAGCAGGAGGTA [SEQ ID NO: 18] |

### Mouse primary hepatocyte treatment

Cryopreserved primary mouse hepatocytes (PRIMACYT Cell Culture Technology GmbH, Germany) were thawed in hepatocyte thawing media (HTM, PRIMACYT Cell Culture Technology GmbH, Germany) and washed 1x with hepatocyte wash media (HWM, PRIMACYT Cell Culture Technology GmbH, Germany). Cells were re-suspended in hepatocyte plating media (HPM Cryo, PRIMACYT Cell Culture Technology GmbH, Germany) at a density of approx. 0.275 x 10⁶ cells/ml. Cells were seeded onto collagen-I coated plates at a density of 88.000 cells/well or 26.400 cells/well for the 48 or 96-well plates (Greiner BioOne). Cells were pre-cultured for 4-6 hrs at 37°C allowing for cell attachment to cell culture plates before the start of treatment. Plating medium was replaced by 315 µl or 108 µl assay media (MHM, PRIMACYT Cell Culture Technology GmbH, Germany), after which conjugates were added from a 10x concentrated stock solution in PBS. Plates were incubated for 72 hr at 37°C being harvested for gene expression and cell viability analysis.

### Human primary hepatocyte treatment

Cryopreserved primary human hepatocytes (Cytes Biotechnologies S.L., Spain) were thawed in hepatocyte thawing media (Cytes Biotechnologies S.L., Spain). Cells were re-suspended in hepatocyte plating media (Cytes Biotechnologies S.L., Spain). Cells were seeded onto collagen-I coated plates at a density of 215.600 cells/well or 66.600 cells/well for the 48 or 96-well plates (Greiner BioOne). Cells were pre-cultured for 4-6 hrs at 37°C allowing for cell attachment to cell culture plates before the start of treatment. Plating medium was replaced by 315 µl or 108 µl maintenance media (Cytes Biotechnologies S.L., Spain), after which conjugates were added from a 10x concentrated stock solution in PBS. Plates were incubated for 72 hr at 37°C being harvested for gene expression and cell viability analysis.

## Claims

1. Pharmaceutical combination comprising:
- a conjugate of an effector molecule and a ligand for asialoglycoprotein receptor (ASGPR), wherein the ligand for ASGPR comprises at least one N-acetylgalactosamine (GalNAc) moiety, preferably three or four GalNAc moieties, more preferably the ligand for ASGPR comprises or consists of (GalNAc)sTris and wherein the effector molecule comprises or consists of an oligonucleotide; and
- a saponin, wherein the saponin is a monodesmosidic triterpene glycoside or a bidesmosidic triterpene glycoside comprising an aglycone core structure selected from quillaic acid and gypsogenin, wherein, optionally, the aldehyde group at position C23 of the quillaic acid or gypsogenin has been derivatised, and optionally comprising a pharmaceutically acceptable excipient and/or pharmaceutically acceptable diluent.

2. Pharmaceutical combination of claim 1 in the form of at least two pharmaceutical compositions comprising:
- a first pharmaceutical composition comprising the conjugate and optionally comprising a pharmaceutically acceptable excipient and/or pharmaceutically acceptable diluent; and
- a second pharmaceutical composition comprising the saponin and optionally comprising a pharmaceutically acceptable excipient and/or pharmaceutically acceptable diluent.

3. Pharmaceutical combination of claim 1 in the form of a single pharmaceutical composition comprising the conjugate, the saponin and optionally a pharmaceutically acceptable excipient and/or pharmaceutically acceptable diluent.

4. Pharmaceutical combination of any one of the previous claims, wherein the ligand for ASGPR and the effector molecule are conjugated via a covalent bond, preferably via at least one linker.

5. Pharmaceutical combination of any one of the previous claims, wherein the effector molecule:
- is an oligonucleotide selected from any one or more of a(n): short interfering RNA (siRNA), short hairpin RNA (shRNA), anti-hairpin-shaped microRNA (miRNA), single-stranded RNA, aptamer RNA, doublestranded RNA (dsRNA), anti-microRNA (anti-miRNA, anti-miR), antisense oligonucleotide (ASO), mRNA, DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON);
- is an oligonucleotide selected from any one of an anti-miRNA, a BNA-AON or an siRNA, such as BNA-based siRNA, preferably selected from chemically modified siRNA, metabolically stable siRNA and chemically modified, metabolically stable siRNA;
- is an oligonucleotide that is capable of silencing any one of genes: HSP27, apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is capable of targeting an aberrant miRNA; and/or
- is an oligonucleotide that, for example when present inside a mammalian cell, is capable of targeting an mRNA involved in expression of any one of proteins: HSP27, apoB, TTR, PCSK9, ALAS1, AT3, GO, CC5, expression product of X gene of HBV, expression product of S gene of HBV, AAT and LDH, or is capable of antagonizing or restoring an miRNA function such as inhibiting an oncogenic miRNA (onco-miR) or suppression of expression of an onco-miR, for example when present inside a mammalian cell.

6. Pharmaceutical combination of any one of the previous claims, wherein the saponin aglycone core structure is quillaic acid or a derivative thereof.

7. Pharmaceutical combination of any one of the previous claims, wherein
• the saponin comprises a saccharide chain bound to the aglycone core structure, which is selected from group A:
GlcA-,
Glc-,
Gal-,
Rha-(1→2)-Ara-,
Gal-(1→2)-[Xyl-(1→3)]-GlcA-,
Glc-(1→2)-[Glc-(1→)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
derivatives thereof,
or
• the saponin comprises a saccharide chain bound to the aglycone core structure, which is selected from group B:
Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc- wherein R1 is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[GIc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[GIc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
Api/Xyl-(1→3)-Xyl-(1→4)-[GIc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
Glc-(1→3)-[Glc-(1→6)]-Gal-, and
derivatives thereof,
or
• the saponin is a bidesmosidic triterpene glycoside comprising a first saccharide chain selected from the group A bound to the aglycone core structure and comprising a second saccharide chain selected from the group B bound to the aglycone core structure.

8. Pharmaceutical combination of any one of the previous claims, wherein the saponin:
- is selected from the group consisting of: *Quillaja* bark saponin, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, stereoisomers thereof, derivatives thereof, and combinations thereof, preferably the saponin is selected from the group consisting of QS-21, a QS-21 derivative, SO1861, a SO1861 derivative, SA1641, a SA1641 derivative, GE1741,a GE1741 derivative and combinations thereof, more preferably the saponin is selected from the group consisting of a QS-21 derivative, a SO1861 derivative and combinations thereof, most preferably the saponin is a SO1861 derivative; or
- is selected from the group consisting of: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1904, stereoisomers thereof, derivatives thereof and combinations thereof, preferably the saponin is selected from the group consisting of QS-21, a QS-21 derivative, SO1861, a SO1861 derivative, SA1641, a SA1641 derivative, GE1741, a GE1741 derivative and combinations thereof, more preferably the saponin is selected from the group consisting of a QS-21 derivative, a SO1861 derivative and combinations thereof, most preferably the saponin is a SO1861 derivative.

9. Pharmaceutical combination of any one of the previous claims, wherein the saponin is a saponin derivative wherein either:
i. the saponin derivative comprises an aglycone core structure comprising an aldehyde group which has been derivatised; or
ii. the saponin derivative comprises a saccharide chain, preferably a saccharide chain selected from group A as defined in claim 7, the saccharide chain comprising a carboxyl group which has been derivatised; or
iii. the saponin derivative comprises a saccharide chain, preferably a saccharide chain selected from group B as defined in claim 7, the saccharide chain comprising an acetoxy (Me(CO)O-) group which has been derivatised; or
iv. any combination of derivatisations i., ii. and iii. is present, optionally any combination of two derivatisations of derivatisations i., ii. and iii.

10. Pharmaceutical combination of any one of the previous claims, wherein the saponin represented by Molecule 1: 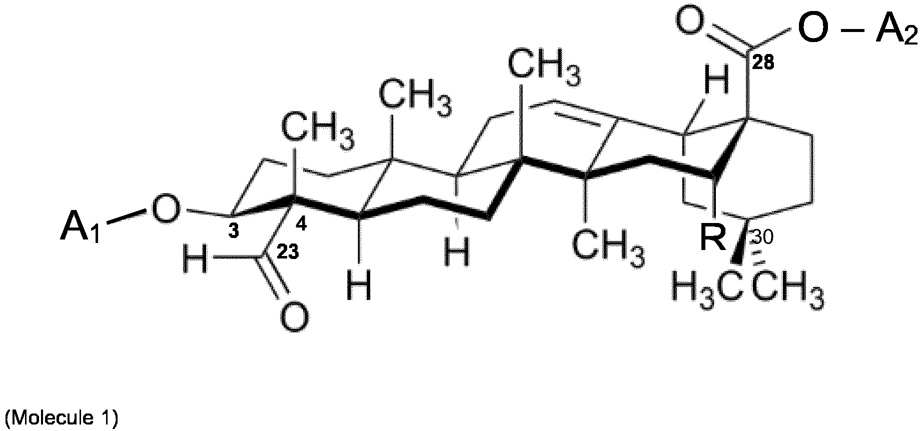 wherein
A₁ represents hydrogen, a monosaccharide or a linear or branched oligosaccharide, preferably
A₁ represents a saccharide chain selected from group A as defined in claim 10, more preferably
A₁ represents a saccharide chain selected from group A as defined in claim 7 and A₁ comprises or consists of a glucuronic acid moiety;
A₂ represents hydrogen, a monosaccharide or a linear or branched oligosaccharide, preferably
A₂ represents a saccharide chain selected from group B as defined in claim 7, more preferably
A₂ represents a saccharide chain selected from group B as defined in claim 7 and A₂ comprises at least one acetoxy (Me(CO)O-) group, such as one, two, three or four acetoxy groups, wherein at least one of A₁ and A₂ is not hydrogen, preferably both A₁ and A₂ are an oligosaccharide chain;
and R is hydrogen in gypsogenin or hydroxyl in quillaic acid;
wherein the saponin derivative corresponds to the saponin represented by Molecule 1 wherein at least one of the following derivatisations is present:
i. the aldehyde group at position C₂₃ of the quillaic acid or gypsogenin has been derivatised;
ii. the carboxyl group of a glucuronic acid moiety of A₁, when A₁ represents a saccharide chain selected from group A as defined in claim 7 and A₁ comprises or consists of a glucuronic acid moiety, has been derivatised; and
iii. one or more, preferably all, of acetoxy group(s) of one saccharide moiety or of two or more saccharide moieties of A₂, when A₂ represents a saccharide chain selected from group B as defined in claim 7 and A₂ comprises at least one acetoxy group, has/have been derivatised;
preferably wherein A₁ represents a saccharide chain selected from group A as defined in claim 7 and comprises or consists of a glucuronic acid moiety and wherein the carboxyl group of a glucuronic acid moiety of A₁ has been derivatised and/or wherein A₂ represents a saccharide chain selected from group B as defined in claim 7 and A₂ comprises at least one acetoxy group and wherein at least one acetoxy group of A₂ has been derivatised.

11. Pharmaceutical combination of claims 10, wherein the saponin derivative corresponds to the saponin represented by Molecule 1 wherein at least one of the following derivatisations is present:
i. the aldehyde group at position C₂₃ of the quillaic acid or gypsogenin has been derivatised by;
- reduction to an alcohol; or
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
- transformation into a hydrazone bond through reaction with N-[ß-maleimidopropionic acid] hydrazide (BMPH) wherein the maleimide group of the BMPH is optionally derivatised by formation of a thioether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. the carboxyl group of a glucuronic acid moiety of A₁, when A₁ represents a saccharide chain selected from group A as defined in claim 7 and A₁ comprises or consists of a glucuronic acid moiety, has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; and
iii. one or more, preferably all, of acetoxy group(s) of one saccharide moiety or of two or more saccharide moieties of A₂, when A₂ represents a saccharide chain selected from group B as defined in claim 7 and A₂ comprises at least one acetoxy group, has/have been derivatised by transformation into a hydroxyl group (HO-) by deacetylation.

12. Pharmaceutical combination of claim 10 or 11, wherein A₁ is Gal-(1→2)-[Xyl-(1→3)]-GlcA and/or A₂ is GIc-(1→3)-Xyl-(1→4)-Rha-(1→2)-[Xyl-(1→3)-4-OAc-Qui-(1→4)]-Fuc, preferably the saponin represented by Molecule 1 is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4OAc-beta-D-quinovopyranosyl-(1→4)]-beta-D-fucopyranoside, more preferably SO1861, GE1741, SA1641 and/or QS-21, or a derivative thereof, most preferably SO1861 or a derivative thereof.

13. Pharmaceutical combination of any one of the claims 9-12, wherein the saponin is a saponin derivative wherein either:
i. the saponin derivative comprises an aglycone core structure comprising an aldehyde group which has been derivatised by:
- reduction to an alcohol; or
- transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH, wherein the maleimide group of the EMCH is optionally derivatised by formation of a thioether bond with mercaptoethanol; or
- transformation into a hydrazon bond through reaction with N-[ß-maleimidopropionic acid] hydrazide (BMPH) wherein the maleimide group of the BMPH is optionally derivatised by formation of a thioether bond with mercaptoethanol; or
- transformation into a hydrazon bond through reaction with N-[κ-maleimidoundecanoic acid] hydrazide (KMUH) wherein the maleimide group of the KMUH is optionally derivatised by formation of a thioether bond with mercaptoethanol; or
ii. the saponin derivative comprises a saccharide chain, preferably a saccharide chain selected from group A as defined in claim 7, the saccharide chain comprising a carboxyl group, preferably a carboxyl group of a glucuronic acid moiety which has been derivatised by transformation into an amide bond through reaction with 2-amino-2-methyl-1,3-propanediol (AMPD) or N-(2-aminoethyl)maleimide (AEM), therewith providing a saponin-Glu-AMPD such as a QS-21-Glu-AMPD or a SO1861-Glu-AMPD or a saponin-Glu-AEM such as a QS-21-Glu-AEM or a SO1861-Glu-AEM; or
iii. the saponin derivative comprises a saccharide chain, preferably a saccharide chain selected from group B as defined in claim 7, the saccharide chain comprising an acetoxy (Me(CO)O-) group which has been derivatised by transformation into a hydroxyl group (HO-) by deacetylation; or
iv. the saponin derivative comprises any combination of derivatisations i., ii. and iii., optionally any combination of two derivatisations i., ii. and iii.;
preferably, the saponin derivative comprises an aglycone core structure wherein the aglycone core structure comprises an aldehyde group which has been derivatised by transformation into a hydrazone bond through reaction with EMCH wherein the maleimide group of the EMCH is optionally derivatised by formation of a thioether bond with mercaptoethanol;
preferably wherein the saponin is a saponin derivative wherein either:
i. the saponin derivative comprises an aglycone core structure comprising an aldehyde group which has been derivatised by transformation into a hydrazone bond through reaction with N-ε-maleimidocaproic acid hydrazide (EMCH), therewith providing a saponin-Ald-EMCH such as a SO1861-Ald-EMCH or a QS-21-Ald-EMCH; or
ii. the saponin comprises a saccharide chain, preferably a saccharide chain selected from group A as defined in claim 7, the saccharide chain comprising a carboxyl group, preferably a carboxyl group of a glucuronic acid moiety which has been derivatised by transformation into an amide bond through reaction with N-(2-aminoethyl)maleimide (AEM), therewith providing a saponin-Glu-AEM such as a QS-21-Glu-AEM or a SO1861-Glu-AEM; or
iii. the saponin derivative comprises a combination of derivatisations i. and ii.

14. Pharmaceutical combination of any one of the previous claims, wherein the saponin is a saponin derivative represented by Molecule 2: 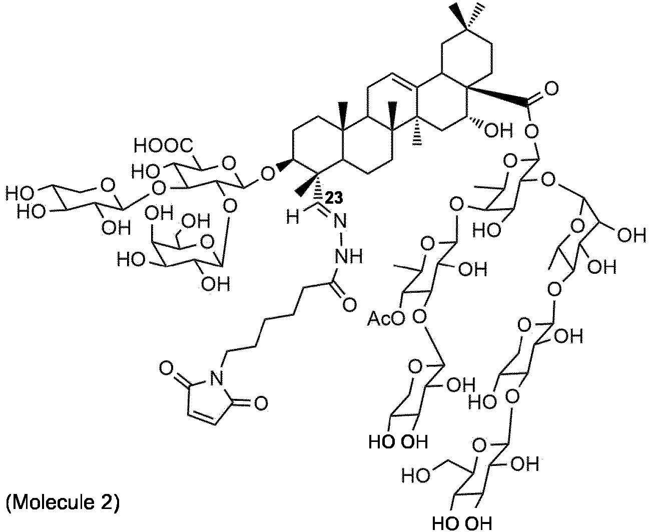 or wherein the saponin derivative is the saponin derivative represented by Molecule 3

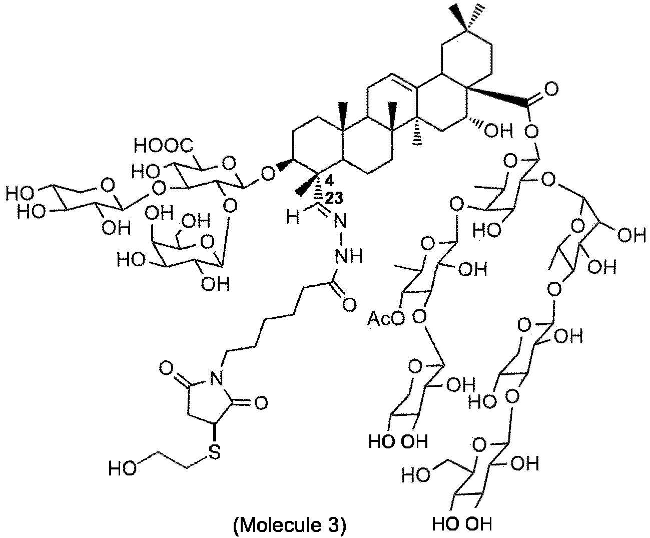

15. Pharmaceutical combination of any one of the previous claims for use:
- as a medicament;
- in the treatment or prophylaxis of a disease or health problem in which an expression product is involved of any one or more of genes: apoB, HSP27, TTR, PCSK9, ALAS1, AT3, GO, CC5, X gene of HBV, S gene of HBV, AAT and LDH; and/or
- in the treatment or prophylaxis of a cancer, an infectious disease, a viral infection, hypercholesterolemia, primary hyperoxaluria, haemophilia A, haemophilia B, AAT related liver disease, acute hepatic porphyria, TTR-mediated amyloidosis, hereditary TTR amyloidosis (hATTR), complement-mediated disease, hepatitis B infection, a disease or disorder relating to HSP27 expression, or an auto-immune disease.

16. *In vitro* or *ex vivo* method for transferring the conjugate as defined in any one of the claims 1-14 from outside a cell to inside said cell, preferably into the cytosol of said cell, comprising the steps of:
a) providing a cell which expresses ASGPR, preferably ASGPR1, on its surface, preferably selected from a liver cell, a virally infected cell and a cancer cell;
b) providing the conjugate as defined in any one of claims 1-14;
c) providing the saponin as defined in any one of claims 1-14;
d) contacting the cell of step a) *in vitro* or *ex vivo* with the conjugate of step b) and the saponin of step c), therewith effecting the transfer of the conjugate from outside the cell inside said cell.

## Patentansprüche

1. Pharmazeutische Kombination, umfassend:
- ein Konjugat aus einem Effektormolekül und einem Liganden für den Asialoglykoprotein-Rezeptor (ASGPR), wobei der Ligand für ASGPR mindestens eine N-Acetylgalactosamin (GalNAc)-Einheit, vorzugsweise drei oder vier GalNAc-Einheiten, umfasst, bevorzugter umfasst oder besteht der Ligand für ASGPR aus (GalNAc)₃Tris, und wobei das Effektormolekül ein Oligonukleotid umfasst oder aus diesem besteht; und
- ein Saponin, wobei das Saponin ein monodesmosidisches Triterpenglykosid oder ein bidesmosidisches Triterpenglykosid ist, das eine Aglykonkernstruktur umfasst, die aus Quillajasäure und Gypsogenin ausgewählt ist, wobei optional die Aldehydgruppe an Position C23 der Quillajasäure oder des Gypsogenins derivatisiert wurde, und optional einen pharmazeutisch annehmbaren Hilfsstoff und/oder ein pharmazeutisch annehmbares Verdünnungsmittel umfasst.

2. Pharmazeutische Kombination nach Anspruch 1 in Form von mindestens zwei pharmazeutischen Zusammensetzungen, umfassend:
- eine erste pharmazeutische Zusammensetzung, die das Konjugat umfasst und optional einen pharmazeutisch annehmbaren Hilfsstoff und/oder ein pharmazeutisch annehmbares Verdünnungsmittel umfasst; und
- eine zweite pharmazeutische Zusammensetzung, die das Saponin umfasst und optional einen pharmazeutisch annehmbaren Hilfsstoff und/oder ein pharmazeutisch annehmbares Verdünnungsmittel umfasst.

3. Pharmazeutische Kombination nach Anspruch 1 in Form einer einzigen pharmazeutischen Zusammensetzung, die das Konjugat, das Saponin und optional einen pharmazeutisch annehmbaren Hilfsstoff und/oder ein pharmazeutisch annehmbares Verdünnungsmittel umfasst.

4. Pharmazeutische Kombination nach einem der vorherigen Ansprüche, wobei der Ligand für ASGPR und das Effektormolekül über eine kovalente Bindung, vorzugsweise über mindestens einen Linker, konjugiert sind.

5. Pharmazeutische Kombination nach einem der vorherigen Ansprüche, wobei das Effektormolekül:
- ein Oligonukleotid ist, das ausgewählt ist aus einem oder mehreren von einer/einem: short interfering RNA (siRNA), short hairpin RNA (shRNA), anti-hairpin-shaped microRNA (miRNA), einzelsträngigen RNA, Aptamer-RNA, doppelsträngigen RNA (dsRNA), anti-microRNA (anti-miRNA, anti-miR), Antisense-Oligonukleotid (ASO), mRNA, DNA, Antisense-DNA, geschlossenen Nukleinsäure (LNA), Bridged Nucleic Acid (BNA), 2'-O,4'-Aminoethylen Bridged Nucleic Acid (BNA^{NC}), BNA-basierter siRNA und BNA-basiertem Antisense-Oligonukleotid (BNA-AON);
- ein Oligonukleotid ist, ausgewählt aus einer anti-miRNA, einem BNA-AON oder einer siRNA, wie z.B. BNA-basierter siRNA, vorzugsweise ausgewählt aus chemisch modifizierter siRNA, metabolisch stabiler siRNA und chemisch modifizierter, metabolisch stabiler siRNA;
- ein Oligonukleotid ist, das in der Lage ist, eines der folgenden Gene stillzulegen: HSP27, Apolipoprotein B (apoB), Transthyretin (TTR), Proprotein Convertase Subtilisin/Kexin Typ 9 (PCSK9), Delta-Aminolävulinat-Synthase 1 (ALAS1), Antithrombin 3 (AT3), Glykolatoxidase (GO), Komplementkomponente C5 (CC5), X-Gen des Hepatitis-B-Virus (HBV), S-Gen des HBV, Alpha-1-Antitrypsin (AAT) und Laktatdehydrogenase (LDH), und/oder in der Lage ist, eine aberrante miRNA zu targetieren; und/oder
- ein Oligonukleotid ist, das zum Beispiel, wenn es in einer Säugetierzelle vorhanden ist, in der Lage ist, eine mRNA zu targetieren, die an der Expression eines der folgenden Proteine beteiligt ist: HSP27, apoB, TTR, PCSK9, ALAS1, AT3, GO, CC5, Expressionsprodukt des X-Gens von HBV, Expressionsprodukt des S-Gens von HBV, AAT und LDH, oder in der Lage ist, einer miRNA-Funktion entgegenzuwirken oder sie wiederherzustellen, wie die Hemmung einer onkogenen miRNA (onco-miR) oder die Unterdrückung der Expression einer onco-miR, wenn sie zum Beispiel in einer Säugetierzelle vorhanden ist.

6. Pharmazeutische Kombination nach einem der vorherigen Ansprüche, wobei die Saponin-Aglykonkernstruktur Quillajasäure oder ein Derivat davon ist.

7. Pharmazeutische Kombination nach einem der vorherigen Ansprüche, wobei
• das Saponin eine an die Aglykonkernstruktur gebundene Saccharidkette umfasst, die aus der Gruppe A ausgewählt ist:
GlcA-,
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1 →2)-GlcA-,
Ara-(1→4)-Rha-(1 →2)-Gal-(1→2)-Rha-(1 →2)-GlcA-,
Ara-(1→4)-Fuc-(1→2)-Gal-(1→2)-Rha-(1 →2)-GlcA-,
Ara-(1→4)-Rha-(1 →2)-Glc-(1→2)-Fuc-(1 →2)-GlcA-,
Ara-(1→4)-Fuc-(1→2)-Glc-(1→2)-Fuc-(1 →2)-GlcA-,
Ara-(1→4)-Rha-(1 →2)-Gal-(1→2)-Fuc-(1 →2)-GlcA-,
Ara-(1→4)-Fuc-(1→2)-Gal-(1→2)-Fuc-(1 →2)-GlcA-,
Xyl-(1→4)-Rha-(1 →2)-Glc-(1→2)-Rha-(1 →2)-GlcA-,
Xyl-(1→4)-Fuc-(1→2)-Glc-(1→2)-Rha-(1 →2)-GlcA-,
Xyl-(1→4)-Rha-(1 →2)-Gal-(1→2)-Rha-(1 →2)-GlcA-,
Xyl-(1 →4)-Fuc-(1→2)-Gal-(1 →2)-Rha-(1 →2)-GlcA-,
Xyl-(1 →4)-Rha-(1 →2)-Glc-(1→2)-Fuc-(1 →2)-GlcA-,
Xyl-(1 →4)-Fuc-(1→2)-Glc-(1→2)-Fuc-(1 →2)-GlcA-,
Xyl-(1 →4)-Rha-(1 →2)-Gal-(1→2)-Fuc-(1 →2)-GlcA-,
Xyl-(1 →4)-Fuc-(1 →2)-Gal-(1 →2)-Fuc-(1 →2)-GlcA-,und Derivate davon,
oder
• das Saponin eine an die Aglykonkernstruktur gebundene Saccharidkette umfasst, die aus der Gruppe B ausgewählt ist:
Glc-,
Gal-,
Rha-(1 →2)-[Xyl-(1→4)]-Rha-,
Rha-(1 →2)-[Ara-(1 →3)-Xyl-(→4)]-Rha-,
Ara-,
Xyl-,
Xyl-(1 →4)-Rha-(1 →2)-[R1-(→4)]-Fuc-, wobei R1 4E-Methoxyzimtsäure ist,
Xyl-(1 →4)-Rha-(1 →2)-[R2-(→4)]-Fuc-, wobei R2 4Z-Methoxyzimtsäure ist,
Xyl-(1 →4)-[Gal-(1 →3)]-Rha-(1 →2)-4-OAc-Fuc-,
Xyl-(1 →4)-[Glc-(1 -73)]-Rha-(1 →2)-3,4-di-OAc-Fuc-,
Xyl-(1→4)-[Glc-(1 →3)]-Rha-(1 →2)-[R3-(→4)]-3-OAc-Fuc-, wobei R3 4E-Methoxyzimtsäure ist,
Glc-(1 →3)-Xyl-(1 →4)-[Glc-(1 →3)]-Rha-(1 →2)-4-OAc-Fuc-,
Glc-(1 →3)-Xyl-(1 →4)-Rha-(1 →2)-4-OAc-Fuc-,
(Ara- oder Xyl-)(1 →3)-(Ara- oder Xyl-)(1→4)-(Rha- oder Fuc-)(1→2)-[4-OAc-(Rha- oder Fuc-)(1→4)]-(Rha- oder Fuc-),
Xyl-(1 →3)-Xyl-(1 →4)-Rha-(1 →2)-[Qui-(1 →4)]-Fuc-,
Api-(1 →3)-Xyl-(1 →4)-[Glc-(1 →3)]-Rha-(1 →2)-Fuc-,
Xyl-(1 →4)-[Gal-(1 →3)]-Rha-(1 →2)-Fuc-,
Xyl-(1 →4)-[Glc-(1 →3)]-Rha-(1 →2)-Fuc-,
Ara/Xyl-(1 →4)-Rha/Fuc-(1 →4)-[Glc/Gal-(1 →2)]-Fuc-,
Api-(1 →3)-Xyl-(1 →4)-[Glc-(1 →3)]-Rha-(1 →2)-[R4-(→4)]-Fuc-, wobei R4 5-O-[5-O-Ara/Api-3,5-Dihydroxy-6-methyl-octanoyl]-3,5-dihydroxy-6-methyl-octansäure ist,
Api-(1→3)-Xyl-(1→4)-Rha-(1→2)-[R5-(→4)]-Fuc-, wobei R5 5-O-[5-O-Ara/Api-3,5-dihydroxy-6-methyl-octanoyl]-3,5-dihydroxy-6-methyl-octansäure ist,
Api-(1 →3)-Xyl-(1 →4)-Rha-(1 →2)-[Rha-(1 →3)]-4-OAc-Fuc-,
Api-(1→3)-Xyl-(1 →4)-[Glc-(1 →3)]-Rha-(1 →2)-[Rha-(1 →3)]-4-OAc-Fuc-,
6-OAc-Glc-(1 →3)-Xyl-(1 →4)-Rha-(1 →2)-[3-OAc-Rha-(1 →3)]-Fuc-,
Glc-(1 →3)-Xyl-(1 →4)-Rha-(1 →2)-[3-OAc-Rha-(1 →3)]-Fuc-,
Xyl-(1 →3)-Xyl-(1 →4)-Rha-(1 →2)-[Qui-(1 →4)]-Fuc-,
Glc-(1 →3)-[Xyl-(1 →4)]-Rha-(1 →2)-[Qui-(1 →4)]-Fuc-,
Glc-(1 →3)-Xyl-(1 →4)-Rha-(1 →2)-[Xyl-(1 →3)-4-OAc-Qui-(1 →4)]-Fuc-,
Xyl-(1 →3)-Xyl-(1 →4)-Rha-(1 →2)-[3,4-di-OAc-Qui-(1 →4)]-Fuc-,
Glc-(1 →3)-[Xyl-(1 →4)]-Rha-(1 →2)-Fuc-,
6-OAc-Glc-(1 →3)-[Xyl-(1 →4)]-Rha-(1 2)-Fuc-,
Glc-(1 →3)-[Xyl-(1 →3)-Xyl-(1 →4)]-Rha-(1 →2)-Fuc-,
Xyl-(1 →3)-Xyl-(1 →4)-Rha-(1 →2)-[Xyl-(1 →3)-4-OAc-Qui-(1 →4)]-Fuc-,
Api/Xyl-(1 →3)-Xyl-(1 →4)-[Glc-(1 →3)]-Rha-(1 →2)-[Rha-(1 →3)]-4OAc-Fuc-,
Api-(1 →3)-Xyl-(1 →4)-[Glc-(1 →3)]-Rha-(1 →2)-[Rha-(1 →3)]-4OAc-Fuc-,
Api/Xyl-(1 →3)-Xyl-(1 →4)-[Glc-(1 →3)]-Rha-(1 →2)-[R6-(→4)]-Fuc-, wobei R6 5-O-[5-O-Rha-(1 →2)-Ara/Api-3,5-dihydroxy-6-methyl-octanoyl]-3,5-dihydroxy-6-methyl-octansäure ist,
Api/Xyl-(1 →3)-Xyl-(1 →4)-[Glc-(1 →3)]-Rha-(1 →2)-[R7-(→4)]-Fuc-, wobei R7 5-O-[5-O-Ara/Api-3,5-dihydroxy-6-methyl-octanoyl]-3,5-dihydroxy-6-methyl-octansäure ist,
Api/Xyl-(1 →3)-Xyl-(1 →4)-[Glc-(1→3)]-Rha-(1→2)-[R8-(→4)]-Fuc-, wobei R8 5-O-[5-O-Ara/Api-3,5-dihydroxy-6-methyl-octanoyl]-3,5-dihydroxy-6-methyl-octansäure ist,
Api-(1 →3)-Xyl-(1 →4)-Rha-(1 →2)-[R9-( →4)]-Fuc-, wobei R9 5-O-[5-O-Ara/Api-3,5-dihydroxy-6-methyl-octanoyl]-3,5-dihydroxy-6-methyl-octansäure ist,
Xyl-(1 →3)-Xyl-(1 →4)-Rha-(1 →2)-[R10-(→4)]-Fuc-, wobei R10 5-O-[5-O-Ara/Api-3,5-dihydroxy-6-methyl-octanoyl]-3,5-dihydroxy-6-methyl-octansäure ist,
Api-(1 →3)-Xyl-(1 →4)-Rha-(1 →2)-[R11-(→3)]-Fuc-, wobei R11 5-O-[5-O-Ara/Api-3,5-dihydroxy-6-methyl-octanoyl]-3,5-dihydroxy-6-methyl-octansäure ist,
Xyl-(1→3)-Xyl-(1→4)-Rha-(1→2)-[R12-(→3)]-Fuc-, wobei R12 5-O-[5-O-Ara/Api-3,5-dihydroxy-6-methyl-octanoyl]-3,5-dihydroxy-6-methyl-octansäure ist,
Glc-(1 →3)-[Glc-(1 →6)]-Gal-, und
deren Derivate,
oder
• das Saponin ein bidesmosidisches Triterpenglykosid ist, das eine erste Saccharidkette umfasst, die ausgewählt ist aus der Gruppe A, die an die Aglykonkernstruktur gebunden ist, und eine zweite Saccharidkette umfasst, die aus der Gruppe B ausgewählt ist, die an die Aglykonkernstruktur gebunden ist.

8. Pharmazeutische Kombination nach einem der vorherigen Ansprüche, wobei das Saponin:
- ausgewählt ist aus der Gruppe bestehend aus: Quillaja-Rindensaponin , NP-017777, NP-017778, NP-017774, NP-018110, NP-017772, NP-018109, NP-017888, NP-017889, NP-018108, SA1641, AEX55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, Stereoisomeren dieser, Derivaten dieser und Kombinationen dieser, wobei das Saponin vorzugsweise ausgewählt ist aus der Gruppe bestehend aus QS-21, einem QS-21-Derivat, SO1861, einem SO1861-Derivat, SA1641, einem SA1641-Derivat, GE1741, einem GE1741-Derivat und Kombinationen dieser, vorzugsweise ist das Saponin aus der Gruppe ausgewählt, die aus einem QS-21-Derivat, einem SO1861-Derivat und Kombinationen dieser besteht, am meisten bevorzugt ist das Saponin ein SO1861-Derivat; oder
- ausgewählt ist aus der Gruppe bestehend aus: SO1861, SA1657, GE1741, SA1641, QS-21, QS-21 A, QS-21 A-api, QS-21 A-xyl, QS-21 B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, Gypsosid A, AG1, AG2, SO1542, SO1584, SO1658, SO1674, SO1832, SO1904, Stereoisomeren dieser, Derivaten dieser und Kombinationen dieser, vorzugsweise ist das Saponin ausgewählt aus der Gruppe bestehend aus QS-21, einem QS-21-Derivat, SO1861, einem SO1861-Derivat, SA1641, einem SA1641-Derivat, GE1741, einem GE1741-Derivat und Kombinationen dieser, bevorzugter ist das Saponin aus der Gruppe ausgewählt, die aus einem QS-21-Derivat, einem SO1861-Derivat und Kombinationen dieser besteht, am meisten bevorzugt ist das Saponin ein SO1861-Derivat.

9. Pharmazeutische Kombination nach einem der vorherigen Ansprüche, wobei das Saponin ein Saponin-Derivat ist, bei dem entweder:
i. das Saponinderivat eine Aglykonkernstruktur umfasst, die eine Aldehydgruppe umfasst, die derivatisiert wurde; oder
ii. das Saponinderivat eine Saccharidkette umfasst, vorzugsweise eine Saccharidkette ausgewählt aus der Gruppe A, wie in Anspruch 7 definiert, wobei die Saccharidkette eine Carboxylgruppe umfasst, die derivatisiert wurde; oder
iii. das Saponinderivat eine Saccharidkette umfasst, vorzugsweise eine Saccharidkette ausgewählt aus der Gruppe B, wie in Anspruch 7 definiert, wobei die Saccharidkette eine Acetoxy (Me(CO)O-)-Gruppe umfasst, die derivatisiert wurde; oder
iv. eine beliebige Kombination der Derivatisierungen i., ii. und iii. vorliegt, optional eine Kombination von zwei Derivatisierungen der Derivatisierungen i., ii. und iii.

10. Pharmazeutische Kombination nach einem der vorherigen Ansprüche, wobei das durch Molekül 1 dargestellte Saponin: 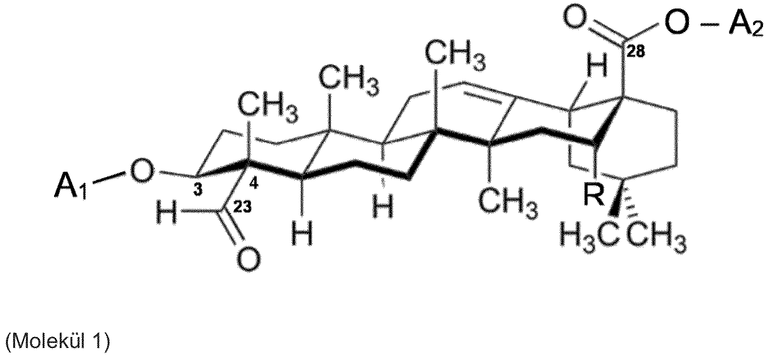 wobei
A₁ für Wasserstoff, ein Monosaccharid oder ein lineares oder verzweigtes Oligosaccharid steht, vorzugsweise steht A₁ für eine Saccharidkette, die aus der in Anspruch 7 definierten Gruppe A ausgewählt ist, noch bevorzugter steht
A₁ für eine Saccharidkette, die aus der in Anspruch 7 definierten Gruppe A ausgewählt ist, und A₁ umfasst oder besteht aus einer Glucuronsäureeinheit;
A₂ steht für Wasserstoff, ein Monosaccharid oder ein lineares oder verzweigtes Oligosaccharid, vorzugsweise steht A₂ für eine Saccharidkette, ausgewählt aus der Gruppe B, wie sie in Anspruch 7 definiert ist, noch bevorzugter steht A₂ für eine Saccharidkette, ausgewählt aus der Gruppe B, wie sie in Anspruch 7 definiert ist, und A₂ umfasst mindestens eine Acetoxy (Me(CO)O-)-Gruppe, wie eine, zwei, drei oder vier Acetoxy-Gruppen, wobei mindestens eines von A₁ und A₂ nicht Wasserstoff ist, vorzugsweise sind sowohl A₁ als auch A₂ eine Oligosaccharidkette;
und R ist Wasserstoff in Gypsogenin oder Hydroxyl in Quillajasäure;
wobei das Saponinderivat dem durch Molekül 1 dargestellten Saponin entspricht, in dem mindestens eine der folgenden Derivatisierungen vorhanden ist:
i. die Aldehydgruppe an der Position C₂₃ der Quillajasäure oder des Gypsogenins wurde derivatisiert;
ii. die Carboxylgruppe einer Glucuronsäureeinheit von A₁, wenn A₁ eine Saccharidkette darstellt, die aus der Gruppe A ausgewählt ist, wie sie in Anspruch 7 definiert ist, und A₁ eine Glucuronsäureeinheit umfasst oder aus dieser besteht, wurde derivatisiert; und
iii. eine oder mehrere, vorzugsweise alle, Acetoxygruppe(n) einer Saccharideinheit oder von zwei oder mehr Saccharideinheiten von A₂, wenn A₂ eine Saccharidkette darstellt, die aus der in Anspruch 7 definierten Gruppe B ausgewählt ist, und A₂ mindestens eine Acetoxygruppe umfasst, wurde(n) derivatisiert;
wobei A₁ vorzugsweise eine Saccharidkette darstellt, die aus der Gruppe A, wie in Anspruch 7 definiert, ausgewählt ist und eine Glucuronsäureeinheit umfasst oder daraus besteht, und wobei die Carboxylgruppe einer Glucuronsäureeinheit von A₁ derivatisiert wurde und/oder wobei A₂ eine Saccharidkette darstellt, die aus der Gruppe B, wie in Anspruch 7 definiert, ausgewählt ist, und A₂ mindestens eine Acetoxygruppe umfasst, und wobei mindestens eine Acetoxygruppe von A₂ derivatisiert wurde.

11. Pharmazeutische Kombination nach Anspruch 10, wobei das Saponinderivat dem durch Molekül 1 dargestellten Saponin entspricht, wobei mindestens eine der folgenden Derivatisierungen vorhanden ist:
i. die Aldehydgruppe an Position C₂₃ der Quillajasäure oder des Gypsogenins wurde derivatisiert durch;
- Reduktion zu einem Alkohol; oder
- Umwandlung in eine Hydrazonbindung durch Reaktion mit N-ε-Maleimidocapronsäurehydrazid (EMCH), wodurch ein Saponin-Ald-EMCH wie ein SO1861-Ald-EMCH oder ein QS-21-Ald-EMCH bereitgestellt wird, wobei die Maleimidgruppe des EMCH optional durch Bildung einer Thioetherbindung mit Mercaptoethanol derivatisiert ist; oder
- Umwandlung in eine Hydrazonbindung durch Reaktion mit N-[β-Maleimidopropion-säure]hydrazid (BMPH), wobei die Maleimidgruppe des BMPH optional durch Bildung einer Thioetherbindung mit Mercaptoethanol derivatisiert ist; oder
- Umwandlung in eine Hydrazonbindung durch Reaktion mit N-[x-Maleimido-undecansäure]hydrazid (KMUH), wobei die Maleimidgruppe des KMUH optional durch Bildung einer Thioetherbindung mit Mercaptoethanol derivatisiert ist;
ii. die Carboxylgruppe einer Glucuronsäuregruppe von A₁, wenn A₁ eine Saccharidkette darstellt, die aus der Gruppe A wie in Anspruch 7 definiert ausgewählt ist, und A₁ eine Glucuronsäureeinheit umfasst oder daraus besteht, wurde durch Umwandlung in eine Amidbindung durch Reaktion mit 2-Amino-2-methyl-1,3-Propandiol (AMPD) oder N-(2-Aminoethyl)-maleimid (AEM) derivatisiert, wodurch ein Saponin-Glu-AMPD wie ein QS-21-Glu-AMPD oder ein SO1861-Glu-AMPD oder ein Saponin-Glu-AEM wie ein QS-21-Glu-AEM oder ein SO1861-Glu-AEM bereitgestellt wird; und
iii. eine oder mehrere, vorzugsweise alle, Acetoxygruppe(n) einer Saccharideinheit oder von zwei oder mehr Saccharideinheiten von A₂, wenn A₂ eine Saccharidkette, ausgewählt aus der Gruppe B, wie in Anspruch 7 definiert, darstellt und A₂ mindestens eine Acetoxygruppe umfasst, wurde(n) durch Umwandlung in eine Hydroxylgruppe (HO-) durch Deacetylierung derivatisiert.

12. Pharmazeutische Kombination nach Anspruch 10 oder 11, wobei A₁ Gal-(1 →2)-[Xyl-(1 →3)]-GlcA ist und/oder A₂ Glc-(1 →3)-Xyl-(1 →4)-Rha-(1 →2)-[Xyl-(1→3)-4-OAc-Qui-(1 →4)]-Fuc ist, vorzugsweise ist das durch Molekül 1 dargestellte Saponin 3-O-beta-D-Galactopyranosyl-(1 →2)-[beta-D-xylopyranosyl-(1→3)]-beta-D-glucuronopyranosyl-Quillajasäure 28-O-beta-D-glucopyranosyl-(1 →3)-beta-D-xylopyranosyl-(1 →4)-alpha-L-rhamnopyranosyl-(1 →2)-[beta-D-xylopyranosyl-(1 →3)-4OAc-beta-D-chinovopyranosyl-(1 →4)]-beta-D-fucopyranosid, bevorzugter SO1861, GE1741, SA1641 und/oder QS-21, oder ein Derivat dieser, am meisten bevorzugt SO1861 oder ein Derivat davon.

13. Pharmazeutische Kombination nach einem der Ansprüche 9-12, wobei das Saponin ein Saponinderivat ist, wobei entweder:
i. das Saponinderivat eine Aglykonkernstruktur umfasst, die eine Aldehydgruppe umfasst, die derivatisiert wurde durch:
- Reduktion zu einem Alkohol; oder
- Umwandlung in eine Hydrazonbindung durch Reaktion mit N-ε-Maleimidocapronsäurehydrazid (EMCH), wodurch ein Saponin-Ald-EMCH wie ein SO1861-Ald-EMCH oder ein QS-21-Ald-EMCH bereitgestellt wird, wobei die Maleimidgruppe des EMCH optional durch Bildung einer Thioetherbindung mit Mercaptoethanol derivatisiert ist; oder
- Umwandlung in eine Hydrazonbindung durch Reaktion mit N-[ß-Maleimidopropion-säure]hydrazid (BMPH), wobei die Maleimidgruppe des BMPH optional durch Bildung einer Thioetherbindung mit Mercaptoethanol derivatisiert ist; oder
- Umwandlung in eine Hydrazonbindung durch Reaktion mit N-[x-Maleimido-undecansäure]hydrazid (KMUH), wobei die Maleimidgruppe des KMUH optional durch Bildung einer Thioetherbindung mit Mercaptoethanol derivatisiert ist; oder
ii. das Saponinderivat eine Saccharidkette umfasst, vorzugsweise eine Saccharidkette, die aus der in Anspruch 7 definierten Gruppe A ausgewählt ist, wobei die Saccharidkette eine Carboxylgruppe umfasst, vorzugsweise eine Carboxylgruppe einer Glucuronsäureeinheit, die derivatisiert wurde durch Umwandlung in eine Amidbindung durch Reaktion mit 2-Amino-2-methyl-1,3-propandiol (AMPD) oder *N*-(2-Aminoethyl)maleimid (AEM), wodurch ein Saponin-Glu-AMPD wie ein QS-21-Glu-AMPD oder ein SO1861-Glu-AMPD oder ein Saponin-Glu-AEM wie ein QS-21-Glu-AEM oder ein SO1861-Glu-AEM bereitgestellt wird; oder
iii. das Saponinderivat eine Saccharidkette umfasst, vorzugsweise eine Saccharidkette, die aus der in Anspruch 7 definierten Gruppe B ausgewählt ist, wobei die Saccharidkette eine Acetoxy- (Me(CO)O-) Gruppe umfasst, die durch Umwandlung in eine Hydroxylgruppe (HO-) durch Deacetylierung derivatisiert wurde; oder
iv. das Saponinderivat eine beliebige Kombination der Derivatisierungen i., ii. und iii. umfasst, optional eine beliebige Kombination aus zwei Derivatisierungen i., ii. und iii;
vorzugsweise umfasst das Saponinderivat eine Aglykonkernstruktur, wobei die Aglykonkernstruktur eine Aldehydgruppe umfasst, die durch Umwandlung in eine Hydrazonbindung durch Reaktion mit EMCH derivatisiert wurde, wobei die Maleimidgruppe des EMCH optional durch Bildung einer Thioetherbindung mit Mercaptoethanol derivatisiert ist;
wobei das Saponin vorzugsweise ein Saponin-Derivat ist, wobei:
i. das Saponinderivat eine Aglykonkernstruktur umfasst, die eine Aldehydgruppe umfasst, die durch Umwandlung in eine Hydrazonbindung durch Reaktion mit N- ε -Maleimidocapronsäurehydrazid (EMCH) derivatisiert wurde, wodurch ein Saponin-Ald-EMCH wie ein SO1861-Ald-EMCH oder ein QS-21-Ald-EMCH bereitgestellt wird; oder
ii. das Saponin eine Saccharidkette umfasst, vorzugsweise eine Saccharidkette, die aus der in Anspruch 7 definierten Gruppe A ausgewählt ist, wobei die Saccharidkette eine Carboxylgruppe umfasst, vorzugsweise eine Carboxylgruppe einer Glucuronsäureeinheit, die durch Umwandlung in eine Amidbindung durch Reaktion mit N-(2-Aminoethyl)maleimid (AEM) derivatisiert wurde, wodurch ein Saponin-Glu-AEM wie ein QS-21-Glu-AEM oder ein SO1861-Glu-AEM bereitgestellt wird; oder
iii. das Saponin-Derivat eine Kombination der Derivatisierungen i. und ii. umfasst.

14. Pharmazeutische Kombination nach einem der vorherigen Ansprüche, wobei das Saponin ein Saponin-Derivat ist, das durch Molekül 2 dargestellt wird: 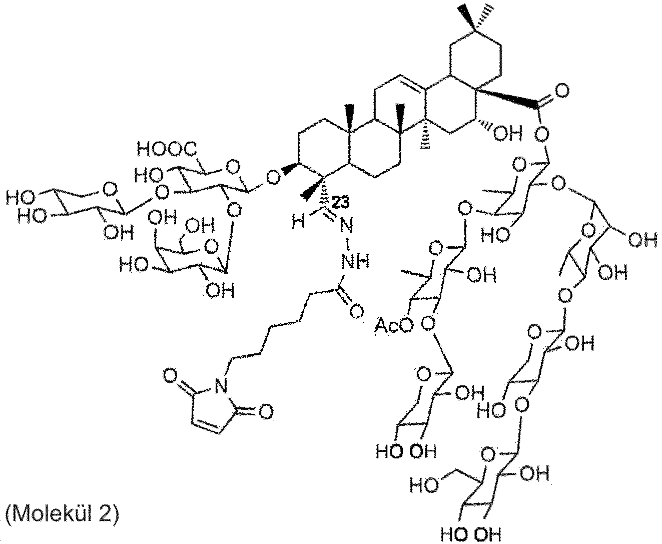 oder wobei das Saponin-Derivat das durch Molekül 3 dargestellte Saponin-Derivat ist

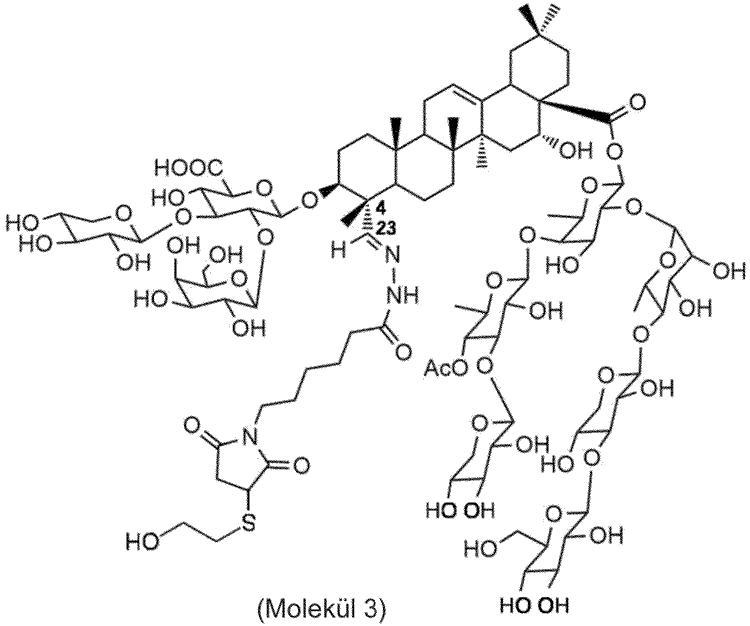

15. Pharmazeutische Kombination nach einem der vorherigen Ansprüche für die folgende Anwendung:
- als Arzneimittel;
- bei der Behandlung oder Prophylaxe einer Krankheit oder eines Gesundheitsproblems, bei dem ein Expressionsprodukt von einem oder mehreren der folgenden Gene involviert ist: apoB, HSP27, TTR, PCSK9, ALAS1, AT3, GO, CC5, X-Gen von HBV, S-Gen von HBV, AAT und LDH; und/oder
- bei der Behandlung oder Prophylaxe von Krebs, einer Infektionskrankheit, einer viralen Infektion, Hypercholesterinämie, primärer Hyperoxalurie, Hämophilie A, Hämophilie B, AAT-bedingter Lebererkrankung, akuter hepatischer Porphyrie, TTR-vermittelter Amyloidose, hereditärer TTR-Amyloidose (hATTR), komplementvermittelter Krankheit, Hepatitis-B-Infektion, einer Krankheit oder Störung im Zusammenhang mit der HSP27-Expression oder einer Autoimmunerkrankung.

16. *In vitro-* oder ex vivo-Verfahren zur Übertragung des Konjugats wie in einem der Ansprüche 1-14 definiert von außerhalb einer Zelle in das Innere der Zelle, vorzugsweise in das Zytosol der Zelle, das die folgenden Schritte umfasst:
a) Bereitstellen einer Zelle, die ASGPR, vorzugsweise ASGPR1, auf ihrer Oberfläche exprimiert, vorzugsweise ausgewählt aus einer Leberzelle, einer vireninfizierten Zelle und einer Krebszelle;
b) Bereitstellen des Konjugats wie in einem der Ansprüche 1-14 definiert;
c) Bereitstellen des Saponins, wie in einem der Ansprüche 1-14 definiert;
d) Kontaktieren der Zelle aus Schritt a) *in vitro* oder *ex vivo* mit dem Konjugat aus Schritt b) und dem Saponin aus Schritt c), wodurch der Transfer des Konjugats von außerhalb der Zelle in das Innere der Zelle bewirkt wird.

## Revendications

1. Combinaison pharmaceutique comprenant :
- un conjugué d'une molécule effectrice et d'un ligand pour le récepteur de l'asialoglycoprotéine (ASGPR), dans laquelle le ligand pour ASGPR comprend au moins une fraction *N*-acétylgalactosamine (GalNAc), de préférence trois ou quatre fractions GalNAc, de manière plus préférée le ligand pour ASGPR comprend ou est constitué de (GalNAc)₃Tris et dans laquelle la molécule effectrice comprend ou est constituée d'un oligonucléotide ; et
- une saponine, dans laquelle la saponine est un glycoside triterpénique monodesmoside ou un glycoside triterpénique bidesmoside comprenant une structure centrale d'aglycone choisie parmi de l'acide quillayique et de la gypsogénine, dans laquelle, facultativement, le groupe aldéhyde en position C23 de l'acide quillayique ou de la gypsogénine a été dérivatisé, et comprenant facultativement un excipient pharmaceutiquement acceptable et/ou un diluant pharmaceutiquement acceptable.

2. Combinaison pharmaceutique selon la revendication 1 sous la forme d'au moins deux compositions pharmaceutiques comprenant :
- une première composition pharmaceutique comprenant le conjugué et comprenant facultativement un excipient pharmaceutiquement acceptable et/ou un diluant pharmaceutiquement acceptable ; et
- une seconde composition pharmaceutique comprenant la saponine et comprenant facultativement un excipient pharmaceutiquement acceptable et/ou un diluant pharmaceutiquement acceptable.

3. Combinaison pharmaceutique selon la revendication 1 sous la forme d'une composition pharmaceutique unique comprenant le conjugué, la saponine et facultativement un excipient pharmaceutiquement acceptable et/ou un diluant pharmaceutiquement acceptable.

4. Combinaison pharmaceutique selon l'une quelconque des revendications précédentes, dans laquelle le ligand pour ASGPR et la molécule effectrice sont conjugués via une liaison covalente, de préférence via au moins un agent de liaison.

5. Combinaison pharmaceutique selon l'une quelconque des revendications précédentes, dans laquelle la molécule effectrice :
- est un oligonucléotide choisi parmi un ou plusieurs des éléments suivants : petit ARN interférent (ARNsi), petit ARN en épingle à cheveux (ARNsh), microARN de forme anti-épingle à cheveux (ARNmi), ARN simple brin, ARN aptamère, ARN double brin (ARNds), anti-microARN (anti-ARNmi, anti-miR), oligonucléotide antisens (ASO), ARNm, ADN, ADN antisens, acide nucléique verrouillé (LNA), acide nucléique ponté (BNA), acide nucléique ponté 2'-O,4'-aminoéthylène (BNA^{NC}), ARNsi à base de BNA, et oligonucléotide antisens à base de BNA (BNA-AON) ;
- est un oligonucléotide choisi parmi l'un quelconque d'un anti-ARNmi, d'un BNA-AON ou d'un ARNsi, tel que ARNsi à base de BNA, de préférence choisi parmi ARNsi chimiquement modifié, ARNsi métaboliquement stable et ARN si chimiquement modifié et métaboliquement stable ;
- est un oligonucléotide capable d'inactiver l'un quelconque des gènes suivants : HSP27, apolipoprotéine B (apoB), transthyrétine (TTR), proprotéine convertase subtilisine/kexine de type 9 (PCSK9), delta-aminolévulinate synthase 1 (ALAS 1), antithrombine 3 (AT3), glycolate oxydase (GO), composante C5 du complément (CC5), gène X du virus de l'hépatite B (VHB), gène S de VHB, alpha-1 antitrypsine (AAT) et lactate déshydrogénase (LDH), et/ou est capable de cibler un ARNmi aberrant ; et/ou
- est un oligonucléotide qui, par exemple lorsqu'il est présent à l'intérieur d'une cellule de mammifère, est capable de cibler un ARNm impliqué dans l'expression de l'une quelconque des protéines suivantes : HSP27, apoB, TTR, PCSK9, ALAS1, AT3, GO, CC5, produit d'expression du gène X de VHB, produit d'expression du gène S de VHB, AAT et LDH, ou est capable de s'opposer ou de restaurer une fonction de ARNmi par exemple en inhibant un ARNmi oncogénique (onco-miR) ou d'atténuer l'expression d'un onco-miR, par exemple lorsqu'il est présent à l'intérieur d'une cellule de mammifère.

6. Combinaison pharmaceutique selon l'une quelconque des revendications précédentes, dans laquelle la structure centrale de saponine aglycone est un acide quillayique ou un dérivatisé de celui-ci.

7. Combinaison pharmaceutique selon l'une quelconque des revendications précédentes, dans laquelle
• la saponine comprend une chaîne saccharide liée à la structure centrale d'aglycone, qui est choisie dans le groupe A :
GlcA-,
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, et
des dérivés de ceux-ci,
ou
• la saponine comprend une chaîne saccharide liée à la structure centrale d'aglycone, qui est choisie dans le groupe B :
Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1-4)-Rha-(1-2)-[R1-(-4)]-Fuc- où R1 est l'acide 4E-méthoxycinnamique,
Xyl-(1-4)-Rha-(1-2)-[R2-(-4)]-Fuc- où R2 est l'acide 4Z-méthoxycinnamique,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- où R3 est l'acide 4E-méthoxycinnamique,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- ou Xyl-)(1→3)-(Ara- ou Xyl-)(1→4)-(Rha- ou Fuc-)(1→2)-[4-OAc-(Rha- ou Fuc-)(1→4)]-(Rha- ou Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- où R4 est l'acide 5-O-[5-O-Ara/Api-3,5-hydroxy-6-méthyl-octanoyl]-3,5-dihydroxy-6-méthyl-octanoïque),
Api-(1-3)-Xyl-(1-4)-Rha-(1-2)-[R5-(-4)]-Fuc- où R5 est l'acide 5-O-[5-O-Ara/Api-3,5-hydroxy-6-méthyl-octanoyl]-3,5-dihydroxy-6-méthyl-octanoïque),
Api-(1→3)-Xyl-(1→4)-Rha-(1→2)-[Rha-(1→3)]-4-OAc-Fuc-,
Api-(1→3)-Xyl-(1→4)-[Gle-(1→3)]-Rha-(1→2)-[Rha-(1→3)]-4-OAc-fuc-,
6-OAc-Gle-(1→3)-Xyl-(1→4)-Rha-(1→2)-[3-OAc-Rha-(1→3)]-Fuc-,
Gle-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Gle-(1→3)-[Xyl-(1→4)]-Rha-(1→2)-[Qui-(1→4)]-Fuc-,
Gle-(1-3)-Xyl-(1-4)-Rha-(1-2)-[Xyl-(1-3)-4-OAc-Qui-(1-4)]-Fuc-,
Xyl-(1→3)-Xyl-(1→4)-Rha-(1→2)-[3,4-di-OAc-Qui-(1→4)]-Fuc-,
Gle-(1→3)-[Xyl-(1→4)]-Rha-(1→2)-Fuc-,
6-OAc-Glc-(1->3)-[Xyl-(1-4)]-Rha-(1->2)-Fuc-,
Gle-(1-)3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Gle-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(-4)]-Fuc- où R6 est l'acide 5-O-[5-O-Rha-(1→2)-Ara/Api-3,5-dihydroxy-6-methyl-octanoyl]-3,5-dihydroxy-6-méthyl-octanoïque),
Api/Xyl-(1→3)-Xyl-(1→4)-[Glc-(1→3)]-Rha-(1→2)-[R7-(→4)]-Fuc-où R7 est l'acide 5-O-[5-O-Ara/Api-3,5-dihydroxy-6-méthyl-octanoyl]-3,5-dihydroxy-6-méthyl-octanoïque),
Api/Xyl-(1→3)-Xyl-(1→4)-[Glc-(1→3)]-Rha-(1→2)-[R8-(→4)]-Fuc-où R8 est l'acide 5-O-[5-O-Ara/Api-3,5-dihydroxy-6-méthyl-octanoyl]-3,5-dihydroxy-6-méthyl-octanoïque),
Api-(1-3)-Xyl-(1-4)-Rha-(1-2)-[R9-(-4)]-Fuc- où R9 est l'acide 5-O-[5-O-Ara/Api-3,5-dihydroxy-6-méthyl-octanoyl]-3,5-dihydroxy-6-méthyl-octanoïque),
Xyl-(1→3)-Xyl-(1→4)-Rha-(1→2)-[R10-(→4)]-Fuc- où R10 est l'acide 5-O-[5-O-Ara/Api-3,5-dihydroxy-6-méthyl-octanoyl]-3,5-dihydroxy-6-méthyl-octanoïque),
Api-(1→3)-Xyl-(1→4)-Rha-(1→2)-[R11-(→3)]-Fuc- où R11 est l'acide 5-O-[5-O-Ara/Api-3,5-dihydroxy-6-méthyl-octanoyl]-3,5-dihydroxy-6-méthyl-octanoïque),
Xyl-(1-3)-Xyl-(1-4)-Rha-(1-2)-[R12-(-3)]-Fuc- où R12 est l'acide 5-O-[5-O-Ara/Api-3,5-dihydroxy-6-méthyl-octanoyl]-3,5-dihydroxy-6-méthyl-octanoïque)
Glc-(1-3)-[Glc-(1-6)]-Gal-, et
des dérivés de ceux-ci,
ou
• la saponine est un glycoside triterpénique bidesmoside comprenant une première chaîne saccharide sélectionnée dans le groupe A liée à la structure centrale d'aglycone et comprenant une seconde chaîne saccharide sélectionnée dans le groupe B liée à la structure centrale d'aglycone.

8. Combinaison pharmaceutique selon l'une quelconque des revendications précédentes, dans laquelle la saponine :
- est choisie dans le groupe constitué de : saponine d'écorce de *Quillaja,* NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1862, SO1904, QS-7, QS1861, QS-7 api, QS1862, QS-17, QS-18, QS-21 A-apio, QS-21 A-xylo, QS-21 B-apio, QS-21 B-xylo, des stéréoisomères de ceux-ci, des dérivés de ceux-ci et des combinaisons de ceux-ci, de préférence la saponine est choisie dans le groupe constitué de QS-21, d'un dérivé de QS-21, SO1861, d'un dérivé de SO1861, SA1641, d'un dérivé de SA1641, GE1741, d'un dérivé de GE1741 et de combinaisons de ceux-ci, de manière plus préférée la saponine est choisie dans le groupe constitué d'un dérivé de QS-21, d'un dérivé de SO1861 et de combinaisons de ceux-ci, de manière la plus préférée, la saponine est un dérivé de SO1861 ; ou
- est choisie dans le groupe constitué de : SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, saponine de Quillaja, Saponinum album, QS-18, Quil-A, Gyp1, gypsoside A, AG1, AG2, SO1542, SO1584, SO1658, SO1674, SO1832, SO1904, de stéréoisomères de ceux-ci, de dérivés de ceux-ci et de combinaisons de ceux-ci, de préférence la saponine est choisie dans le groupe constitué de QS-21, d'un dérivé de QS-21, de SO1861, d'un dérivé de SO1861, de SA1641, d'un dérivé de SA1641, de GE1741, d'un dérivé de GE1741 et de combinaisons de ceux-ci, de manière plus préférée la saponine est choisie dans le groupe constitué d'un dérivé de QS-21, d'un dérivé de SO1861 et de combinaisons de ceux-ci, de manière la plus préférée, la saponine est un dérivé de SO1861.

9. Combinaison pharmaceutique selon l'une quelconque des revendications précédentes, dans laquelle la saponine est un dérivé de saponine, dans laquelle :
i. le dérivé de saponine comprend une structure centrale d'aglycone comprenant un groupe aldéhyde qui a été dérivatisé ; ou
ii. le dérivé de saponine comprend une chaîne saccharide, de préférence une chaîne saccharide choisie dans le groupe A tel que défini dans la revendication 7, la chaîne saccharide comprenant un groupe carboxyle qui a été dérivatisé ; ou
iii. le dérivé de saponine comprend une chaîne saccharide, de préférence une chaîne saccharidique choisie dans le groupe B tel que défini dans la revendication 7, la chaîne saccharide comprenant un groupe acétoxy (Me(CO)O-) qui a été dérivatisé ; ou
iv. toute combinaison de dérivatisations i., ii. et iii. est présente, facultativement, toute combinaison de deux dérivatisations de dérivatisations i., ii. et iii.

10. Combinaison pharmaceutique selon l'une quelconque des revendications précédentes, dans laquelle la saponine est représentée par la Molécule 1 : 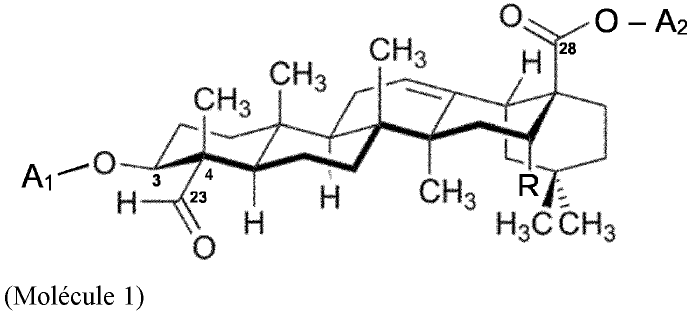 dans laquelle
A₁ représente de l'hydrogène, un monosaccharide ou un oligosaccharide linéaire ou ramifié, de préférence
A₁ représente une chaîne saccharide choisie dans le groupe A tel que défini dans la revendication 7, de manière plus préférée
A₁ représente une chaîne saccharide choisie dans le groupe A tel que défini dans la revendication 7 et A₁ comprend ou est constitué d'une fraction acide glucuronique ;
A₂ représente de l'hydrogène, un monosaccharide ou un oligosaccharide linéaire ou ramifié, de préférence
A₂ représente une chaîne saccharide choisie dans le groupe B tel que défini dans la revendication 7, de manière plus préférée
A₂ représente une chaîne saccharide choisie dans le groupe B tel que défini dans la revendication 7 et A₂ comprend au moins un groupe acétoxy (Me(CO)O-), tel qu'un, deux, trois ou quatre groupes acétoxy, dans laquelle au moins l'un de A₁ et A₂ n'est pas de l'hydrogène, de préférence A₁ et A₂ sont tous deux une chaîne oligosaccharide ;
et R est de l'hydrogène dans la gypsogénine ou l'hydroxyle dans l'acide quillayique ;
dans laquelle le dérivé de saponine correspond à la saponine représentée par la Molécule 1 dans laquelle au moins l'une des dérivatisations suivantes est présente :
i. le groupe aldéhyde en position C₂₃ de l'acide quillayique ou de la gypsogénine a été dérivatisé ;
ii. le groupe carboxyle d'une fraction acide glucuronique de A₁, lorsque A₁ représente une chaîne saccharide choisie dans le groupe A tel que défini dans la revendication 7 et A₁ comprend ou est constitué d'une fraction acide glucuronique, a été dérivatisé ; et
iii. un ou plusieurs, de préférence la totalité, de groupe(s) acétoxy d'une fraction saccharide ou de deux, ou plus, fractions saccharide de A₂, lorsque A₂ représente une chaîne saccharide choisie dans le groupe B tel que défini dans la revendication 7 et A₂ comprend au moins un groupe acétoxy, a/ont été dérivatisé(s) ;
de préférence, dans laquelle A₁ représente une chaîne saccharide choisie dans le groupe A tel que défini dans la revendication 7 et comprend ou est constitué d'une fraction acide glucuronique et dans laquelle le groupe carboxyle d'une fraction acide glucuronique de A₁ a été dérivatisé et/ou dans laquelle A₂ représente une chaîne saccharide choisie dans le groupe B tel que défini dans la revendication 7 et A₂ comprend au moins un groupe acétoxy et dans laquelle au moins un groupe acétoxy de A₂ a été dérivatisé.

11. Combinaison pharmaceutique selon la revendication 10, dans laquelle le dérivé de saponine correspond à la saponine représentée par la Molécule 1, dans laquelle au moins l'une des dérivatisations suivantes est présente :
i. le groupe aldéhyde en position C₂₃ de l'acide quillayique ou de la gypsogénine a été dérivatisé par ;
- réduction en un alcool ; ou
- transformation en une liaison hydrazone par réaction avec l'hydrazide d'acide N-ε-maléimidocaproïque (EMCH), fournissant ainsi une saponine-Ald-EMCH telle qu'une SO1861-Ald-EMCH ou une QS-21-Ald-EMCH, dans laquelle le groupe maléimide de EMCH est facultativement dérivatisé par formation d'une liaison thioéther avec du mercaptoéthanol ; ou
- transformation en une liaison hydrazone par réaction avec l'hydrazide de N-[acide β-maléimidopropionique] (BMPH), dans laquelle le groupe maléimide de BMPH est facultativement dérivatisé par formation d'une liaison thioéther avec du mercaptoéthanol ; ou
- transformation en une liaison hydrazone par réaction avec l'hydrazide de N-[acide κ-maléimidoundécanoïque] (KMUH), dans laquelle le groupe maléimide de KMUH est facultativement dérivatisé par formation d'une liaison thioéther avec du mercaptoéthanol ;
ii. le groupe carboxyle d'une fraction acide glucuronique de A₁, lorsque A₁ représente une chaîne saccharide choisie dans le groupe A tel que défini dans la revendication 7 et A₁ comprend ou est constitué d'une fraction acide glucuronique, a été dérivatisé par transformation en une liaison amide par réaction avec 2-amino-2-méthyl-1,3-propanediol (AMPD) ou *N-(2-*aminoéthyl)maléimide (AEM), fournissant ainsi une saponine-Glu-AMPD telle que QS-21-Glu-AMPD ou SO1861-Glu-AMPD ou saponine-Glu-AEM telle que QS-21-Glu-AEM ou SOI861-Glu-AEM ; et
iii. un ou plusieurs, de préférence la totalité, de groupe(s) acétoxy d'une fraction saccharide ou de deux, ou plus, fractions saccharide de A₂, lorsque A₂ représente une chaîne saccharide choisie dans le groupe B tel que défini dans la revendication 7 et A₂ comprend au moins un groupe acétoxy, a/ont été dérivatisé(s) par transformation en un groupe hydroxyle (HO-) par désacétylation.

12. Combinaison pharmaceutique selon la revendication 10 ou 11, dans laquelle A₁ est Gal-(1→2)-[Xyl-(1→3)]-GlcA et/ou A₂ est Glc-(1→3)-Xyl-(1→4)-Rha-(1→2)-[Xyl-(1→3)-4-OAc-Qui-(1→4)]-Fuc, de préférence la saponine représentée par la Molécule 1 est 3-O-bêta-D-galactopyranosyl-(1→2)-[bêta-D-xylopyranosyl-(1→3)]-bêta-acide D-glucuronopyranosyle quillayique 28-O-bêta-D-glucopyranosyl-(1-3)-bêta-D-xylopyranosyl-(1→4)-alpha-L-rhamnopyranosyl-(1→2)-[bêta-D-xylopyranosyl-(1→3)-4OAc-bêta-D-quinovopyranosyl-(1→4)]-bêta-D-fucopyranoside, de manière plus préférée SO1861, GE1741, SA1641 et/ou QS-21, ou un dérivé de ceux-ci, de manière la plus préférée SO1861 ou un dérivé de celle-ci.

13. Combinaison pharmaceutique selon l'une quelconque des revendications 9 à 12, dans laquelle la saponine est un dérivé de saponine, dans laquelle :
i. le dérivé de saponine comprend une structure centrale d'aglycone comprenant un groupe aldéhyde qui a été dérivatisé par :
- réduction en un alcool ; ou
- transformation en une liaison hydrazone par réaction avec l'hydrazide d'acide N-ε-maléimidocaproïque (EMCH), fournissant ainsi une saponine-Ald-EMCH telle qu'une SO1861-Ald-EMCH ou une QS-21-Ald-EMCH, dans laquelle le groupe maléimide de EMCH est facultativement dérivatisé par formation d'une liaison thioéther avec du mercaptoéthanol ; ou
- transformation en une liaison hydrazone par réaction avec l'hydrazide de N-[acide β-maléimidopropionique] (BMPH), dans laquelle le groupe maléimide de BMPH est facultativement dérivatisé par formation d'une liaison thioéther avec du mercaptoéthanol ; ou
- transformation en une liaison hydrazone par réaction avec l'hydrazide de N-[acide κ-maléimidoundécanoïque] (KMUH), dans laquelle le groupe maléimide de KMUH est facultativement dérivatisé par formation d'une liaison thioéther avec du mercaptoéthanol ; ou
ii. le dérivé de saponine comprend une chaîne saccharide, de préférence une chaîne saccharide choisie dans le groupe A tel que défini dans la revendication 7, la chaîne saccharide comprenant un groupe carboxyle, de préférence un groupe carboxyle d'une fraction acide glucuronique qui a été dérivatisé par transformation en une liaison amide par réaction avec 2-amino-2-méthyl-1,3-propanediol (AMPD) ou N-(2-aminoéthyl)maléimide (AEM), fournissant ainsi une saponine-Glu-AMPD telle que QS-21-Glu-AMPD ou SO1861-Glu-AMPD ou une saponine-Glu-AEM telle que QS-21-Glu-AEM ou SO1861-Glu-AEM ; ou
iii. le dérivé de saponine comprend une chaîne saccharide, de préférence une chaîne saccharidique choisie dans le groupe B tel que défini dans la revendication 7, la chaîne saccharide comprenant un groupe acétoxy (Me(CO)O-) qui a été dérivatisé par transformation en un groupe hydroxyle (HO-) par désacétylation ; ou
iv. le dérivé de saponine comprend toute combinaison de dérivatisations i., ii. et iii., facultativement toute combinaison de deux dérivatisations i., ii. et iii. ;
de préférence, le dérivé de saponine comprend une structure centrale d'aglycone dans laquelle la structure centrale d'aglycone comprend un groupe aldéhyde qui a été dérivatisé par transformation en une liaison hydrazone par réaction avec EMCH, dans laquelle le groupe maléimide de EMCH est facultativement dérivatisé par la formation d'une liaison thioéther avec du mercaptoéthanol ;
de préférence, dans laquelle la saponine est un dérivé de saponine, dans laquelle :
i. le dérivé de saponine comprend une structure centrale d'aglycone comprenant un groupe aldéhyde qui a été dérivatisé par transformation en une liaison hydrazone par réaction avec l'hydrazide d'acide N-ε-maléimidocaproïque (EMCH), fournissant ainsi une saponine-Ald-EMCH telle qu'une SO1861-Ald-EMCH ou une QS-21-Ald-EMCH ; ou
ii. la saponine comprend une chaîne saccharide, de préférence une chaîne saccharidique choisie dans le groupe A tel que défini dans la revendication 7, la chaîne saccharide comprenant un groupe carboxyle, de préférence un groupe carboxyle d'une fraction acide glucuronique qui a été dérivatisé par transformation en une liaison amide par réaction avec *N*-(2-aminoéthyl)maléimide (AEM), fournissant ainsi une saponine-Glu-AEM telle qu'une QS-21-Glu-AEM ou une SO1861-Glu-AEM ; ou
iii. le dérivé de saponine comprend une combinaison de dérivatisations i. et ii.

14. Combinaison pharmaceutique selon l'une quelconque des revendications précédentes, dans laquelle la saponine est un dérivé de saponine représenté par la Molécule 2 : 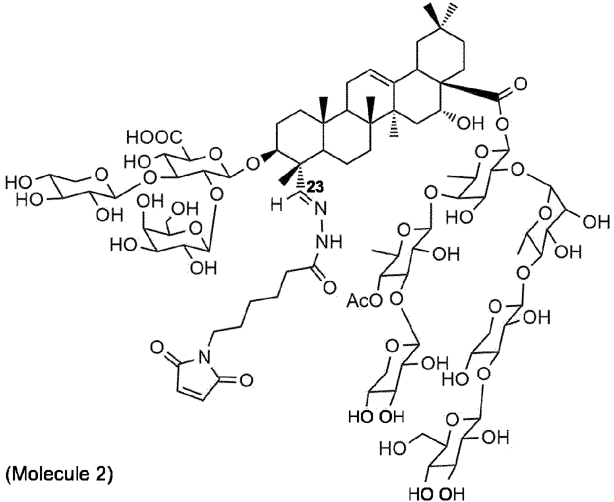 ou dans laquelle le dérivé de saponine est le dérivé de saponine représenté par la Molécule 3

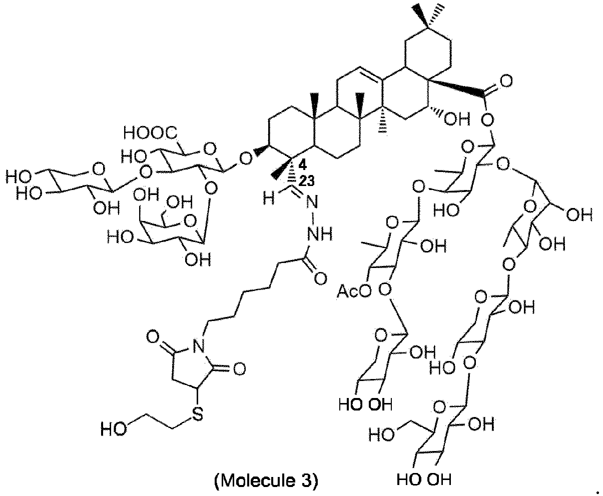

15. Combinaison pharmaceutique selon l'une quelconque des revendications précédentes pour une utilisation :
- comme médicament ;
- dans le traitement ou la prophylaxie d'une maladie ou d'un problème de santé dans lequel un produit d'expression est impliqué dans un ou plusieurs quelconques des gènes : apoB, HSP27, TTR, PCSK9, ALAS1, AT3, GO, CC5, gène X de VHB, gène S de VHB, AAT et LDH ; et/ou
- dans le traitement ou la prophylaxie d'un cancer, d'une maladie infectieuse, d'une infection virale, d'une hypercholestérolémie, hyperoxalurie primaire, hémophilie A, hémophilie B, maladie hépatique liée à AAT, porphyrie hépatique aiguë, amylose médiée par TTR, amylose TTR héréditaire (hATTR), maladie médiée par le complément, infection par l'hépatite B, d'une maladie ou d'un trouble lié à l'expression de HSP27, ou d'une maladie auto-immune.

16. Procédé *in vitro* ou *ex vivo* de transfert du conjugué tel que défini dans l'une quelconque des revendications 1 à 14 de l'extérieur d'une cellule vers l'intérieur de ladite cellule, de préférence dans le cytosol de ladite cellule, comprenant les étapes consistant à :
a) fournir une cellule qui exprime ASGPR, de préférence ASGPR1, sur sa surface, de préférence sélectionnée parmi une cellule hépatique, une cellule infectée par virus et une cellule cancéreuse ;
b) fournir le conjugué tel que défini dans l'une quelconque des revendications 1 à 14 ;
c) fournir la saponine telle que définie dans l'une quelconque des revendications 1 à 14 ;
d) mettre en contact la cellule de l'étape a) *in vitro* ou *ex vivo* avec le conjugué de l'étape b) et la saponine de l'étape c), en effectuant ainsi le transfert du conjugué de l'extérieur de la cellule vers l'intérieur de ladite cellule.
